# EUROPÄISCHE PATENTSCHRIFT

(11) **EP 2 152 659 B1**
(45) Veröffentlichungstag und Bekanntmachung des Hinweises auf die Patenterteilung: **24.04.2013**
(21) Anmeldenummer: 08760294.2
(22) Anmeldetag: 30.05.2008
(51) Int. Cl.: C07C 57/04, C12P 7/62, C08F 220/06, C07C 69/54

(54) **EIN VERFAHREN ZUR HERSTELLUNG VON METHACRYLSÄURE ODER METHACRYLSÄUREESTERN**
A PROCESS FOR PREPARING METHACRYLIC ACID OR METHACRYLIC ESTERS
PROCÉDÉ DE PRODUCTION D'ACIDE MÉTHACRYLIQUE OU D'ESTERS D'ACIDE MÉTHACRYLIQUE

(30) Priorität: 01.06.2007 WO PCT/EP2007/055394
(43) Veröffentlichungstag der Anmeldung: 17.02.2010
(73) Patentinhaber: Evonik Röhm GmbH, 64293 Darmstadt (DE)
(72) Erfinder: MARX, Achim, 63571 Gelnhausen (DE); PÖTTER, Markus, 48149 Münster (DE); BUCHHOLZ, Stefan, 63456 Hanau (DE); MAY, Alexander, 64342 Seeheim (DE); SIEGERT, Hermann, 64342 Seeheim-Jugenheim (DE); ALBER, Birgit, 70599 Stuttgart (DE); FUCHS, Georg, 79423 Heltersheim (DE); EGGELING, Lothar, 52428 Jülich (DE)
(86) Internationale Anmeldenummer: PCT/EP2008/056707
(87) Internationale Veröffentlichungsnummer: WO 2008/145737

(56) Entgegenhaltungen:
- EP-A- 1 186 592
- US-A- 3 562 320
- KOROTKOVA NATALIA ET AL: "Glyoxylate regeneration pathway in the methylotroph Methylobacterium extorquens AM1" JOURNAL OF BACTERIOLOGY, Bd. 184, Nr. 6, März 2002 (2002-03), Seiten 1750-1758, XP002496577 ISSN: 0021-9193 in der Anmeldung erwähnt
- LEE I Y ET AL: "High production of D-beta-hydroxyisobutyric acid from methacrylic acid by Candida rugosa and its mutant" BIOPROCESS ENGINEERING, Bd. 16, Nr. 5, 1997, Seiten 247-252, XP002496578 ISSN: 0178-515X

### SEQUENCE LISTING

<110> Evonik Degussa GmbH
<120> Mikrobiologische Herstellung von 3-Hydroxyisobuttersäure
<130> DR82387
<160> 45
< 170> PatentIn version 3.4
<210> 1
   <211> 2214
   <212> DNA
   <213> Corynebacterium glutamicum ATCC 13032
<400> 1
   atgacgtcga tccctaattt ttcagacatc ccattgactg ctgagacacg tgcatcggag 60
   tcacacaacg ttgacgccgg caaggtgtgg aacactcccg aaggcattga tgtcaagcgc 120
   gtattcacgc aggctgaccg cgacgaggcg caagcggcgg gacatccggt ggattctttg 180
   ccaggtcaaa agccatttat gcgcgggccg tacccaacta tgtacaccaa tcagccgtgg 240
   acgattcgcc agtacgcagg cttttcaacc gccgcggaat ccaatgcgtt ttatcggagg 300
   aaccttgctg cgggtcaaaa aggtttgtcg gttgcgttcg atctagcgac ccaccgcggt 360
   tatgactcgg ataatgagcg cgtggtcggc gatgtgggta tggccggcgt ggcgattgat 420
   tcgattttgg atatgcgtca gctgtttgat ggcattgatt tgtccagcgt gtcggtgtcg 480
   atgaccatga atggcgctgt gctgccgatt cttgcgttct atatcgtggc ggctgaggaa 540
   caaggtgtgg gtccggagca gcttgcgggc acgatccaga atgacatctt gaaagaattt 600
   atggtgcgca acacctatat ttatccgccg aagccgtcga tgcgcatcat ttccaacatc 660
   tttgagtaca cctccttgaa gatgccacgt tttaactcca tttcgatttc tggctatcac 720
   atccaggaag cgggagcgac tgccgatttg gagctggcct acactctggc ggatggtatt 780
   gaatacatcc gtgcaggtaa agaggtaggc cttgacgtgg ataagttcgc gcctcgtctg 840
   tccttcttct ggggtatttc tatgtacacc ttcatggaga tcgcaaagct gcgtgcggga 900
   cgactgctgt ggagcgagtt ggtggcaaaa ttcgatccga aaaacgccaa gtcccagtcg 960
   ctgcgcacgc actcgcagac ctctggttgg tcgttgaccg cgcaggatgt gtacaacaac 1020
   gtcgcccgca ccgcgattga ggcgatggct gcaacccagg gccacaccca gtcgctgcac 1080
   accaatgcac ttgatgaggc gttggcgctg cccaccgatt tctctgctcg tatcgcccga 1140
   aacacccagc tgttgctgca gcaggaatct ggcacggtgc gtccagttga tccatgggcg 1200
   ggctcctatt acgtggagtg gttgaccaat gagctggcta accgcgcgcg caagcacatc 1260
   gatgaggtgg aggaagccgg cggaatggcg caggccaccg cgcagggaat tcctaagctg 1320
   cgcattgagg aatcagcggc acgcacccag gctcgcattg attccggccg ccaggcgctg 1380
   atcggcgtga atcgctacgt ggcggaagaa gatgaggaaa ttgaagtcct caaggttgac 1440
   aacaccaagg ttcgcgcaga acagttggct aaactcgcgc aactgaaagc agagcgcaac 1500
   gatgcggaag tcaaggctgc gctggatgcg ttgacagctg ctgcccgcaa cgagcataaa 1560
   gagccagggg atttggatca gaacctgctc aaacttgccg tcgatgctgc gcgcgcaaaa 1620
   gctaccattg gagagatctc cgatgctttg gaagttgtct ttggccgcca cgaagcagaa 1680
   atcaggacgc tgtctggcgt gtacaaggat gaggttggaa aggaaggcac agtgagcaac 1740
   gtcgaacgcg cgatcgccct ggctgacgcc tttgaggctg aggaaggccg ccgcccacgt 1800
   atctttattg ccaagatggg ccaggatgga catgaccgtg gacagaaggt tgtcgcgtct 1860
   gcctatgctg acctgggcat ggacgtggat gttggaccgc tgtttcaaac tccagccgaa 1920
   gctgcccgcg ccgccgtgga cgccgatgtt cacgtggtgg gtatgtcttc gctggcagca 1980
   ggccacctca ccttgctgcc cgagctgaag aaagaacttg cagctcttgg ccgcgatgac 2040
   attctggtca ccgtgggcgg cgtcattccg ccgggcgatt tccaggatct ctacgatatg 2100
   ggtgccgccg cgatttaccc tccaggaacc gtcatcgcgg agtcggcgat cgatctgatc 2160
   acccgactcg ccgcacacct gggctttgac ctggatgtgg atgtgaatga gtga 2214
<210> 2
   <211> 737
   <212> PRT
   <213> Corynebacterium *glutamicum* ATCC 13032
<400> 2
   Met Thr Ser Ile Pro Asn Phe Ser Asp Ile Pro Leu Thr Ala Glu Thr
   1 5 10 15
   Arg Ala Ser Glu Ser His Asn Val Asp Ala Gly Lys Val Trp Asn Thr
   20 25 30
   Pro Glu Gly Ile Asp Val Lys Arg Val Phe Thr Gln Ala Asp Arg Asp
   35 40 45
   Glu Ala Gln Ala Ala Gly His Pro Val Asp Ser Leu Pro Gly Gln Lys
   50 55 60
   Pro Phe Met Arg Gly Pro Tyr Pro Thr Met Tyr Thr Asn Gln Pro Trp
   65 70 75 80
   Thr Ile Arg Gln Tyr Ala Gly Phe Ser Thr Ala Ala Glu Ser Asn Ala
   85 90 95
   Phe Tyr Arg Arg Asn Leu Ala Ala Gly Gln Lys Gly Leu Ser Val Ala
   100 105 110
   Phe Asp Leu Ala Thr His Arg Gly Tyr Asp Ser Asp Asn Glu Arg Val
   115 120 125
   Val Gly Asp Val Gly Met Ala Gly Val Ala Ile Asp Ser Ile Leu Asp
   130 135 140
   Met Arg Gln Leu Phe Asp Gly Ile Asp Leu Ser Ser Val Ser Val Ser
   145 150 155 160
   Met Thr Met Asn Gly Ala Val Leu Pro Ile Leu Ala Phe Tyr Ile Val
   165 170 175
   Ala Ala Glu Glu Gln Gly Val Gly Pro Glu Gln Leu Ala Gly Thr Ile
   180 185 190
   Gln Asn Asp Ile Leu Lys Glu Phe Met Val Arg Asn Thr Tyr Ile Tyr
   195 200 205
   Pro Pro Lys Pro Ser Met Arg Ile Ile Ser Asn Ile Phe Glu Tyr Thr
   210 215 220
   Ser Leu Lys Met Pro Arg Phe Asn Ser Ile Ser Ile Ser Gly Tyr His
   225 230 235 240
   Ile Gln Glu Ala Gly Ala Thr Ala Asp Leu Glu Leu Ala Tyr Thr Leu
   245 250 255
   Ala Asp Gly Ile Glu Tyr Ile Arg Ala Gly Lys Glu Val Gly Leu Asp
   260 265 270
   Val Asp Lys Phe Ala Pro Arg Leu Ser Phe Phe Trp Gly Ile Ser Met
   275 280 285
   Tyr Thr Phe Met Glu Ile Ala Lys Leu Arg Ala Gly Arg Leu Leu Trp
   290 295 300
   Ser Glu Leu Val Ala Lys Phe Asp Pro Lys Asn Ala Lys Ser Gln Ser
   305 310 315 320
   Leu Arg Thr His Ser Gln Thr Ser Gly Trp Ser Leu Thr Ala Gln Asp
   325 330 335
   Val Tyr Asn Asn Val Ala Arg Thr Ala Ile Glu Ala Met Ala Ala Thr
   340 345 350
   Gln Gly His Thr Gln Ser Leu His Thr Asn Ala Leu Asp Glu Ala Leu
   355 360 365
   Ala Leu Pro Thr Asp Phe Ser Ala Arg Ile Ala Arg Asn Thr Gln Leu
   370 375 380
   Leu Leu Gln Gln Glu Ser Gly Thr Val Arg Pro Val Asp Pro Trp Ala
   385 390 395 400
   Gly Ser Tyr Tyr Val Glu Trp Leu Thr Asn Glu Leu Ala Asn Arg Ala
   405 410 415
   Arg Lys His Ile Asp Glu Val Glu Glu Ala Gly Gly Met Ala Gln Ala
   420 425 430
   Thr Ala Gln Gly Ile Pro Lys Leu Arg Ile Glu Glu Ser Ala Ala Arg
   435 440 445
   Thr Gln Ala Arg Ile Asp Ser Gly Arg Gln Ala Leu Ile Gly Val Asn
   450 455 460
   Arg Tyr Val Ala Glu Glu Asp Glu Glu Ile Glu Val Leu Lys Val Asp
   465 470 475 480
   Asn Thr Lys Val Arg Ala Glu Gln Leu Ala Lys Leu Ala Gln Leu Lys
   485 490 495
   Ala Glu Arg Asn Asp Ala Glu Val Lys Ala Ala Leu Asp Ala Leu Thr
   500 505 510
   Ala Ala Ala Arg Asn Glu His Lys Glu Pro Gly Asp Leu Asp Gln Asn
   515 520 525
   Leu Leu Lys Leu Ala Val Asp Ala Ala Arg Ala Lys Ala Thr Ile Gly
   530 535 540
   Glu Ile Ser Asp Ala Leu Glu Val Val Phe Gly Arg His Glu Ala Glu
   545 550 555 560
   Ile Arg Thr Leu Ser Gly Val Tyr Lys Asp Glu Val Gly Lys Glu Gly
   565 570 575
   Thr Val Ser Asn Val Glu Arg Ala Ile Ala Leu Ala Asp Ala Phe Glu
   580 585 590
   Ala Glu Glu Gly Arg Arg Pro Arg Ile Phe Ile Ala Lys Met Gly Gln
   595 600 605
   Asp Gly His Asp Arg Gly Gln Lys Val Val Ala Ser Ala Tyr Ala Asp
   610 615 620
   Leu Gly Met Asp Val Asp Val Gly Pro Leu Phe Gln Thr Pro Ala Glu
   625 630 635 640
   Ala Ala Arg Ala Ala Val Asp Ala Asp Val His Val Val Gly Met Ser
   645 650 655
   Ser Leu Ala Ala Gly His Leu Thr Leu Leu Pro Glu Leu Lys Lys Glu
   660 665 670
   Leu Ala Ala Leu Gly Arg Asp Asp Ile Leu Val Thr Val Gly Gly Val
   675 680 685
   Ile Pro Pro Gly Asp Phe Gln Asp Leu Tyr Asp Met Gly Ala Ala Ala
   690 695 700
   Ile Tyr Pro Pro Gly Thr Val Ile Ala Glu Ser Ala Ile Asp Leu Ile
   705 710 715 720
   Thr Arg Leu Ala Ala His Leu Gly Phe Asp Leu Asp Val Asp Val Asn
   725 730 735
   Glu
<210> 3
   <211> 1071
   <212> DNA
   <213> Sulfolobus *tokodaii*
<400> 3
   atgaggagaa cattaaaagc cgcaatatta ggtgctactg gtttagtagg aatcgaatac 60
   gtaagaatgc tatcaaatca tccttatatt aaaccagcat atttagctgg aaaaggttca 120
   gtgggtaaac cgtatggtga ggtagtaaga tggcaaacag taggacaagt tcctaaggaa 180
   atagctgata tggaaataaa accaactgat cctaagttaa tggatgatgt agacataata 240
   ttttctccat tacctcaagg tgctgctggc ccagtagaag aacaatttgc aaaagaagga 300
   ttccctgtga ttagtaattc accagatcat agatttgatc ctgatgttcc cttattggtt 360
   cctgaactaa atcctcatac tattagctta attgatgagc aaagaaaaag aagagaatgg 420
   aaaggattta tagtaactac accactatgc acagcccagg gtgcagcaat accattaggt 480
   gctatattta aagattataa gatggatgga gcatttataa ctactattca atcgctatct 540
   ggtgccggtt atccaggaat accatcatta gatgtagtag ataatatctt gcctttaggt 600
   gatggatacg atgccaagac gataaaagag atcttcagaa ttttaagcga agttaagaga 660
   aatgtagatg aacctaaatt agaagatgta agcttagcag caacaactca tagaatagct 720
   actatacatg gtcattatga agtactatat gtatcgttca aagaggaaac tgctgctgaa 780
   aaagttaagg agactttaga aaactttaga ggggaaccac aagatctaaa attaccaact 840
   gcaccttcaa agccaattat cgttatgaat gaggatacaa gacctcaagt ctattttgat 900
   agatgggctg gggatattcc aggaatgagt gtagttgtag gtagattaaa gcaagtgaat 960
   aagagaatga taaggttagt atcattaatt cataacacgg tcagaggagc cgcaggagga 1020
   ggtatattag cagctgaatt acttgtcgaa aaaggatata ttgaaaagta a 1071
<210> 4
   <211> 356
   <212> PRT
   <213> Sulfolobus *tokodaii*
<400> 4
   Met Arg Arg Thr Leu Lys Ala Ala Ile Leu Gly Ala Thr Gly Leu Val
   1 5 10 15
   Gly Ile Glu Tyr Val Arg Met Leu Ser Asn His Pro Tyr Ile Lys Pro
   20 25 30
   Ala Tyr Leu Ala Gly Lys Gly Ser Val Gly Lys Pro Tyr Gly Glu Val
   35 40 45
   Val Arg Trp Gln Thr Val Gly Gln Val Pro Lys Glu Ile Ala Asp Met
   50 55 60
   Glu Ile Lys Pro Thr Asp Pro Lys Leu Met Asp Asp Val Asp Ile Ile
   65 70 75 80
   Phe Ser Pro Leu Pro Gln Gly Ala Ala Gly Pro Val Glu Glu Gln Phe
   85 90 95
   Ala Lys Glu Gly Phe Pro Val Ile Ser Asn Ser Pro Asp His Arg Phe
   100 105 110
   Asp Pro Asp Val Pro Leu Leu Val Pro Glu Leu Asn Pro His Thr Ile
   115 120 125
   Ser Leu Ile Asp Glu Gln Arg Lys Arg Arg Glu Trp Lys Gly Phe Ile
   130 135 140
   Val Thr Thr Pro Leu Cys Thr Ala Gln Gly Ala Ala Ile Pro Leu Gly
   145 150 155 160
   Ala Ile Phe Lys Asp Tyr Lys Met Asp Gly Ala Phe Ile Thr Thr Ile
   165 170 175
   Gln Ser Leu Ser Gly Ala Gly Tyr Pro Gly Ile Pro Ser Leu Asp Val
   180 185 190
   Val Asp Asn Ile Leu Pro Leu Gly Asp Gly Tyr Asp Ala Lys Thr Ile
   195 200 205
   Lys Glu Ile Phe Arg Ile Leu Ser Glu Val Lys Arg Asn Val Asp Glu
   210 215 220
   Pro Lys Leu Glu Asp Val Ser Leu Ala Ala Thr Thr His Arg Ile Ala
   225 230 235 240
   Thr Ile His Gly His Tyr Glu Val Leu Tyr Val Ser Phe Lys Glu Glu
   245 250 255
   Thr Ala Ala Glu Lys Val Lys Glu Thr Leu Glu Asn Phe Arg Gly Glu
   260 265 270
   Pro Gln Asp Leu Lys Leu Pro Thr Ala Pro Ser Lys Pro Ile Ile Val
   275 280 285
   Met Asn Glu Asp Thr Arg Pro Gln Val Tyr Phe Asp Arg Trp Ala Gly
   290 295 300
   Asp Ile Pro Gly Met Ser Val Val Val Gly Arg Leu Lys Gln Val Asn
   305 310 315 320
   Lys Arg Met Ile Arg Leu Val Ser Leu Ile His Asn Thr Val Arg Gly
   325 330 335
   Ala Ala Gly Gly Gly Ile Leu Ala Ala Glu Leu Leu Val Glu Lys Gly
   340 345 350
   Tyr Ile Glu Lys
   355
<210> 5
   <211> 1293
   <212> DNA
   <213> Rhodobacter sphaeroides
<400> 5
   atggccctcg acgtgcagag cgatatcgtc gcctacgacg cgcccaagaa ggacctctac 60
   gagatcggcg agatgccgcc tctcggccat gtgccgaagg agatgtatgc ttgggccatc 120
   cggcgcgagc gtcatggcga gccggatcag gccatgcaga tcgaggtggt cgagacgccc 180
   tcgatcgaca gccacgaggt gctcgttctc gtgatggcgg cgggcgtgaa ctacaacggc 240
   atctgggccg gcctcggcgt gcccgtctcg ccgttcgacg gtcacaagca gccctatcac 300
   atcgcgggct ccgacgcgtc gggcatcgtc tgggcggtgg gcgacaaggt caagcgctgg 360
   aaggtgggcg acgaggtcgt gatccactgc aaccaggacg acggcgacga cgaggaatgc 420
   aacggcggcg acccgatgtt ctcgcccacc cagcggatct ggggctacga gacgccggac 480
   ggctccttcg cccagttcac ccgcgtgcag gcgcagcagc tgatgaagcg tccgaagcac 540
   ctgacctggg aagaggcggc ctgctacacg ctgaccctcg ccaccgccta ccggatgctc 600
   ttcggccaca agccgcacga cctgaagccg gggcagaacg tgctggtctg gggcgcctcg 660
   ggcggcctcg gctcctacgc gatccagctc atcaacacgg cgggcgccaa tgccatcggc 720
   gtcatctcag aggaagacaa gcgcgacttc gtcatggggc tgggcgccaa gggcgtcatc 780
   aaccgcaagg acttcaagtg ctggggccag ctgcccaagg tgaactcgcc cgaatataac 840
   gagtggctga aggaggcgcg caagttcggc aaggccatct gggacatcac cggcaagggc 900
   atcaacgtcg acatggtgtt cgaacatccg ggcgaggcga ccttcccggt ctcgtcgctg 960
   gtggtgaaga agggcggcat ggtcgtgatc tgcgcgggca ccaccggctt caactgcacc 1020
   ttcgacgtcc gctacatgtg gatgcaccag aagcgcctgc agggcagcca tttcgccaac 1080
   ctcaagcagg cctccgcggc caaccagctg atgatcgagc gccgcctcga tccctgcatg 1140
   tccgaggtct tcccctgggc cgagatcccg gctgcccata cgaagatgta taagaaccag 1200
   cacaagcccg gcaacatggc ggtgctggtg caggccccgc gcacggggtt gcgcaccttc 1260
   gccgacgtgc tcgaggccgg ccgcaaggcc tga 1293
<210> 6
   <211> 430
   <212> PRT
   <213> Rhodobacter sphaeroides
<400> 6
   Met Ala Leu Asp Val Gln Ser Asp Ile Val Ala Tyr Asp Ala Pro Lys
   1 5 10 15
   Lys Asp Leu Tyr Glu Ile Gly Glu Met Pro Pro Leu Gly His Val Pro
   20 25 30
   Lys Glu Met Tyr Ala Trp Ala Ile Arg Arg Glu Arg His Gly Glu Pro
   35 40 45
   Asp Gln Ala Met Gln Ile Glu Val Val Glu Thr Pro Ser Ile Asp Ser
   50 55 60
   His Glu Val Leu Val Leu Val Met Ala Ala Gly Val Asn Tyr Asn Gly
   65 70 75 80
   Ile Trp Ala Gly Leu Gly Val Pro Val Ser Pro Phe Asp Gly His Lys
   85 90 95
   Gln Pro Tyr His Ile Ala Gly Ser Asp Ala Ser Gly Ile Val Trp Ala
   100 105 110
   Val Gly Asp Lys Val Lys Arg Trp Lys Val Gly Asp Glu Val Val Ile
   115 120 125
   His Cys Asn Gln Asp Asp Gly Asp Asp Glu Glu Cys Asn Gly Gly Asp
   130 135 140
   Pro Met Phe Ser Pro Thr Gln Arg Ile Trp Gly Tyr Glu Thr Pro Asp
   145 150 155 160
   Gly Ser Phe Ala Gln Phe Thr Arg Val Gln Ala Gln Gln Leu Met Lys
   165 170 175
   Arg Pro Lys His Leu Thr Trp Glu Glu Ala Ala Cys Tyr Thr Leu Thr
   180 185 190
   Leu Ala Thr Ala Tyr Arg Met Leu Phe Gly His Lys Pro His Asp Leu
   195 200 205
   Lys Pro Gly Gln Asn Val Leu Val Trp Gly Ala Ser Gly Gly Leu Gly
   210 215 220
   Ser Tyr Ala Ile Gln Leu Ile Asn Thr Ala Gly Ala Asn Ala Ile Gly
   225 230 235 240
   Val Ile Ser Glu Glu Asp Lys Arg Asp Phe Val Met Gly Leu Gly Ala
   245 250 255
   Lys Gly Val Ile Asn Arg Lys Asp Phe Lys Cys Trp Gly Gln Leu Pro
   260 265 270
   Lys Val Asn Ser Pro Glu Tyr Asn Glu Trp Leu Lys Glu Ala Arg Lys
   275 280 285
   Phe Gly Lys Ala Ile Trp Asp Ile Thr Gly Lys Gly Ile Asn Val Asp
   290 295 300
   Met Val Phe Glu His Pro Gly Glu Ala Thr Phe Pro Val Ser Ser Leu
   305 310 315 320
   Val Val Lys Lys Gly Gly Met Val Val Ile Cys Ala Gly Thr Thr Gly
   325 330 335
   Phe Asn Cys Thr Phe Asp Val Arg Tyr Met Trp Met His Gln Lys Arg
   340 345 350
   Leu Gln Gly Ser His Phe Ala Asn Leu Lys Gln Ala Ser Ala Ala Asn
   355 360 365
   Gln Leu Met Ile Glu Arg Arg Leu Asp Pro Cys Met Ser Glu Val Phe
   370 375 380
   Pro Trp Ala Glu Ile Pro Ala Ala His Thr Lys Met Tyr Lys Asn Gln
   385 390 395 400
   His Lys Pro Gly Asn Met Ala Val Leu Val Gln Ala Pro Arg Thr Gly
   405 410 415
   Leu Arg Thr Phe Ala Asp Val Leu Glu Ala Gly Arg Lys Ala
   420 425 430
<210> 7
   <211> 1233
   <212> DNA
   <213> Pseudomonas aeruginosa
<400> 7
   atgagtgatt acgagccgtt gcgtctgcat gtcccggagc ccaccgggcg tcctggctgc 60
   aagaccgact tttcctatct gcacctgtcc cccgccggcg aggtacgcaa gccgccggtg 120
   gatgtcgagc ccgccgagac cagcgacctg gcctacagcc tggtacgtgt gctcgacgac 180
   gacggccacg ccgtcggtcc ctggaatccg cagctcagca acgaacaact gctgcgcggc 240
   atgcgggcga tgctcaagac ccgcctgttc gacgcgcgca tgctcaccgc gcaacggcag 300
   aaaaagcttt ccttctatat gcaatgcctc ggcgaggaag ccatcgccac cgcccacacc 360
   ctggccctgc gcgacggcga catgtgcttt ccgacctatc gccagcaagg catcctgatc 420
   acccgcgaat acccgctggt ggacatgatc tgccagcttc tctccaacga ggccgacccg 480
   ctcaagggcc gccagctgcc gatcatgtac tcgagcaagg aggcaggttt cttctccatc 540
   tccggcaacc tcgccaccca gttcatccag gcggtcggct ggggcatggc ctcggcgatc 600
   aagggcgaca cgcgcatcgc ctcggcctgg atcggcgacg gcgccaccgc cgagtcggac 660
   ttccacaccg ccctcacctt cgcccatgtc taccgcgcgc cggtaatcct caacgtggtc 720
   aacaaccagt gggcgatctc caccttccag gccatcgccg gcggcgaagg caccaccttc 780
   gccaaccgtg gcgtgggctg cgggatcgcc tcgctgcggg tcgacggcaa tgacttcctg 840
   gcggtctacg ccgcctccga gtgggccgcc gagcgcgccc ggcgcaacct cgggccgagc 900
   ctgatcgaat gggtcaccta ccgcgccggc ccgcactcga cttcggacga cccgtccaag 960
   taccgccccg ccgacgactg gaccaacttc ccgctgggcg acccgatcgc ccgcctgaag 1020
   cggcacatga tcggcctcgg catctggtcg gaggaacagc acgaagccac ccacaaggcc 1080
   ctcgaagccg aagtactggc cgcgcagaaa caggcggaga gccatggcac cctgatcgac 1140
   ggccgggtgc cgagcgccgc cagcatgttc gaggacgtct atgcagaact gccggagcac 1200
   ctgcgccggc aacgccagga gctcggggta tga 1233
<210> 8
   <211> 1049
   <212> DNA
   <213> Pseudomonas aeruginosa
<400> 8
   atgccatgaa cccgcaacac gagaacgccc agacggtcac cagcatgacc atgatccagg 60
   cgctgcgctc ggcgatggac atcatgctcg agcgcgacga cgacgtggtg gtattcggcc 120
   aggacgtcgg ctacttcggc ggcgtgttcc gctgcaccga aggcctgcag aagaaatacg 180
   gcacctcgcg ggtgttcgat gcgccgatct ccgagagcgg catcatcggc gccgcggtcg 240
   gcatgggtgc ctacggcctg cgcccggtgg tggagatcca gttcgccgac tacgtctacc 300
   cggcctccga ccagttgatc tccgaggcgg cgcgcctgcg ctatcgctcg gccggcgact 360
   tcatcgtgcc gatgaccgta cgcatgccct gtggcggcgg catctacggc gggcaaacgc 420
   acagccagag cccggaggcg atgttcaccc aggtctgcgg cctgcgcacg gtgatgccgt 480
   ccaaccccta cgacgccaag ggcctgctga tcgcctgcat cgagaacgac gacccggtga 540
   tcttcctcga gcccaagcgc ctctacaacg gcccgttcga tggccaccac gaccgcccgg 600
   tgacgccctg gtccaagcat ccggccagcc aggtgccgga cggctactac aaggtgccgc 660
   tggacaaggc ggcgatcgtc cgccccggcg cggcgctgac cgtgctgacc tacggcacca 720
   tggtctacgt ggcccaggcc gcggccgacg agaccggcct ggacgccgag atcatcgacc 780
   tgcgcagcct ctggccgctg gacctggaaa ccatcgtcgc ctcggtgaag aagaccggcc 840
   gctgcgtcat cgcccacgag gcgacccgca cctgcgggtt cggcgccgag ctgatgtcgc 900
   tggtgcagga gcactgcttc caccacctgg aggcgccgat cgagcgcgtc accggttggg 960
   acacccccta cccgcatgcc caggagtggg cgtatttccc cggccccgcg cgcgtcggcg 1020
   cggcattcaa gcgtgtgatg gaggtctga 1049
<210> 9
   <211> 924
   <212> DNA
   <213> Pseudomonas aeruginosa
<400> 9
   atgtcgatgg ccgatcgtga tggcgtgatc tggtatgacg gtgaactggt gcagtggcgc 60
   gacgcgacca cgcacgtgct gacccatacc ctgcactatg gaatgggcgt gttcgagggc 120
   gtgcgcgcct acgacacccc gcagggcacg gcgatcttcc gcctgcaggc gcataccgac 180
   cggctgttcg actccgcgca catcatgaac atgcagatcc cgtacagccg cgacgagatc 240
   aacgaggcga cccgcgccgc cgtgcgcgag aacaacctgg aaagcgccta tatccgcccg 300
   atggtgttct acggaagcga aggcatgggc ctgcgcgcca gcggcctgaa ggtccatgtg 360
   atcatcgccg cctggagctg gggcgcctac atgggcgagg aagccctgca gcaaggcatc 420
   aaggtgcgca ccagttcctt cacccgccac cacgtcaaca tctcgatgac ccgcgccaag 480
   tccaacggcg cctacatcaa ctcgatgctg gccctccagg aagcgatctc cggcggcgcc 540
   gacgaggcca tgatgctcga tccggaaggc tacgtggccg aaggctccgg cgagaacatc 600
   ttcatcatca aggatggcgt gatctacacc ccggaagtca ccgcctgcct gaacggcatc 660
   actcgtaaca ctatcctgac cctggccgcc gaacacggtt ttaaactggt cgagaagcgc 720
   atcacccgcg acgaggtgta catcgccgac gaggccttct tcactggcac tgccgcggaa 780
   gtcacgccga tccgcgaagt ggacggtcgc aagatcggcg ccggccgccg tggcccggtc 840
   accgaaaagc tgcagaaagc ctatttcgac ctggtcagcg gcaagaccga ggcccacgcc 900
   gagtggcgta ccctggtcaa gtaa 924
<210> 10
   <211> 1128
   <212> DNA
   <213> Acinetobacter calcoaceticus
<400> 10
   atgcaattta atgaagaaca gctattaatt caggatatgg cgaaaagttt tgccaatgaa 60
   cagattaaat ctaatgcagc agaatgggat aagcatagca tttttccaaa agacgttttg 120
   tcccaaatgg ggcaattggg ttttatggga atgctggtga gtgagaaatg gggcggatca 180
   aatacaggaa atttagctta tgtgctggca cttgaagaaa tcgctgccgc agatggtgcg 240
   acttcaacca ttatgagtgt acataattct gttggctgtg tacccattgc taaatttggt 300
   acagaggagc aaaagcagaa atatctagtg cctttagcac aaggtgaaat gatcggtgca 360
   tttgctttaa cggaaccaca tacaggttcc gatgccgcag ccattaaaac ccgagcaatt 420
   aaacaaggtg atgaatggat tattaatggc gctaaacaat ttataacatc aggtcataat 480
   gcgggcgtga ttattgtatt tgctgtgaca gatccgaatg cagggaaaaa agggctgagt 540
   gcatttattg tgccgcgtga aaccttgggt tatgaggtga ttcgcaccga agaaaaattg 600
   ggtttacatg cgtcagatac gtgccaaatt gctttaacgg atgttcgagt acatcacagc 660
   ttaatgcttg gtcaggaagg tgagggacta aaaatagcat tgtctaatct ggaaggtggc 720
   cgtattggga ttgcagcgca agccgttggt ttggcacgtg ctgcactaga agaagcgaca 780
   aaatatgcca aagagcgtgt gacctttgga aagcctattt ttgagcatca ggcgttagcc 840
   tttcgtttag ccagtatggc cacagaaatt gaagcagcac gacaattggt tcattacgca 900
   gcgcggctta aagaagctgg aaaaccttgt ttaaatgaag catcaatggc gaaattattt 960
   tcatctgaaa tggtcgaacg cgtatgttct gctgctttgc aaatctttgg tggctatggc 1020
   tatttaaaag actttcccat cgagcgaatt tatcgtgatg cacgtatttg ccagatttat 1080
   gaaggtacaa gtgatattca gcgtttagtg atagcaagaa gcctataa 1128
<210> 11
   <211> 774
   <212> DNA
   <213> Acinetobacter calcoaceticus
<400> 11
   atgacattcg caacaatttt attggaaaaa cgtaagggtg tgggcttgat tacacttaac 60
   cgtccaaaag cattaaatgc tttaaactca gaattaattt atgaaataaa tttagcctta 120
   gacgatttag aaaatgatca aacgattggt tgtatcgtcc ttacaggttc agaaaaagcc 180
   tttgccgcag gtgcggatat caaagaaatg gcagaattaa cttttccaaa tatttatttt 240
   gatgattttt ttagtcttgc agatcgtatt gcacagcgtc gtaagccttt aattgccgca 300
   gtgagtggtt atgctttagg tggtggctgt gagttagcac tcatgtgtga ctttatttat 360
   tgtgccgaca atgccaagtt tgcactacca gaagtaactt taggtgtcat tcctggtatt 420
   ggtggaacac agcgtctaac gcttgcaata ggcaaagcca aagccatgga aatgtgtttg 480
   actgcacggc aaatgcaggc tgctgaggca gaacaaagtg gtttggtggc acgcgttttt 540
   agtaaagaag aacttttaga acaaacctta caggctgccg aaaaaatagc ggaaaaatca 600
   cgggtatcta ccataatgat taaagagtca attaatcgag cttttgaagt gagtttagca 660
   gagggtttac gttttgagcg ccgaatgttc cattcagttt ttgcgacctt agatcagaaa 720
   gaaggcatgc aagcatttat tgataaacgt ccagcccaat ttaaacatca ataa 774
<210> 12
   <211> 1029
   <212> DNA
   <213> Acinetobacter calcoaceticus
<400> 12
   atgactacta ctgacaatca tttactcatt gaacataaaa acgctttagg aacaattatt 60
   ttaaatcgtc cagcgagtct gaacgcgcta tctctagaaa tgattaatgc gattcgtcaa 120
   caagttgagg attggcaagg tgatgtaaat gttcaggcca tattaattaa atcaaatagt 180
   cctaaagcat tttgtgcagg tggtgatatt cgctatcttt atgaaagtta taaaagtgga 240
   tcagaagagt ataaagatta tttcattgct gaatatgaga tgctcaatag cattcgaacg 300
   tctaaaaaaa cagtgattgt tttattggat ggatatgtat tgggtggtgg ttttggttta 360
   gcacaggctt gtcatatctt ggtgagtagt gaaaaatcac gattttcaat gccagaaaca 420
   gcaataggtt ttttcccaga tgttgcagcg acttatttct tatctcgttt agatgatgtt 480
   ggggtatatt tggcactgac tggtgatcaa atcagtagta gtgatgcatt gtatttagat 540
   ctgattgatt atcatgttcc gagtcagaat tttgagcgac tagaaaatgc attcagccaa 600
   tcacagaact tagataaatt tcatattcag aagattattt ctgcttatat ctccagccct 660
   gttcagagtg aactcagtct atggcttgaa gccattcgtc agcattttgg tcttaaaaat 720
   gtgcaagata tcgaagaaag tttgaaaaat gaacaagatc ccaactatca agtatggaca 780
   agtaaagtgt taaatacttt gcaacaacgt tcctctattg caaaaaaaac cagtttaaag 840
   ttacagctgc tagggcgtgg atggtcatta cagcaatgta tgcgtatcga gcgaaaatta 900
   caggatatct ggtttgaaca tggtgatatg attgagggtg ttcgagcgtt gattattgat 960
   aaagataaac aaccgcaatg gcagcagcat aatgcgactt tagataatat attaggccaa 1020
   ttaggttag 1029
<210> 13
   <211> 31
   <212> DNA
   <213> Artificial Sequence
<220>
   <223> Primer
<400> 13
   atgcaattta atgaagaaca gctattaatt c 31
<210> 14
   <211> 27
   <212> DNA
   <213> Artificial Sequence
<220>
   <223> Primer
<400> 14
   cagtctgaaa tgactaacct aattggc 27
<210> 15
   <211> 29
   <212> DNA
   <213> Artificial Sequence
<220>
   <223> Primer
<400> 15
   acggaattct gaaggagctg gcaactatg 29
<210> 16
   <211> 28
   <212> DNA
   <213> Artificial Sequence
<220>
   <223> Primer
<400> 16
   ttgtcgactt acttgaccag ggtacgcc 28
<210> 17
   <211> 31
   <212> DNA
   <213> Artificial Sequence
<220>
   <223> Primer
<400> 17
   acagatctgg aggcctgtca tgagtgatta c 31
<210> 18
   <211> 25
   <212> DNA
   <213> Artificial Sequence
<220>
   <223> Primer
<400> 18
   atgggtaccc attcagacct ccatc 25
<210> 19
   <211> 6960
   <212> DNA
   <213> Artificial
<220>
   <223> New Plasmid
<400> 19
   ggggaattgt gagcggataa caattcccct gtagaaataa ttttgtttaa ctttaataag 60
   gagatatacc atgggcagca gccatcacca tcatcaccac agccaggatc cgaattctga 120
   aggagctggc aactatgtcg atggccgatc gtgatggcgt gatctggtat gacggtgaac 180
   tggtgcagtg gcgcgacgcg accacgcacg tgctgaccca taccctgcac tatggaatgg 240
   gcgtgttcga gggcgtgcgc gcctacgaca ccccgcaggg cacggcgatc ttccgcctgc 300
   aggcgcatac cgaccggctg ttcgactccg cgcacatcat gaacatgcag atcccgtaca 360
   gccgcgacga gatcaacgag gcgacccgcg ccgccgtgcg cgagaacaac ctggaaagcg 420
   cctatatccg cccgatggtg ttctacggaa gcgaaggcat gggcctgcgc gccagcggcc 480
   tgaaggtcca tgtgatcatc gccgcctgga gctggggcgc ctacatgggc gaggaagccc 540
   tgcagcaagg catcaaggtg cgcaccagtt ccttcacccg ccaccacgtc aacatctcga 600
   tgacccgcgc caagtccaac ggcgcctaca tcaactcgat gctggccctc caggaagcga 660
   tctccggcgg cgccgacgag gccatgatgc tcgatccgga aggctacgtg gccgaaggct 720
   ccggcgagaa catcttcatc atcaaggatg gcgtgatcta caccccggaa gtcaccgcct 780
   gcctgaacgg catcactcgt aacactatcc tgaccctggc cgccgaacac ggttttaaac 840
   tggtcgagaa gcgcatcacc cgcgacgagg tgtacatcgc cgacgaggcc ttcttcactg 900
   gcactgccgc ggaagtcacg ccgatccgcg aagtggacgg tcgcaagatc ggcgccggcc 960
   gccgtggccc ggtcaccgaa aagctgcaga aagcctattt cgacctggtc agcggcaaga 1020
   ccgaggccca cgccgagtgg cgtaccctgg tcaagtaagt cgacaagctt gcggccgcat 1080
   aatgcttaag tcgaacagaa agtaatcgta ttgtacacgg ccgcataatc gaaattaata 1140
   cgactcacta taggggaatt gtgagcggat aacaattccc catcttagta tattagttaa 1200
   gtataagaag gagatataca tatggcagat ctggaggcct gtcatgagtg attacgagcc 1260
   gttgcgtctg catgtcccgg agcccaccgg gcgtcctggc tgcaagaccg acttttccta 1320
   tctgcacctg tcccccgccg gcgaggtacg caagccgccg gtggatgtcg agcccgccga 1380
   gaccagcgac ctggcctaca gcctggtacg tgtgctcgac gacgacggcc acgccgtcgg 1440
   tccctggaat ccgcagctca gcaacgaaca actgctgcgc ggcatgcggg cgatgctcaa 1500
   gacccgcctg ttcgacgcgc gcatgctcac cgcgcaacgg cagaaaaagc tttccttcta 1560
   tatgcaatgc ctcggcgagg aagccatcgc caccgcccac accctggccc tgcgcgacgg 1620
   cgacatgtgc tttccgacct atcgccagca aggcatcctg atcacccgcg aatacccgct 1680
   ggtggacatg atctgccagc ttctctccaa cgaggccgac ccgctcaagg gccgccagct 1740
   gccgatcatg tactcgagca aggaggcagg tttcttctcc atctccggca acctcgccac 1800
   ccagttcatc caggcggtcg gctggggcat ggcctcggcg atcaagggcg acacgcgcat 1860
   cgcctcggcc tggatcggcg acggcgccac cgccgagtcg gacttccaca ccgccctcac 1920
   cttcgcccat gtctaccgcg cgccggtaat cctcaacgtg gtcaacaacc agtgggcgat 1980
   ctccaccttc caggccatcg ccggcggcga aggcaccacc ttcgccaacc gtggcgtggg 2040
   ctgcgggatc gcctcgctgc gggtcgacgg caatgacttc ctggcggtct acgccgcctc 2100
   cgagtgggcc gccgagcgcg cccggcgcaa cctcgggccg agcctgatcg aatgggtcac 2160
   ctaccgcgcc ggcccgcact cgacttcgga cgacccgtcc aagtaccgcc ccgccgacga 2220
   ctggaccaac ttcccgctgg gcgacccgat cgcccgcctg aagcggcaca tgatcggcct 2280
   cggcatctgg tcggaggaac agcacgaagc cacccacaag gccctcgaag ccgaagtact 2340
   ggccgcgcag aaacaggcgg agagccatgg caccctgatc gacggccggg tgccgagcgc 2400
   cgccagcatg ttcgaggacg tctatgcaga actgccggag cacctgcgcc ggcaacgcca 2460
   ggagctcggg gtatgaatgc catgaacccg caacacgaga acgcccagac ggtcaccagc 2520
   atgaccatga tccaggcgct gcgctcggcg atggacatca tgctcgagcg cgacgacgac 2580
   gtggtggtat tcggccagga cgtcggctac ttcggcggcg tgttccgctg caccgaaggc 2640
   ctgcagaaga aatacggcac ctcgcgggtg ttcgatgcgc cgatctccga gagcggcatc 2700
   atcggcgccg cggtcggcat gggtgcctac ggcctgcgcc cggtggtgga gatccagttc 2760
   gccgactacg tctacccggc ctccgaccag ttgatctccg aggcggcgcg cctgcgctat 2820
   cgctcggccg gcgacttcat cgtgccgatg accgtacgca tgccctgtgg cggcggcatc 2880
   tacggcgggc aaacgcacag ccagagcccg gaggcgatgt tcacccaggt ctgcggcctg 2940
   cgcacggtga tgccgtccaa cccctacgac gccaagggcc tgctgatcgc ctgcatcgag 3000
   aacgacgacc cggtgatctt cctcgagccc aagcgcctct acaacggccc gttcgatggc 3060
   caccacgacc gcccggtgac gccctggtcc aagcatccgg ccagccaggt gccggacggc 3120
   tactacaagg tgccgctgga caaggcggcg atcgtccgcc ccggcgcggc gctgaccgtg 3180
   ctgacctacg gcaccatggt ctacgtggcc caggccgcgg ccgacgagac cggcctggac 3240
   gccgagatca tcgacctgcg cagcctctgg ccgctggacc tggaaaccat cgtcgcctcg 3300
   gtgaagaaga ccggccgctg cgtcatcgcc cacgaggcga cccgcacctg cgggttcggc 3360
   gccgagctga tgtcgctggt gcaggagcac tgcttccacc acctggaggc gccgatcgag 3420
   cgcgtcaccg gttgggacac cccctacccg catgcccagg agtgggcgta tttccccggc 3480
   cccgcgcgcg tcggcgcggc attcaagcgt gtgatggagg tctgaatggg taccctcgag 3540
   tctggtaaag aaaccgctgc tgcgaaattt gaacgccagc acatggactc gtctactagc 3600
   gcagcttaat taacctaggc tgctgccacc gctgagcaat aactagcata accccttggg 3660
   gcctctaaac gggtcttgag gggttttttg ctgaaacctc aggcatttga gaagcacacg 3720
   gtcacactgc ttccggtagt caataaaccg gtaaaccagc aatagacata agcggctatt 3780
   taacgaccct gccctgaacc gacgaccggg tcatcgtggc cggatcttgc ggcccctcgg 3840
   cttgaacgaa ttgttagaca ttatttgccg actaccttgg tgatctcgcc tttcacgtag 3900
   tggacaaatt cttccaactg atctgcgcgc gaggccaagc gatcttcttc ttgtccaaga 3960
   taagcctgtc tagcttcaag tatgacgggc tgatactggg ccggcaggcg ctccattgcc 4020
   cagtcggcag cgacatcctt cggcgcgatt ttgccggtta ctgcgctgta ccaaatgcgg 4080
   gacaacgtaa gcactacatt tcgctcatcg ccagcccagt cgggcggcga gttccatagc 4140
   gttaaggttt catttagcgc ctcaaataga tcctgttcag gaaccggatc aaagagttcc 4200
   tccgccgctg gacctaccaa ggcaacgcta tgttctcttg cttttgtcag caagatagcc 4260
   agatcaatgt cgatcgtggc tggctcgaag atacctgcaa gaatgtcatt gcgctgccat 4320
   tctccaaatt gcagttcgcg cttagctgga taacgccacg gaatgatgtc gtcgtgcaca 4380
   acaatggtga cttctacagc gcggagaatc tcgctctctc caggggaagc cgaagtttcc 4440
   aaaaggtcgt tgatcaaagc tcgccgcgtt gtttcatcaa gccttacggt caccgtaacc 4500
   agcaaatcaa tatcactgtg tggcttcagg ccgccatcca ctgcggagcc gtacaaatgt 4560
   acggccagca acgtcggttc gagatggcgc tcgatgacgc caactacctc tgatagttga 4620
   gtcgatactt cggcgatcac cgcttccctc atactcttcc tttttcaata ttattgaagc 4680
   atttatcagg gttattgtct catgagcgga tacatatttg aatgtattta gaaaaataaa 4740
   caaatagcta gctcactcgg tcgctacgct ccgggcgtga gactgcggcg ggcgctgcgg 4800
   acacatacaa agttacccac agattccgtg gataagcagg ggactaacat gtgaggcaaa 4860
   acagcagggc cgcgccggtg gcgtttttcc ataggctccg ccctcctgcc agagttcaca 4920
   taaacagacg cttttccggt gcatctgtgg gagccgtgag gctcaaccat gaatctgaca 4980
   gtacgggcga aacccgacag gacttaaaga tccccaccgt ttccggcggg tcgctccctc 5040
   ttgcgctctc ctgttccgac cctgccgttt accggatacc tgttccgcct ttctccctta 5100
   cgggaagtgt ggcgctttct catagctcac acactggtat ctcggctcgg tgtaggtcgt 5160
   tcgctccaag ctgggctgta agcaagaact ccccgttcag cccgactgct gcgccttatc 5220
   cggtaactgt tcacttgagt ccaacccgga aaagcacggt aaaacgccac tggcagcagc 5280
   cattggtaac tgggagttcg cagaggattt gtttagctaa acacgcggtt gctcttgaag 5340
   tgtgcgccaa agtccggcta cactggaagg acagatttgg ttgctgtgct ctgcgaaagc 5400
   cagttaccac ggttaagcag ttccccaact gacttaacct tcgatcaaac cacctcccca 5460
   ggtggttttt tcgtttacag ggcaaaagat tacgcgcaga aaaaaaggat ctcaagaaga 5520
   tcctttgatc ttttctactg aaccgctcta gatttcagtg caatttatct cttcaaatgt 5580
   agcacctgaa gtcagcccca tacgatataa gttgtaattc tcatgttagt catgccccgc 5640
   gcccaccgga aggagctgac tgggttgaag gctctcaagg gcatcggtcg agatcccggt 5700
   gcctaatgag tgagctaact tacattaatt gcgttgcgct cactgcccgc tttccagtcg 5760
   ggaaacctgt cgtgccagct gcattaatga atcggccaac gcgcggggag aggcggtttg 5820
   cgtattgggc gccagggtgg tttttctttt caccagtgag acgggcaaca gctgattgcc 5880
   cttcaccgcc tggccctgag agagttgcag caagcggtcc acgctggttt gccccagcag 5940
   gcgaaaatcc tgtttgatgg tggttaacgg cgggatataa catgagctgt cttcggtatc 6000
   gtcgtatccc actaccgaga tgtccgcacc aacgcgcagc ccggactcgg taatggcgcg 6060
   cattgcgccc agcgccatct gatcgttggc aaccagcatc gcagtgggaa cgatgccctc 6120
   attcagcatt tgcatggttt gttgaaaacc ggacatggca ctccagtcgc cttcccgttc 6180
   cgctatcggc tgaatttgat tgcgagtgag atatttatgc cagccagcca gacgcagacg 6240
   cgccgagaca gaacttaatg ggcccgctaa cagcgcgatt tgctggtgac ccaatgcgac 6300
   cagatgctcc acgcccagtc gcgtaccgtc ttcatgggag aaaataatac tgttgatggg 6360
   tgtctggtca gagacatcaa gaaataacgc cggaacatta gtgcaggcag cttccacagc 6420
   aatggcatcc tggtcatcca gcggatagtt aatgatcagc ccactgacgc gttgcgcgag 6480
   aagattgtgc accgccgctt tacaggcttc gacgccgctt cgttctacca tcgacaccac 6540
   cacgctggca cccagttgat cggcgcgaga tttaatcgcc gcgacaattt gcgacggcgc 6600
   gtgcagggcc agactggagg tggcaacgcc aatcagcaac gactgtttgc ccgccagttg 6660
   ttgtgccacg cggttgggaa tgtaattcag ctccgccatc gccgcttcca ctttttcccg 6720
   cgttttcgca gaaacgtggc tggcctggtt caccacgcgg gaaacggtct gataagagac 6780
   accggcatac tctgcgacat cgtataacgt tactggtttc acattcacca ccctgaattg 6840
   actctcttcc gggcgctatc atgccatacc gcgaaaggtt ttgcgccatt cgatggtgtc 6900
   cgggatctcg acgctctccc ttatgcgact cctgcattag gaaattaata cgactcacta 6960
<210> 20
   <211> 8757
   <212> DNA
   <213> Artificial
<220>
   <223> New Plasmid
<400> 20
   caaggagatg gcgcccaaca gtcccccggc cacggggcct gccaccatac ccacgccgaa 60
   acaagcgctc atgagcccga agtggcgagc ccgatcttcc ccatcggtga tgtcggcgat 120
   ataggcgcca gcaaccgcac ctgtggcgcc ggtgatgccg gccacgatgc gtccggcgta 180
   gaggatcgag atctcgatcc cgcgaaatta atacgactca ctatagggga attgtgagcg 240
   gataacaatt cccctctaga aataattttg tttaacttta agaaggaatt caggagccct 300
   tatgcaattt aatgaagaac agctattaat tcaggatatg gcgaaaagtt ttgccaatga 360
   acagattaaa tctaatgcag cagaatggga taagcatagc atttttccaa aagacgtttt 420
   gtcccaaatg gggcaattgg gttttatggg aatgctggtg agtgagaaat ggggcggatc 480
   aaatacagga aatttagctt atgtgctggc acttgaagaa atcgctgccg cagatggtgc 540
   gacttcaacc attatgagtg tacataattc tgttggctgt gtacccattg ctaaatttgg 600
   tacagaggag caaaagcaga aatatctagt gcctttagca caaggtgaaa tgatcggtgc 660
   atttgcttta acggaaccac atacaggttc cgatgccgca gccattaaaa cccgagcaat 720
   taaacaaggt gatgaatgga ttattaatgg cgctaaacaa tttataacat caggtcataa 780
   tgcgggcgtg attattgtat ttgctgtgac agatccgaat gcagggaaaa aagggctgag 840
   tgcatttatt gtgccgcgtg aaaccttggg ttatgaggtg attcgcaccg aagaaaaatt 900
   gggtttacat gcgtcagata cgtgccaaat tgctttaacg gatgttcgag tacatcacag 960
   cttaatgctt ggtcaggaag gtgagggact aaaaatagca ttgtctaatc tggaaggtgg 1020
   ccgtattggg attgcagcgc aagccgttgg tttggcacgt gctgcactag aagaagcgac 1080
   aaaatatgcc aaagagcgtg tgacctttgg aaagcctatt tttgagcatc aggcgttagc 1140
   ctttcgttta gccagtatgg ccacagaaat tgaagcagca cgacaattgg ttcattacgc 1200
   agcgcggctt aaagaagctg gaaaaccttg tttaaatgaa gcatcaatgg cgaaattatt 1260
   ttcatctgaa atggtcgaac gcgtatgttc tgctgctttg caaatctttg gtggctatgg 1320
   ctatttaaaa gactttccca tcgagcgaat ttatcgtgat gcacgtattt gccagattta 1380
   tgaaggtaca agtgatattc agcgtttagt gatagcaaga agcctataac tgacctttgc 1440
   tgctgtattt ttatcataaa attaagataa ggattctaaa aatgacattc gcaacaattt 1500
   tattggaaaa acgtaagggt gtgggcttga ttacacttaa ccgtccaaaa gcattaaatg 1560
   ctttaaactc agaattaatt tatgaaataa atttagcctt agacgattta gaaaatgatc 1620
   aaacgattgg ttgtatcgtc cttacaggtt cagaaaaagc ctttgccgca ggtgcggata 1680
   tcaaagaaat ggcagaatta acttttccaa atatttattt tgatgatttt tttagtcttg 1740
   cagatcgtat tgcacagcgt cgtaagcctt taattgccgc agtgagtggt tatgctttag 1800
   gtggtggctg tgagttagca ctcatgtgtg actttattta ttgtgccgac aatgccaagt 1860
   ttgcactacc agaagtaact ttaggtgtca ttcctggtat tggtggaaca cagcgtctaa 1920
   cgcttgcaat aggcaaagcc aaagccatgg aaatgtgttt gactgcacgg caaatgcagg 1980
   ctgctgaggc agaacaaagt ggtttggtgg cacgcgtttt tagtaaagaa gaacttttag 2040
   aacaaacctt acaggctgcc gaaaaaatag cggaaaaatc acgggtatct accataatga 2100
   ttaaagagtc aattaatcga gcttttgaag tgagtttagc agagggttta cgttttgagc 2160
   gccgaatgtt ccattcagtt tttgcgacct tagatcagaa agaaggcatg caagcattta 2220
   ttgataaacg tccagcccaa tttaaacatc aataatagga tgaagcgatg actactactg 2280
   acaatcattt actcattgaa cataaaaacg ctttaggaac aattatttta aatcgtccag 2340
   cgagtctgaa cgcgctatct ctagaaatga ttaatgcgat tcgtcaacaa gttgaggatt 2400
   ggcaaggtga tgtaaatgtt caggccatat taattaaatc aaatagtcct aaagcatttt 2460
   gtgcaggtgg tgatattcgc tatctttatg aaagttataa aagtggatca gaagagtata 2520
   aagattattt cattgctgaa tatgagatgc tcaatagcat tcgaacgtct aaaaaaacag 2580
   tgattgtttt attggatgga tatgtattgg gtggtggttt tggtttagca caggcttgtc 2640
   atatcttggt gagtagtgaa aaatcacgat tttcaatgcc agaaacagca ataggttttt 2700
   tcccagatgt tgcagcgact tatttcttat ctcgtttaga tgatgttggg gtatatttgg 2760
   cactgactgg tgatcaaatc agtagtagtg atgcattgta tttagatctg attgattatc 2820
   atgttccgag tcagaatttt gagcgactag aaaatgcatt cagccaatca cagaacttag 2880
   ataaatttca tattcagaag attatttctg cttatatctc cagccctgtt cagagtgaac 2940
   tcagtctatg gcttgaagcc attcgtcagc attttggtct taaaaatgtg caagatatcg 3000
   aagaaagttt gaaaaatgaa caagatccca actatcaagt atggacaagt aaagtgttaa 3060
   atactttgca acaacgttcc tctattgcaa aaaaaaccag tttaaagtta cagctgctag 3120
   ggcgtggatg gtcattacag caatgtatgc gtatcgagcg aaaattacag gatatctggt 3180
   ttgaacatgg tgatatgatt gagggtgttc gagcgttgat tattgataaa gataaacaac 3240
   cgcaatggca gcagcataat gcgactttag ataatatatt aggccaatta ggttagtcat 3300
   ttcagactga agggcgagct caattcgaag cttgaaggta agcctatccc taaccctctc 3360
   ctcggtctcg attctacgcg taccggtcat catcaccatc accattgagt ttgatccggc 3420
   tgctaacaaa gcccgaaagg aagctgagtt ggctgctgcc accgctgagc aataactagc 3480
   ataacccctt ggggcctcta aacgggtctt gaggggtttt ttgctgaaag gaggaactat 3540
   atccggatat cccgcaagag gcccggcagt accggcataa ccaagcctat gcctacagca 3600
   tccagggtga cggtgccgag gatgacgatg agcgcattgt tagatttcat acacggtgcc 3660
   tgactgcgtt agcaatttaa ctgtgataaa ctaccgcatt aaagcttatc gatgataagc 3720
   tgtcaaacat gagaattaat tcttgaagac gaaagggcct cgtgatacgc ctatttttat 3780
   aggttaatgt catgataata atggtttctt agacgtcagg tggcactttt cggggaaatg 3840
   tgcgcggaac ccctatttgt ttatttttct aaatacattc aaatatgtat ccgctcatga 3900
   gacaataacc ctgataaatg cttcaataat attgaaaaag gaagagtatg agtattcaac 3960
   atttccgtgt cgcccttatt cccttttttg cggcattttg ccttcctgtt tttgctcacc 4020
   cagaaacgct ggtgaaagta aaagatgctg aagatcagtt gggtgcacga gtgggttaca 4080
   tcgaactgga tctcaacagc ggtaagatcc ttgagagttt tcgccccgaa gaacgttttc 4140
   caatgatgag cacttttaaa gttctgctat gtggcgcggt attatcccgt gttgacgccg 4200
   ggcaagagca actcggtcgc cgcatacact attctcagaa tgacttggtt gagtactcac 4260
   cagtcacaga aaagcatctt acggatggca tgacagtaag agaattatgc agtgctgcca 4320
   taaccatgag tgataacact gcggccaact tacttctgac aacgatcgga ggaccgaagg 4380
   agctaaccgc ttttttgcac aacatggggg atcatgtaac tcgccttgat cgttgggaac 4440
   cggagctgaa tgaagccata ccaaacgacg agcgtgacac cacgatgcct gcagcaatgg 4500
   caacaacgtt gcgcaaacta ttaactggcg aactacttac tctagcttcc cggcaacaat 4560
   taatagactg gatggaggcg gataaagttg caggaccact tctgcgctcg gcccttccgg 4620
   ctggctggtt tattgctgat aaatctggag ccggtgagcg tgggtctcgc ggtatcattg 4680
   cagcactggg gccagatggt aagccctccc gtatcgtagt tatctacacg acggggagtc 4740
   aggcaactat ggatgaacga aatagacaga tcgctgagat aggtgcctca ctgattaagc 4800
   attggtaact gtcagaccaa gtttactcat atatacttta gattgattta aaacttcatt 4860
   tttaatttaa aaggatctag gtgaagatcc tttttgataa tctcatgacc aaaatccctt 4920
   aacgtgagtt ttcgttccac tgagcgtcag accccgtaga aaagatcaaa ggatcttctt 4980
   gagatccttt ttttctgcgc gtaatctgct gcttgcaaac aaaaaaacca ccgctaccag 5040
   cggtggtttg tttgccggat caagagctac caactctttt tccgaaggta actggcttca 5100
   gcagagcgca gataccaaat actgtccttc tagtgtagcc gtagttaggc caccacttca 5160
   agaactctgt agcaccgcct acatacctcg ctctgctaat cctgttacca gtggctgctg 5220
   ccagtggcga taagtcgtgt cttaccgggt tggactcaag acgatagtta ccggataagg 5280
   cgcagcggtc gggctgaacg gggggttcgt gcacacagcc cagcttggag cgaacgacct 5340
   acaccgaact gagataccta cagcgtgagc tatgagaaag cgccacgctt cccgaaggga 5400
   gaaaggcgga caggtatccg gtaagcggca gggtcggaac aggagagcgc acgagggagc 5460
   ttccaggggg aaacgcctgg tatctttata gtcctgtcgg gtttcgccac ctctgacttg 5520
   agcgtcgatt tttgtgatgc tcgtcagggg ggcggagcct atggaaaaac gccagcaacg 5580
   cggccttttt acggttcctg gccttttgct ggccttttgc tcacatgttc tttcctgcgt 5640
   tatcccctga ttctgtggat aaccgtatta ccgcctttga gtgagctgat accgctcgcc 5700
   gcagccgaac gaccgagcgc agcgagtcag tgagcgagga agcggaagag cgcctgatgc 5760
   ggtattttct ccttacgcat ctgtgcggta tttcacaccg caatggtgca ctctcagtac 5820
   aatctgctct gatgccgcat agttaagcca gtatacactc cgctatcgct acgtgactgg 5880
   gtcatggctg cgccccgaca cccgccaaca cccgctgacg cgccctgacg ggcttgtctg 5940
   ctcccggcat ccgcttacag acaagctgtg accgtctccg ggagctgcat gtgtcagagg 6000
   ttttcaccgt catcaccgaa acgcgcgagg cagctgcggt aaagctcatc agcgtggtcg 6060
   tgaagcgatt cacagatgtc tgcctgttca tccgcgtcca gctcgttgag tttctccaga 6120
   agcgttaatg tctggcttct gataaagcgg gccatgttaa gggcggtttt ttcctgtttg 6180
   gtcactgatg cctccgtgta agggggattt ctgttcatgg gggtaatgat accgatgaaa 6240
   cgagagagga tgctcacgat acgggttact gatgatgaac atgcccggtt actggaacgt 6300
   tgtgagggta aacaactggc ggtatggatg cggcgggacc agagaaaaat cactcagggt 6360
   caatgccagc gcttcgttaa tacagatgta ggtgttccac agggtagcca gcagcatcct 6420
   gcgatgcaga tccggaacat aatggtgcag ggcgctgact tccgcgtttc cagactttac 6480
   gaaacacgga aaccgaagac cattcatgtt gttgctcagg tcgcagacgt tttgcagcag 6540
   cagtcgcttc acgttcgctc gcgtatcggt gattcattct gctaaccagt aaggcaaccc 6600
   cgccagccta gccgggtcct caacgacagg agcacgatca tgcgcacccg tggccaggac 6660
   ccaacgctgc ccgagatgcg ccgcgtgcgg ctgctggaga tggcggacgc gatggatatg 6720
   ttctgccaag ggttggtttg cgcattcaca gttctccgca agaattgatt ggctccaatt 6780
   cttggagtgg tgaatccgtt agcgaggtgc cgccggcttc cattcaggtc gaggtggccc 6840
   ggctccatgc accgcgacgc aacgcgggga ggcagacaag gtatagggcg gcgcctacaa 6900
   tccatgccaa cccgttccat gtgctcgccg aggcggcata aatcgccgtg acgatcagcg 6960
   gtccaatgat cgaagttagg ctggtaagag ccgcgagcga tccttgaagc tgtccctgat 7020
   ggtcgtcatc tacctgcctg gacagcatgg cctgcaacgc gggcatcccg atgccgccgg 7080
   aagcgagaag aatcataatg gggaaggcca tccagcctcg cgtcgcgaac gccagcaaga 7140
   cgtagcccag cgcgtcggcc gccatgccgg cgataatggc ctgcttctcg ccgaaacgtt 7200
   tggtggcggg accagtgacg aaggcttgag cgagggcgtg caagattccg aataccgcaa 7260
   gcgacaggcc gatcatcgtc gcgctccagc gaaagcggtc ctcgccgaaa atgacccaga 7320
   gcgctgccgg cacctgtcct acgagttgca tgataaagaa gacagtcata agtgcggcga 7380
   cgatagtcat gccccgcgcc caccggaagg agctgactgg gttgaaggct ctcaagggca 7440
   tcggtcgaga tcccggtgcc taatgagtga gctaacttac attaattgcg ttgcgctcac 7500
   tgcccgcttt ccagtcggga aacctgtcgt gccagctgca ttaatgaatc ggccaacgcg 7560
   cggggagagg cggtttgcgt attgggcgcc agggtggttt ttcttttcac cagtgagacg 7620
   ggcaacagct gattgccctt caccgcctgg ccctgagaga gttgcagcaa gcggtccacg 7680
   ctggtttgcc ccagcaggcg aaaatcctgt ttgatggtgg ttaacggcgg gatataacat 7740
   gagctgtctt cggtatcgtc gtatcccact accgagatat ccgcaccaac gcgcagcccg 7800
   gactcggtaa tggcgcgcat tgcgcccagc gccatctgat cgttggcaac cagcatcgca 7860
   gtgggaacga tgccctcatt cagcatttgc atggtttgtt gaaaaccgga catggcactc 7920
   cagtcgcctt cccgttccgc tatcggctga atttgattgc gagtgagata tttatgccag 7980
   ccagccagac gcagacgcgc cgagacagaa cttaatgggc ccgctaacag cgcgatttgc 8040
   tggtgaccca atgcgaccag atgctccacg cccagtcgcg taccgtcttc atgggagaaa 8100
   ataatactgt tgatgggtgt ctggtcagag acatcaagaa ataacgccgg aacattagtg 8160
   caggcagctt ccacagcaat ggcatcctgg tcatccagcg gatagttaat gatcagccca 8220
   ctgacgcgtt gcgcgagaag attgtgcacc gccgctttac aggcttcgac gccgcttcgt 8280
   tctaccatcg acaccaccac gctggcaccc agttgatcgg cgcgagattt aatcgccgcg 8340
   acaatttgcg acggcgcgtg cagggccaga ctggaggtgg caacgccaat cagcaacgac 8400
   tgtttgcccg ccagttgttg tgccacgcgg ttgggaatgt aattcagctc cgccatcgcc 8460
   gcttccactt tttcccgcgt tttcgcagaa acgtggctgg cctggttcac cacgcgggaa 8520
   acggtctgat aagagacacc ggcatactct gcgacatcgt ataacgttac tggtttcaca 8580
   ttcaccaccc tgaattgact ctcttccggg cgctatcatg ccataccgcg aaaggttttg 8640
   cgccattcga tggtgtccgg gatctcgacg ctctccctta tgcgactcct gcattaggaa 8700
   gcagcccagt agtaggttga ggccgttgag caccgccgcc gcaaggaatg gtgcatg 8757
<210> 21
   <211> 28
   <212> DNA
   <213> Artificial Sequence
<220>
   <223> Primer
<400> 21
   attatcccat ggggagaaca ttaaaagc 28
<210> 22
   <211> 24
   <212> DNA
   <213> Artificial Sequence
<220>
   <223> Primer
<400> 22
   cgggatcctt acttttcaat atat 24
<210> 23
   <211> 35
   <212> DNA
   <213> Artificial Sequence
<220>
   <223> Primer
<400> 23
   cggtcgacaa ggagatatag atatgactga tctcacaaag actgc 35
<210> 24
   <211> 23
   <212> DNA
   <213> Artificial Sequence
<220>
   <223> Primer
<400> 24
   cttaggcttt gtcgaacgcc tcc 23
<210> 25
   <211> 870
   <212> DNA
   <213> Artificial
<220>
   <223> New Vector
<400> 25
   atggaaaagg tggcattcat cggcctgggc gcaatgggct acccaatggc aggtcacctg 60
   gctcgccgct tcccaaccct ggtgtggaac cgcaccttcg aaaaggcact gcgccaccag 120
   gaagagttcg gctccgaagc agtgccactg gaacgcgtgg ctgaagcacg cgtgatcttc 180
   acctgcctgc caaccacccg cgaagtgtac gaagtggcag aagcactgta cccatacctg 240
   cgcgaaggca cctactgggt ggatgcaacc tccggcgaac cagaagcatc ccgccgcctg 300
   gctgaacgcc tgcgcgaaaa gggcgtgacc tacctggatg caccagtgtc cggtggcacc 360
   tccggtgcag aagcaggcac cctgaccgtt atgctgggcg gtccagaaga agcagtcgaa 420
   cgcgtccgcc cattcctggc ctacgcaaag aaggtggtcc acgtcggccc agttggtgca 480
   ggccacgcag tgaaggcaat caacaacgca ctgctggccg tgaacctgtg ggcagcaggc 540
   gaaggtctgc tggccctggt gaagcagggc gtgtccgcag aaaaggccct ggaagtgatc 600
   aacgcatcct ccggccgctc caacgcaacc gaaaacctga tcccacagcg cgttctgacc 660
   cgcgcattcc caaagacctt cgcactgggc ctgctggtga aggatctggg catcgcaatg 720
   ggcgtgctgg atggcgaaaa ggcaccatcc ccactgctgc gcctggctcg cgaagtctac 780
   gagatggcaa agcgcgaact cggcccagat gcagatcacg tggaagcact gcgcctgctc 840
   gaacgctggg gcggtgtgga aatccgctaa 870
<210> 26
   <211> 47
   <212> DNA
   <213> Artificial Sequence
<220>
   <223> Primer
<400> 26
   tgaagatcct aggaggttta aacatatgcc gttaatagcc gggattg 47
<210> 27
   <211> 33
   <212> DNA
   <213> Artificial Sequence
<220>
   <223> Primer
<400> 27
   tatatagtcg acttaattcg cctgaccggc cag 33
<210> 28
   <211> 29
   <212> DNA
   <213> Artificial Sequence
<220>
   <223> Primer
<400> 28
   tatataacat gtcgctttca ccgccaggc 29
<210> 29
   <211> 50
   <212> DNA
   <213> Artificial Sequence
<220>
   <223> Primer
<400> 29
   catatgttta aacctcctag gatcttcagt ttctctcact taacggcagg 50
<210> 30
   <211> 29
   <212> DNA
   <213> Artificial Sequence
<220>
   <223> Primer
<400> 30
   tatataccat ggcgctttca ccgccaggc 29
<210> 31
   <211> 33
   <212> DNA
   <213> Artificial Sequence
<220>
   <223> Primer
<400> 31
   tatatagtcg acttaattcg cctgaccggc cag 33
<210> 32
   <211> 40
   <212> DNA
   <213> Artificial Sequence
<220>
   <223> Primer
<400> 32
   tatatacata tgaaaagatc aaaacgattt gcagtactgg 40
<210> 33
   <211> 37
   <212> DNA
   <213> Artificial Sequence
<220>
   <223> Primer
<400> 33
   tatatagtcg acttagcttc ctttacgcag cttatgc 37
<210> 34
   <211> 34
   <212> DNA
   <213> Artificial Sequence
<220>
   <223> Primer
<400> 34
   aaaacatatg aattttcatc atctggctta ctgg 34
<210> 35
   <211> 49
   <212> DNA
   <213> Artificial Sequence
<220>
   <223> Primer
<400> 35
   aaaacatatg tatatttcct tctttcaggc ctccaggctt atccagatg 49
<210> 36
   <211> 30
   <212> DNA
   <213> Artificial Sequence
<220>
   <223> Primer
<400> 36
   ggaggcaacc atggccctcg acgtgcagag 30
<210> 37
   <211> 32
   <212> DNA
   <213> Artificial Sequence
<220>
   <223> Primer
<400> 37
   gagacttgcg gatccctccg atcaggcctt gc 32
<210> 38
   <211> 30
   <212> DNA
   <213> Artificial Sequence
<220>
   <223> Primer
<400> 38
   aaaacatatg atcgacactg cgccccttgc 30
<210> 39
   <211> 28
   <212> DNA
   <213> Artificial Sequence
<220>
   <223> Primer
<400> 39
   aagacgtcct accgctcgcc ggccgtcc 28
<210> 40
   <211> 3967
   <212> DNA
   <213> Artificial Sequence
<220>
   <223> New Plasmid
<400> 40
   atgcgccgca ccctgaaggc agcaatcctg ggcgccaccg gcctggtggg catcgaatac 60
   gtgcgcatgc tgtccaacca cccatacatc aagccagcat acctggccgg caagggctcc 120
   gttggcaagc catacggcga agtggtgcgc tggcagaccg tgggccaggt gccaaaggaa 180
   atcgcagata tggaaatcaa gccaaccgat ccaaagctga tggatgatgt ggatatcatc 240
   ttctccccac tgccacaggg tgcagcaggc ccagtggaag aacagttcgc aaaggaaggc 300
   ttcccagtga tctccaactc cccagatcac cgcttcgatc cagatgtgcc actgctggtg 360
   ccagaactca acccacacac catctccctg atcgatgaac agcgcaagcg ccgcgaatgg 420
   aagggcttca tcgtgaccac cccactgtgc accgcacagg gcgcagcaat cccactgggc 480
   gcaatcttca aggattacaa gatggatggc gcattcatca ccaccatcca gtccctgtcc 540
   ggcgcaggct acccaggtat cccatccctg gatgtggtgg ataacatcct gccactgggc 600
   gatggctacg atgcaaagac catcaaggaa atcttccgca tcctgtccga agtgaagcgc 660
   aacgtggatg aaccaaagct ggaagatgtg tccctggccg caaccaccca ccgcatcgca 720
   accatccacg gccactacga agtgctgtac gtgtccttca aggaagaaac cgcagcagaa 780
   aaggtgaagg aaaccctgga aaacttccgc ggcgaaccac aggatctgaa gctgccaacc 840
   gcaccatcca agccaatcat cgtgatgaac gaagataccc gcccacaggt gtacttcgat 900
   cgctgggcag gcgatatccc aggcatgtcc gtggtggtgg gccgcctgaa gcaggtgaac 960
   aagcgcatga tccgcctggt gtccctgatc cacaacaccg ttcgcggcgc agcaggtggt 1020
   ggtatcctgg ccgcagaact cctggtggaa aagggctaca tcgaaaagta agaattccgc 1080
   gagctccagc ttttgttccc tttagtgagg gttaattgcg cgcttggcgt aatcatggtc 1140
   atagctgttt cctgtgtgaa attgttatcc gctcacaatt ccacacaaca tacgagccgg 1200
   aagcataaag tgtaaagcct ggggtgccta atgagtgagc taactcacat taattgcgtt 1260
   gcgctcactg cccgctttcc agtcgggaaa cctgtcgtgc cagctgcatt aatgaatcgg 1320
   ccaacgcgcg gggagaggcg gtttgcgtat tgggcgctct tccgcttcct cgctcactga 1380
   ctcgctgcgc tcggtcgttc ggctgcggcg agcggtatca gctcactcaa aggcggtaat 1440
   acggttatcc acagaatcag gggataacgc aggaaagaac atgtgagcaa aaggccagca 1500
   aaaggccagg aaccgtaaaa aggccgcgtt gctggcgttt ttccataggc tccgcccccc 1560
   tgacgagcat cacaaaaatc gacgctcaag tcagaggtgg cgaaacccga caggactata 1620
   aagataccag gcgtttcccc ctggaagctc cctcgtgcgc tctcctgttc cgaccctgcc 1680
   gcttaccgga tacctgtccg cctttctccc ttcgggaagc gtggcgcttt ctcatagctc 1740
   acgctgtagg tatctcagtt cggtgtaggt cgttcgctcc aagctgggct gtgtgcacga 1800
   accccccgtt cagcccgacc gctgcgcctt atccggtaac tatcgtcttg agtccaaccc 1860
   ggtaagacac gacttatcgc cactggcagc agccactggt aacaggatta gcagagcgag 1920
   gtatgtaggc ggtgctacag agttcttgaa gtggtggcct aactacggct acactagaag 1980
   aacagtattt ggtatctgcg ctctgctgaa gccagttacc ttcggaaaaa gagttggtag 2040
   ctcttgatcc ggcaaacaaa ccaccgctgg tagcggtggt ttttttgttt gcaagcagca 2100
   gattacgcgc agaaaaaaag gatctcaaga agatcctttg atcttttcta cggggtctga 2160
   cgctcagtgg aacgaaaact cacgttaagg gattttggtc atgagattat caaaaaggat 2220
   cttcacctag atccttttaa attaaaaatg aagttttaaa tcaatctaaa gtatatatga 2280
   gtaaacttgg tctgacagtt accaatgctt aatcagtgag gcacctatct cagcgatctg 2340
   tctatttcgt tcatccatag ttgcctgact ccccgtcgtg tagataacta cgatacggga 2400
   gggcttacca tctggcccca gtgctgcaat gataccgcga gaaccacgct caccggctcc 2460
   agatttatca gcaataaacc agccagccgg aagggccgag cgcagaagtg gtcctgcaac 2520
   tttatccgcc tccatccagt ctattaattg ttgccgggaa gctagagtaa gtagttcgcc 2580
   agttaatagt ttgcgcaacg ttgttgccat tgctacaggc atcgtggtgt cacgctcgtc 2640
   gtttggtatg gcttcattca gctccggttc ccaacgatca aggcgagtta catgatcccc 2700
   catgttgtgc aaaaaagcgg ttagctcctt cggtcctccg atcgttgtca gaagtaagtt 2760
   ggccgcagtg ttatcactca tggttatggc agcactgcat aattctctta ctgtcatgcc 2820
   atccgtaaga tgcttttctg tgactggtga gtactcaacc aagtcattct gagaatagtg 2880
   tatgcggcga ccgagttgct cttgcccggc gtcaatacgg gataataccg cgccacatag 2940
   cagaacttta aaagtgctca tcattggaaa acgttcttcg gggcgaaaac tctcaaggat 3000
   cttaccgctg ttgagatcca gttcgatgta acccactcgt gcacccaact gatcttcagc 3060
   atcttttact ttcaccagcg tttctgggtg agcaaaaaca ggaaggcaaa atgccgcaaa 3120
   aaagggaata agggcgacac ggaaatgttg aatactcata ctcttccttt ttcaatatta 3180
   ttgaagcatt tatcagggtt attgtctcat gagcggatac atatttgaat gtatttagaa 3240
   aaataaacaa ataggggttc cgcgcacatt tccccgaaaa gtgccaccta aattgtaagc 3300
   gttaatattt tgttaaaatt cgcgttaaat ttttgttaaa tcagctcatt ttttaaccaa 3360
   taggccgaaa tcggcaaaat cccttataaa tcaaaagaat agaccgagat agggttgagt 3420
   gttgttccag tttggaacaa gagtccacta ttaaagaacg tggactccaa cgtcaaaggg 3480
   cgaaaaaccg tctatcaggg ctatggccca ctacgtgaac catcacccta atcaagtttt 3540
   ttggggtcga ggtgccgtaa agcactaaat cggaacccta aagggagccc ccgatttaga 3600
   gcttgacggg gaaagccggc gaacgtggcg agaaaggaag ggaagaaagc gaaaggagcg 3660
   ggcgctaggg cgctggcaag tgtagcggtc acgctgcgcg taaccaccac acccgccgcg 3720
   cttaatgcgc cgctacaggg cgcgtcccat tcgccattca ggctgcgcaa ctgttgggaa 3780
   gggcgatcgg tgcgggcctc ttcgctatta cgccagctgg cgaaaggggg atgtgctgca 3840
   aggcgattaa gttgggtaac gccagggttt tcccagtcac gacgttgtaa aacgacggcc 3900
   agtgagcgcg cgtaatacga ctcactatag ggcgaattgg gtaccgcgga tccaaggaga 3960
   tatagat
<210> 41
   <211> 23
   <212> DNA
   <213> Artificial Sequence
<220>
   <223> Primer
<400> 41
   aaccatgggc cgcaccctga agg 23
<210> 42
   <211> 33
   <212> DNA
   <213> Artificial Sequence
<220>
   <223> Primer
<400> 42
   aaggatcctt acttttcgat gtagcccttt tcc 33
<210> 43
   <211> 3766
   <212> DNA
   <213> Artificial Sequence
<220>
   <223> New Plasmid
<400> 43
   atggaaaagg tggcattcat cggcctgggc gcaatgggct acccaatggc aggtcacctg 60
   gctcgccgct tcccaaccct ggtgtggaac cgcaccttcg aaaaggcact gcgccaccag 120
   gaagagttcg gctccgaagc agtgccactg gaacgcgtgg ctgaagcacg cgtgatcttc 180
   acctgcctgc caaccacccg cgaagtgtac gaagtggcag aagcactgta cccatacctg 240
   cgcgaaggca cctactgggt ggatgcaacc tccggcgaac cagaagcatc ccgccgcctg 300
   gctgaacgcc tgcgcgaaaa gggcgtgacc tacctggatg caccagtgtc cggtggcacc 360
   tccggtgcag aagcaggcac cctgaccgtt atgctgggcg gtccagaaga agcagtcgaa 420
   cgcgtccgcc cattcctggc ctacgcaaag aaggtggtcc acgtcggccc agttggtgca 480
   ggccacgcag tgaaggcaat caacaacgca ctgctggccg tgaacctgtg ggcagcaggc 540
   gaaggtctgc tggccctggt gaagcagggc gtgtccgcag aaaaggccct ggaagtgatc 600
   aacgcatcct ccggccgctc caacgcaacc gaaaacctga tcccacagcg cgttctgacc 660
   cgcgcattcc caaagacctt cgcactgggc ctgctggtga aggatctggg catcgcaatg 720
   ggcgtgctgg atggcgaaaa ggcaccatcc ccactgctgc gcctggctcg cgaagtctac 780
   gagatggcaa agcgcgaact cggcccagat gcagatcacg tggaagcact gcgcctgctc 840
   gaacgctggg gcggtgtgga aatccgctaa gaattccgcg agctccagct tttgttccct 900
   ttagtgaggg ttaattgcgc gcttggcgta atcatggtca tagctgtttc ctgtgtgaaa 960
   ttgttatccg ctcacaattc cacacaacat acgagccgga agcataaagt gtaaagcctg 1020
   gggtgcctaa tgagtgagct aactcacatt aattgcgttg cgctcactgc ccgctttcca 1080
   gtcgggaaac ctgtcgtgcc agctgcatta atgaatcggc caacgcgcgg ggagaggcgg 1140
   tttgcgtatt gggcgctctt ccgcttcctc gctcactgac tcgctgcgct cggtcgttcg 1200
   gctgcggcga gcggtatcag ctcactcaaa ggcggtaata cggttatcca cagaatcagg 1260
   ggataacgca ggaaagaaca tgtgagcaaa aggccagcaa aaggccagga accgtaaaaa 1320
   ggccgcgttg ctggcgtttt tccataggct ccgcccccct gacgagcatc acaaaaatcg 1380
   acgctcaagt cagaggtggc gaaacccgac aggactataa agataccagg cgtttccccc 1440
   tggaagctcc ctcgtgcgct ctcctgttcc gaccctgccg cttaccggat acctgtccgc 1500
   ctttctccct tcgggaagcg tggcgctttc tcatagctca cgctgtaggt atctcagttc 1560
   ggtgtaggtc gttcgctcca agctgggctg tgtgcacgaa ccccccgttc agcccgaccg 1620
   ctgcgcctta tccggtaact atcgtcttga gtccaacccg gtaagacacg acttatcgcc 1680
   actggcagca gccactggta acaggattag cagagcgagg tatgtaggcg gtgctacaga 1740
   gttcttgaag tggtggccta actacggcta cactagaaga acagtatttg gtatctgcgc 1800
   tctgctgaag ccagttacct tcggaaaaag agttggtagc tcttgatccg gcaaacaaac 1860
   caccgctggt agcggtggtt tttttgtttg caagcagcag attacgcgca gaaaaaaagg 1920
   atctcaagaa gatcctttga tcttttctac ggggtctgac gctcagtgga acgaaaactc 1980
   acgttaaggg attttggtca tgagattatc aaaaaggatc ttcacctaga tccttttaaa 2040
   ttaaaaatga agttttaaat caatctaaag tatatatgag taaacttggt ctgacagtta 2100
   ccaatgctta atcagtgagg cacctatctc agcgatctgt ctatttcgtt catccatagt 2160
   tgcctgactc cccgtcgtgt agataactac gatacgggag ggcttaccat ctggccccag 2220
   tgctgcaatg ataccgcgag aaccacgctc accggctcca gatttatcag caataaacca 2280
   gccagccgga agggccgagc gcagaagtgg tcctgcaact ttatccgcct ccatccagtc 2340
   tattaattgt tgccgggaag ctagagtaag tagttcgcca gttaatagtt tgcgcaacgt 2400
   tgttgccatt gctacaggca tcgtggtgtc acgctcgtcg tttggtatgg cttcattcag 2460
   ctccggttcc caacgatcaa ggcgagttac atgatccccc atgttgtgca aaaaagcggt 2520
   tagctccttc ggtcctccga tcgttgtcag aagtaagttg gccgcagtgt tatcactcat 2580
   ggttatggca gcactgcata attctcttac tgtcatgcca tccgtaagat gcttttctgt 2640
   gactggtgag tactcaacca agtcattctg agaatagtgt atgcggcgac cgagttgctc 2700
   ttgcccggcg tcaatacggg ataataccgc gccacatagc agaactttaa aagtgctcat 2760
   cattggaaaa cgttcttcgg ggcgaaaact ctcaaggatc ttaccgctgt tgagatccag 2820
   ttcgatgtaa cccactcgtg cacccaactg atcttcagca tcttttactt tcaccagcgt 2880
   ttctgggtga gcaaaaacag gaaggcaaaa tgccgcaaaa aagggaataa gggcgacacg 2940
   gaaatgttga atactcatac tcttcctttt tcaatattat tgaagcattt atcagggtta 3000
   ttgtctcatg agcggataca tatttgaatg tatttagaaa aataaacaaa taggggttcc 3060
   gcgcacattt ccccgaaaag tgccacctaa attgtaagcg ttaatatttt gttaaaattc 3120
   gcgttaaatt tttgttaaat cagctcattt tttaaccaat aggccgaaat cggcaaaatc 3180
   ccttataaat caaaagaata gaccgagata gggttgagtg ttgttccagt ttggaacaag 3240
   agtccactat taaagaacgt ggactccaac gtcaaagggc gaaaaaccgt ctatcagggc 3300
   tatggcccac tacgtgaacc atcaccctaa tcaagttttt tggggtcgag gtgccgtaaa 3360
   gcactaaatc ggaaccctaa agggagcccc cgatttagag cttgacgggg aaagccggcg 3420
   aacgtggcga gaaaggaagg gaagaaagcg aaaggagcgg gcgctagggc gctggcaagt 3480
   gtagcggtca cgctgcgcgt aaccaccaca cccgccgcgc ttaatgcgcc gctacagggc 3540
   gcgtcccatt cgccattcag gctgcgcaac tgttgggaag ggcgatcggt gcgggcctct 3600
   tcgctattac gccagctggc gaaaggggga tgtgctgcaa ggcgattaag ttgggtaacg 3660
   ccagggtttt cccagtcacg acgttgtaaa acgacggcca gtgagcgcgc gtaatacgac 3720
   tcactatagg gcgaattggg taccgcggat ccaaggagat atagat 3766
<210> 44
   <211> 29
   <212> DNA
   <213> Artificial Sequence
<220>
   <223> Primer
<400> 44
   aaaacatatg gaaaaggtgg cattcatcg 29
<210> 45
   <211> 30
   <212> DNA
   <213> Artificial Sequence
<220>
   <223> Primer
<400> 45
   aaaagatctt tagcggattt ccacaccgcc 30

## Beschreibung

Die vorliegende Erfindung betrifft ein Verfahren zur Herstellung von Methacrylsäure oder Methacrylsäureestern sowie ein Verfahren zur Herstellung von Polymethacrylsäure oder Polymethacrylsäureestern.

Methacrylsäure ist ein bedeutendes Zwischenprodukt, das insbesondere in Form seiner Alkylester zur Herstellung von Polymerisaten Verwendung findet. Ein bekanntes Methacrylsäurederivat ist zum Beispiel der Methylester der Methacrylsäure. Derzeit beträgt die Weltjahresproduktion an Methacrylsäuremethylester ca. 1,5 Millionen Tonnen. Die Polymethacrylsäureester sind vielfältig einsetzbare Grundstoffe auf dem Kunststoffsektor.

Die kommerzielle Produktion von Methacrylsäure erfolgt üblicherweise durch heterogene, zweistufig katalysierte Gasphasenoxidation von C₄-Kohlenstoffverbindungen wie Butylen, Isobutylen, Butan, iso-Butan, t-Butylalkohol oder Methacrolein an festen Multimetalloxidmassen als Katalysator. Das dabei erhaltene Produktgasgemisch, welches neben der Methacrylsäure noch zahlreiche Nebenprodukte enthält, wird anschließend entweder einer Totalkondensation unter Erhalt einer wässrigen Methacrylsäurelösung unterzogen oder in einem geeigneten Lösungsmittelgemisch absorbiert. Danach folgt üblicherweise eine weitere Aufreinigung der so erhaltenen flüssigen Phasen durch Destillation, Kristallisation, Extraktion oder durch eine Kombination dieser Maßnahmen. Neben der katalytischen Gasphasenoxidation von C₄-Kohlenstoffverbindungen kann Methacrylsäure auch durch eine katalytische, oxidative Dehydrogenierung aus Isobuttersäure gebildet werden, wie dies beispielsweise in der EP-A-0 356 315 beschrieben wird. Eine weitere Möglichkeit zur Herstellung von Methacrylsäure bietet der sogenannte "ACH-Prozess", bei dem Acetoncyanohydrin und Schwefelsäure unter Bildung von Methacrylamid als Zwischenprodukt umgesetzt werden, welches dann mit Wasser zur Methacrylsäure weiterreagiert. Anschließend erfolgt eine destillative Aufreinigung der so erhaltenen Methacrylsäure. Dieses Verfahren ist beispielsweise in der EP-A-1 359 137 beschrieben.

EP1186592 beschreibt ein Verfahren zur Produktion von Methacryläten, bei dem α-Hydroxyisobuttersäure mit einem Alkohol in Anwesenheit eines festen Katalysators reagiert. US 3,562,320 hingegen zeigt die Produktion von Methacrylsäure in einem Verfahren, bei dem die α-Hydroxyisobuttersäure unter Einfluss von Hitze und einem Metall-Salz-Katalysator dehydriert wird.

Der Nachteil dieser herkömmlichen Verfahren zur Herstellung von Methacrylsäure besteht unter anderem darin, dass es sowohl bei der Herstellung der Methacrylsäure selbst als auch bei den nachfolgenden, destillativen Aufreingungsschritten aufgrund der hohen Polymerisationsneigung der Methacrylsäure durch die thermisch belastenden Verfahrensschritte zu einer Bildung von Dimeren oder Oligomeren kommt, was neben dem zusätzlichen Aufreinigungsaufwand auch mit einem Ausbeuteverlust verbunden ist.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, ein Verfahren zur Herstellung von Methacrylsäure anzugeben, bei dem mit möglichst wenig thermisch belastenden Schritten die Methacrylsäure erhalten werden kann.

Auch sollte es dieses Verfahren ermöglichen, die Methacrylsäure aus nachwachsenden Rohstoffen, insbesondere aus Kohlehydraten und/oder aus Glycerin herzustellen.

Einen Beitrag zur Lösung der vorstehend genannten Aufgaben leistet ein Verfahren zur Herstellung von Methacrylsäure oder Methacrylsäureestern, umfassend die Verfahrensschritte
IA) Herstellung von 3-Hydroxyisobuttersäure durch ein Verfahren umfassend den Verfahrensschritt des in Kontakt bringens einer Zelle, welche gegenüber ihrem Wildtyp derart gentechnisch verändert wurde, dass sie im Vergleich zu ihrem Wildtyp mehr 3-Hydroxyisobuttersäure oder auf 3-Hydroxyisobuttersäure basierende Polyhydroxyalkanoate zu bilden vermag, mit einem Nährmedium beinhaltend als Kohlenstoffquelle Kohlenhydrate, Glycerin, Kohlendioxid, Methan, Methanol, L-Valin oder L-Glutamat unter Bedingungen, unter denen aus der Kohlenstoffquelle 3-Hydroxyisobuttersäure oder auf 3-Hydroxyisobuttersäure basierende Polyhydroxyalkanoate gebildet werden, gegebenenfalls Aufreinigung der 3-Hydroxyisobuttersäure aus dem Nährmedium sowie gegebenenfalls Neutralisation der 3-Hydroxyisobuttersäure,wobei die Bildung der 3-Hydroxyisobuttersäure oder der auf 3-Hydroxyisobuttersäure basierenden Polyhydroxyalkanoate vorzugsweise über Methylmalonat-Semialdehyd oder über 3-Hydroxyisobutyryl-Coenzym A als Vorprodukt erfolgt;
IB) Dehydratisierung der 3-Hydroxyisobuttersäure unter Bildung von Methacrylsäure sowie gegebenenfalls Veresterung Methacrylsäure.

Sofern die Bildung von 3-Hydroxyisobuttersäure oder von auf 3-Hydroxyisobuttersäure basierenden Polyhydroxyalkanoaten über Methylmalonat-Semialdehyd als Vorprodukt erfolgt, ist es weiterhin bevorzugt, dass die Bildung über Succinyl-Coenzym A, Propionyl-Coenzym A oder Acryloyl-Coenzym A, besonders bevorzugt über Succinyl-Coenzym A als weiteres Zwischenprodukt erfolgt. Sofern die Bildung von 3-Hydroxyisobuttersäure oder von auf 3-Hydroxyisobuttersäure basierenden Polyhydroxyalkanoaten über 3-Hydroxyisobutyryl-Coenzym A als Vorprodukt erfolgt, ist es weiterhin bevorzugt, dass die Bildung über Isobutyryl-Coenzym A oder über 3-Hydroxybutyryl-Coenzym A, vorzugsweise über 3-Hydroxybutyryl-Coenzym A als weiteres Zwischenprodukt erfolgt.

Der Begriff "Vorprodukt", wie er hierin verwendet wird, definiert eine chemische Verbindung, die in nur einem einzigen Reaktionsschritt auf enzymatischem Weg zu 3-Hydroxyisobuttersäure umgesetzt werden kann, während der Begriff "Zwischenprodukt" eine chemische Verbindung definiert, die nicht in nur einem Reaktionsschritt auf enzymatischem Weg zu 3-Hydroxyisobuttersäure umgesetzt werden kann.

Der Begriff "3-Hydroxyisobuttersäure", wie er hierin verwendet wird, beschreibt stets die entsprechende C₄-Carbonsäure in derjenigen Form, in der sie nach Bildung durch die entsprechenden Mikroorganismen in Abhängigkeit vom pH-Wert vorliegt. Der Begriff umfasst somit stets die reine Säureform (3-Hydroxyisobuttersäure), die reine Basenform (3-Hydroxyisobutyrat) sowie Mischungen aus protonierter und deprotonierter Form der Säure. Weiterhin umfasst der Begriff "3-Hydroxyisobuttersäure" grundsätzlich sowohl das (R)- als auch das (S)-Stereoisomer, wobei das (S)-Stereoisomer besonders bevorzugt ist.

Die Formulierung "dass sie im Vergleich zu ihrem Wildtyp mehr 3-Hydroxyisobuttersäure oder auf 3-Hydroxyisobuttersäure basierende Polyhydroxyalkanoate zu bilden vermag" betrifft auch den Fall, dass der Wildtyp der gentechnisch veränderten Zelle überhaupt keine 3-Hydroxyisobuttersäure oder keine auf 3-Hydroxyisobuttersäure basierenden Polyhydroxyalkanoate, zumindest aber keine nachweisbaren Mengen dieser Verbindungen zu bilden vermag und erst nach der gentechnischen Veränderung nachweisbare Mengen dieser Komponenten gebildet werden können.

Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Aus der 3-Hydroxyisobuttersäure wird anschließend durch eine schonende Dehydratisierungsreaktion Methacrylsäure erhalten. Im Falle von auf 3-Hydroxyisobuttersäure basierenden Polyhydroxyalkanoaten können die in den Zellen enthaltenen Vesikel, welche mit diesen Polyhydroxyalkanoaten gefüllt sind, isoliert und anschließend die Polymere unter Erhalt von 3-Hydroxyisobuttersäure gespalten werden, welche dann unter Erhalt von Methacrylsäure dehydratisiert werden kann.

Dabei ist es erfindungsgemäß bevorzugt, dass die in dem erfindungsgemäßen Verfahren eingesetzte, gentechnisch veränderte Zelle derart gentechnisch verändert ist, dass sie in einem definierten Zeitintervall, vorzugsweise innerhalb von 2 Stunden, noch mehr bevorzugt innerhalb von 8 Stunden und am meisten bevorzugt innerhalb von 24 Stunden, mindestens 2mal, besonders bevorzugt mindestens 10mal, darüber hinaus bevorzugt mindestens 100mal, darüber hinaus noch mehr bevorzugt mindestens 1.000mal und am meisten bevorzugt mindestens 10.000mal mehr 3-Hydroxyisobuttersäure oder auf 3-Hydroxyisobuttersäure basierende Polyhydroxyalkanoate bildet als der Wildtyp der Zelle. Die Zunahme der Produktbildung kann dabei beispielsweise dadurch bestimmt werden, dass die in dem erfindungsgemäßen Verfahren eingesetzte Zelle und die Wildtyp-Zelle jeweils getrennt unter gleichen Bedingungen (gleiche Zelldichte, gleiches Nährmedium, gleiche Kulturbedingungen) für ein bestimmtes Zeitintervall in einem geeigneten Nährmedium kultiviert werden und anschlie-βend die Menge an Zielprodukt (3-Hydroxyisobuttersäure oder auf 3-Hydroxyisobuttersäure basierende Polyhydroxyalkanoate) im Nährmedium bestimmt wird.

Die in dem erfindungsgemäßen Verfahren eingesetzten Zellen können Prokaryonten oder Eukaryonten sein. Dabei kann es sich um Säugetierzellen (wie etwa Zellen aus dem Menschen), um pflanzliche Zellen oder um Mikroorganismen wie Hefen, Pilze oder Bakterien handeln, wobei Mikroorganismen besonders bevorzugt und Bakterien und Hefen am meisten bevorzugt sind.

Als Bakterien, Hefen oder Pilze sind insbesondere diejenigen Bakterien, Hefen oder Pilze geeignet, die bei der Deutschen Sammlung von Mikroorganismen und Zellkulturen GmbH (DSMZ), Braunschweig, Deutschland, als Bakterien-, Hefe-oder Pilz-Stämme hinterlegt sind.

Erfindungsgemäß geeignete Bakterien gehören zu den Gattungen, die unter
http://www.dsmz.de/species/bacteria.htm
aufgeführt sind, erfindungsgemäß geeignete Hefen gehören zu denjenigen Gattungen, die unter
http://www.dsmz.de/species/yeasts.htm
aufgeführt sind und erfindungsgemäß geeignete Pilze sind diejenigen, die unter
http://www.dsmz.de/species/fungi.htm
aufgeführt sind.

Erfindungsgemäß besonders bevorzugt werden Zellen der Gattungen *Corynebacterium, Brevibacterium, Bacillus, Acinetobacter, Lactobacillus, Lactococcus, Candida, Pichia, Kluveromyces, Saccharomyces, Escherichia, Zymomonas, Yarrowia, Methylobacterium, Ralstonia, Pseudomonas, Burkholderia* und *Clostridium,* wobei *Brevibacterium flavum, Brevibacterium lactofermentum, Escherichia coli, Saccharomyces cerevisiae, Kluveromyces lactis, Candida blankii, Candida rugosa, Corynebacterium glutamicum, Corynebacterium efficiens, Zymonomas mobilis, Yarrowia lipolytica, Methylobacterium extorquens, Ralstonia eutropha, insbesondere Ralstonia eutropha H16, Rhodospirillum rubrum, Rhodobacter sphaeroides, Paracoccus versutus, Pseudomonas aeroginosa, Acinetobacter calcoaceticus* oder *Pichia pastoris* eingesetzt.

Gemäß einer ersten Variante des erfindungsgemäßen Verfahrens wird eine Zelle eingesetzt, bei der die Bildung von 3-Hydroxyisobuttersäure oder von auf 3-Hydroxyisobuttersäure basierenden Polyhydroxyalkanoaten über Methylmalonat-Semialdehyd als Vorprodukt erfolgt.

Gemäß einer ersten besonderen Ausführungsform dieser ersten Variante des erfindungsgemäßen Verfahrens ist es bevorzugt, dass die Bildung der 3-Hydroxyisobuttersäure oder des auf 3-Hydroxyisobuttersäure basierenden Polyhydroxyalkanoates vorzugsweise über Succinyl-Coenzym A als Zwischenprodukt erfolgt, wobei die in dieser Ausführungsform des erfindungsgemäßen Verfahrens eingesetzte, gentechnisch veränderte Zelle vorzugsweise Kohlenhydrate, Glycerin oder Glutamat als Kohlenstoffquelle zu verwerten vermag.

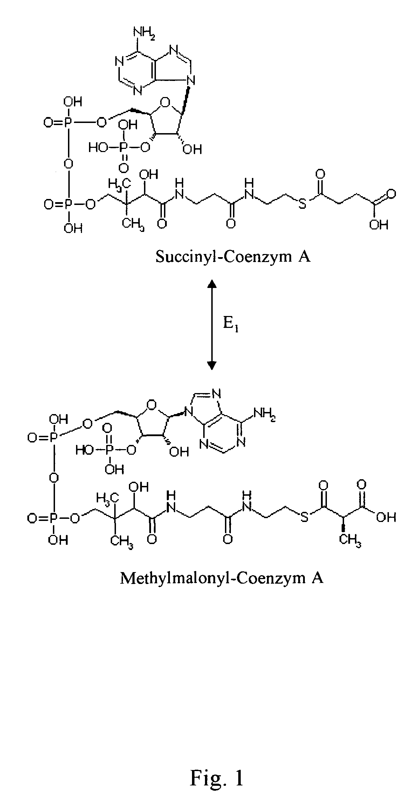

Dabei kann es im Zusammenhang mit der ersten besonderen Ausführungsform der ersten Variante des erfindungsgemäßen Verfahrens vorteilhaft sein, dass die in dieser Ausführungsform des erfindungsgemäßen Verfahrens eingesetzte, gentechnisch veränderte Zelle im Vergleich zu ihrem Wildtyp eine gesteigerte Aktivität eines Enzyms E₁ aufweist, welches die Umsetzung von Succinyl-Coenzym A zu Methylmalonyl-Coenzym A katalysiert (siehe die Figur 1).

Der Begriff "gesteigerte Aktivität eines Enzyms", wie er vorstehend im Zusammenhang mit dem Enzym E₁ und in den nachfolgenden Ausführungen im Zusammenhang mit den Enzymen E₂ usw. verwendet wird, ist vorzugsweise als gesteigerte intrazelluläre Aktivität zu verstehen.

Die nun folgenden Ausführungen zur Erhöhung der Enzymaktivität in Zellen gelten sowohl für die Erhöhung der Aktivität des Enzyms E₁ als auch für alle nachfolgend genannten Enzyme, deren Aktivität gegebenenfalls erhöht werden kann. Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder einer Kombination dieser Methoden mit einem Vektor erzeugt, der das gewünschte Gen, ein Allel dieses Gens oder Teile davon und einen die Expression des Gens ermöglichenden Vektor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der Enzym-Aktivität in Zellen am Beispiel der Pyruvat-Carboxylase gibt DE-A-100 31 999.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden gentechnisch veränderte Zellen erhalten. Besonders bevorzugte Mutanten von Enzymen sind insbesondere auch solche Enzyme, die nicht mehr oder zumindest im Vergleich zum Wildtyp-Enzym vermindert *feedback*-inhibierbar sind.

Wird die Erhöhung der Enzymaktivität durch Erhöhung der Expression eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "*Enhancer*" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Zur Erhöhung der Expression der jeweiligen Gene werden zum Beispiel episomale Plasmide eingesetzt. Als Plasmide eignen sich insbesondere solche, die in coryneformen Bakterien repliziert werden. Zahlreiche bekannte Plasmidvektoren, wie zum Beispiel pZ1 (Menkel et al., Applied and Environmental Microbiology 64: 549-554 (1989)), pEKEx1 (Eikmanns et al., Gene 107: 69-74 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie zum Beispiel solche, die auf pCG4 (US 4,489,160) oder pNG2 (Serwold-Davis et al., FEMS Microbiology Letters 66: 119-124 (1990)) oder pAG1 (US 5,158,891) beruhen, können in gleicher Weise eingesetzt werden.

Weiterhin eignen sich auch solche Plasmidvektoren, mit Hilfe derer man das Verfahren der Genamplifikation durch Integration in das Chromosom anwenden kann, so wie es beispielsweise von Reinscheid et al. (Applied and Environmental Microbiology 60: 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise *Escherichia coli*), nicht aber in *Corynebacterium glutamicum* repliziert werden kann. Als Vektoren kommen beispielsweise pSUP301 (Simon et al., Bio/Technology 1: 784-791 (1983)), pK18mob oder pK19mob (Schäfer et al., Gene 145: 69-73 (1994)), pGEM-T (Promega Corporation, Madison, Wisconsin, USA), pCR2.1-TOPO (Shuman, Journal of Biological Chemistry 269: 32678-84 (1994)), pCR^{®}Blunt (Invitrogen, Groningen, Niederlande), pEM1 (Schrumpf et al., Journal of Bacteriology 173: 4510-4516)) oder pBGS8 (Spratt et al., Gene 41: 337-342 (1986)) in Frage. Der Plasmidvektor, der das zu amplifizierende Gen enthält, wird anschließend durch Konjugation oder Transformation in den gewünschten Stamm von *Corynebacterium glutamicum* überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Letters 123: 343-347 (1994) beschrieben. Nach homologer Rekombination mittels eines "cross-over"-Ereignisses enthält der resultierende Stamm mindestens zwei Kopien des betreffenden Gens.

Unter der vorstehend und in den nachfolgenden Ausführungen verwendeten Formulierung "eine gegenüber ihrem Wildtyp gesteigerte Aktivität eines Enzyms Eₓ" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die in dem erfindungsgemäßen Verfahren eingesetzte, gentechnisch veränderte Zelle, welche "eine gegenüber ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms Eₓ induziert wird.

Unter der nachfolgend verwendeten Formulierung "verminderte Aktivität eines Enzyms Eₓ" wird dementsprechend vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verminderte Aktivität verstanden. Die Verminderung der Aktivität eines bestimmten Enzyms kann beispielsweise durch gezielte Mutation, durch Zugabe von kompetitven oder nicht kompetitiven Inhibitoren oder durch andere, dem Fachmann bekannte Maßnahmen zur Verminderung der Expression eines bestimmten Enzyms erfolgen.

Bei dem Enzym E₁, welches die Umsetzung von Succinyl-Coenzym A zu Methylmalonyl-Coenzym A katalysiert, handelt es sich vorzugsweise um eine Methylmalonyl-Coenzym A-Mutase (EC 5.4.99.2) ist. Dieses Enzym wird vorzugsweise von den Genen kodiert ausgewählt aus der Gruppe bestehend aus mut, mutA, mutB, sbm, sbmA, sbmB, sbm5, bhbA, mcmA, mcmA1, mcmA2, mcmB, mcm1, mcm2, mcm3, icmA, meaA1 und meaA2. Die Nukleotidsequenz dieser Gene können beispielsweise der *"Kyoto Encyclopedia of Genes and Genomes*" (KEGG-Datenbank), den Datenbanken des National Center for Biotechnology Information (NCBI) der National Library of Medicine (Bethesda, MD, USA) oder der Nukleotidsequenz-Datenbank der European Molecular Biologies Laboratories (EMBL, Heidelberg, Deutschland bzw. Cambridge, UK) entnommen werden.

Gemäß einer besonders bevorzugten Ausführungsform der ersten Variante des erfindungsgemäßen Verfahrens handelt es sich bei dem Enzym E₁ um die Methylmalonyl-Coenzym A-Mutase aus *Corynebacterium glutamicum* ATCC 13032, die von einem Gen mit der DNA-Sequenz gemäß SEQ.-ID-NR. 01 kodiert und die Aminosäuresequenz gemäß SEQ.-ID-Nr. 02 aufweist.

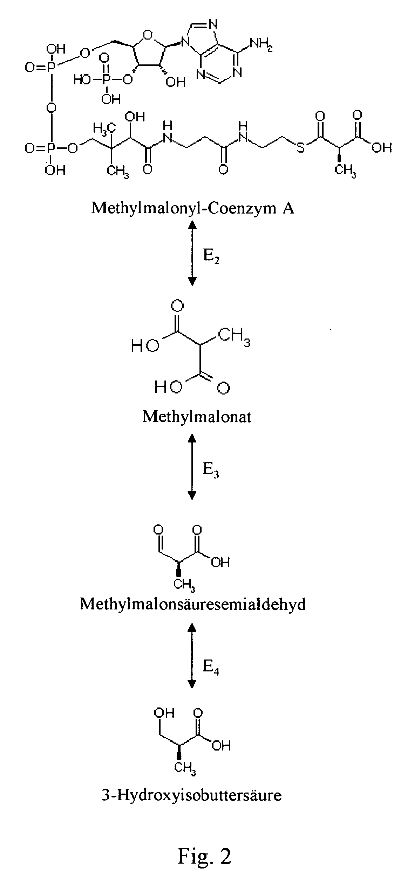

Weiterhin ist es gemäß einer ersten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden, bevorzugt, dass die gemäß dieser Alternative des erfindungsgemäßen Verfahrens eingesetzte gentechnisch veränderte Zelle, gegebenenfalls zusätzlich zur gesteigerten Aktivität des Enzyms E₁, eine im Vergleich zu ihrem Wildtyp gesteigerte Aktivität mindestens eines der folgenden Enzyme E₂ bis E₄ aufweist (siehe die Figur 2):
- eines Enzyms E₂, welches die Umsetzung von Methylmalonyl-Coenzym A zu Methylmalonat katalysiert;
- eines Enzyms E₃, welches die Umsetzung von Methylmalonat zu Methylmalonatsemialdehyd katalysiert;
- eines Enzyms E₄, welches die Umsetzung von Methylmalonatsemialdehyd zu 3-Hydroxyisobuttersäure katalysiert.

Erfindungsgemäß besonders werden Zellen eingesetzt, bei denen die Aktivität folgender Enzyme oder Enzymkombinationen gesteigert ist: E₂, E₃, E₄, E₂E₃, E₂E₄, E₃E₄, E₂E₃E₄, wobei E₂E₃E₄ am meisten bevorzugt ist. Weiterhin ist es möglich, dass ein Enzym auch mindestens zwei der vorstehend beschriebenen Reaktionsschritte zu katalysieren vermag. So kann beispielsweise ein Enzym eingesetzt werden, welches sowohl die Aktivität des Enzyms E₂ als auch die des Enzyms E₃ aufweist (und somit die Umsetzung von Methylmalonyl-Coenzym A direkt zum Methylmalonat-Semialdehyd katalysiert), wie beispielsweise die Malyonyl-Coenzym A-Reduktase aus *Sulfolobus tokodaii,* welche von der DNA-Sequenz mit der Seq.-ID.-Nr. 03 kodiert wird und welche die Aminosäuresequenz gemäß Seq.-ID.-Nr. 04 aufweist, oder aber ein Enzym, welches alle drei Enzymaktivitäten E₂, E₃ und E₄ aufweist, wie die Malonyl-Coenzym A-Reduktase aus *Chloroflexus aurantiacus* (Hügler et al., Journal of Bacteriology 184, Seiten 2.404-2.410, 2002).

In diesem Zusammenhang ist es besonders bevorzugt, dass das Enzym
- E₂: eine Methylmalonyl-Coenzym A-Hydrolase (EC 3.1.2.17),
- E₃: eine Aldehyd-Dehydrogenase (EC 1.2.1.3) oder eine Aldehyd-Oxidase (EC 1.2.3.1) und
- E₄: eine 3-Hydroxyisobutyrat-Dehydrogenase (EC 1.1.1.31) oder eine 3-Hydroxyacyl-Coenzym A-Dehydrogenase (EC 1.1.1.35)
ist.

Das Enzym E₂ wird vorzugsweise vom aox1-Gen kodiert. Die Methylmalonyl-Coenzym A-Hydrolase aus der Leber der Ratte ist beispielsweise in Kovachy et al., "Recognition, isolation, and characterization of rat liver D-methylmalonyl coenzyme A hydrolase", J. Biol. Chem. 258 (1983), Seiten 11.415-11.421 beschrieben.

Das Enzym E₃ wird vorzugsweise von Genen ausgewählt aus der Gruppe bestehend aus aldh2, aldh3a1, aldh3a2, aldh1b1, aldh9a1, aldh7a1, aldh1a4, aldh1a1, aldh1a2, mgc80785, mgc83352, mgc89020, dmel-CG31075, cg3752, cg9629, alh-9, alh-1, alh-2, f508.35, t7O23.15, f15I1.19, tT17F15.130, ald1, ald2, ald4, ald5, ald6, acl044Wp, adr417wp, msc7, tb06.5F5.780, aldH, puuC, putA, aldA, badH, alkH, pcD, rsp1591, rs01031, exaC, acoD, dhaL, pchA, aldB, dhaS, betB, ywdH, ycbD, aldX, aldY, aldA1, aldA2, aldC, pcd, cgl0546, cgl2668, cgl2796, scg11A.05, sci30A.27c, sce9.27c, sck13.05c, sc5H4.03, thcA, gabD2, alkH, aldH, aldh1, aldY1, aldY2, aldY3, aldY4, aldY5, aldY6, aldY7 und aldhT kodiert.

Geeignete Gene für das Enzym E₄ sind ausgewählt aus der Gruppe bestehend aus hibadh, cg15093, cg15093, cg4747, mwL2.23, t13k14.90, f19b15.150, hibA, ygbJ, mmsB, mmsB, garR, tsar, mmsB-1, mmsB-2, yfjR, ykwC, ywjF, hibD, glxR, SCM1.40c, hibD, ehhahd, hadh2, hadhsc, hsd17B4, loc488110, had, mgC81885, hadh2-prov, cg3415, cg7113, ech-1, ech-8, ech-9, ard-1, yfcX, fadB, faoA, fadB2x, hbd-1, hbd-2, hbd-3, hbd-4, hbd-5, hbd-6, hbd-7, hbd-8, hbd-9, hbd-10, fadJ, rs04421, rs02946, rs05766, bbsD, bbsC, fadB1, fadB2, fadB5, hbdA, pimF, fabJ-1, fabJ, scbac19f3.11, sci35.13, scbac8d1.10c, sc5f2a.15, sc6a5.38, fadC2, fadC4, fadC5, fadC6, had und paaH. Weitere geeignete 3-Hydroxyisobutyrat-Dehydrogenasen sind beispielsweise in Bannerjee et al. (1970), J. Biol. Chem, 245, Seiten 1.828 bis 1.835, Steele et al-(1992), J. Biol. Chem., 267, Seiten 13.585 bis 13.592, Harris et al. (1988), J. Biol. Chem., 263, Seiten 327 bis 331, Harris et al., Biochim. Biophys. Acta, 1645 (1), Seiten 89 bis 95, Hawes et al. (2000), Methods Enzymol., 324, Seiten 218 bis 228, Harris et al., J. Biol. Chem., 275 (49), Seiten 38.780 bis 38. 786, Rougraff et al. (1988), J. Biol. Chem.. , 263(1), Seiten 327 bis 331, Robinson et al., J. Biol. Chem.., 225, Seiten 511 bis 521, Hawes et al. (1995), Biochemistry, 34, Seiten 4.231 bis 4.237, Hasegawa J. (1981), Agric. Biol. Chem., 45, Seiten 2.805 bis 2814, Hawes et al. (1996), FEBS Lett., 389, Seiten 263 bis 267, Hawes et al. (1996), Enzymology and Molecular Biology of Carbonyl Metabolism, Plenum Press, New York, Seiten 395 bis 402, Adams et al. (1994), Structure, 2, Seiten 651 bis 668, Zhang et al. (1999), Biochemistry, 38, Seiten 11.231 bis 11.238, Mirny et al., (1999), J. Mol. Biol., 291, Seiten 177 bis 196 und Lokanath et al- (2005), J Mol Biol.*,* beschrieben.

Die Nukleotidsequenzen der vorstehend genannten Gene sowie weiterer Gene für die Enzyme E₂ bis E₄ können unter anderem auch der KEGG-Datenbank, der NCBI-Datenbank oder EMBL-Datenbank entnommen werden.

Gemäß einer besonders bevorzugten Ausführungsform dieser Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden, ist es bevorzugt, dass für die Umsetzung von Methylmalonyl-Coenzym A zum Methylmalonat-Semialdehyd die Malonyl-Coenzym A-Reduktase aus *Sulfolobus tokodaii* eingesetzt wird, welche von DNA-Sequenz mit der Seq.-ID.-Nr. 03 kodiert wird und welche die Aminosäuresequenz gemäß Seq.-ID.-Nr. 04 aufweist. Gemäß einer anderen besonders bevorzugten Ausführungsform dieser Variante wird für die Umsetzung von Methylmalonyl-Coenzym A zur 3-Hydroxyisobuttersäure die Malonyl-Coenzym A-Reduktase aus *Chloroflexus aurantiacus* eingesetzt (Hügler et al., Journal of Bacteriology 184, Seiten 2.404-2.410, 2002).

Weiterhin ist es in Zusammenhang mit dieser ersten Alternative der ersten besonderen Ausführungsform des erfindungsgemäßen Verfahrens bevorzugt, dass die gemäß dieser Ausführungsform eingesetzte, gentechnisch veränderte Zelle eine im Vergleich zu ihrem Wildtyp verminderte Aktivität eines Enzyms E₅ aufweist, welches die Umsetzung von Methylmalonatsemialdehyd zu Propionyl-Coenzym A aufweist, wobei es sich bei diesem Enzym vorzugsweise um eine Methylmalonatsemialdehyd-Dehydrogenase (EC 1.2.1.27) handelt.

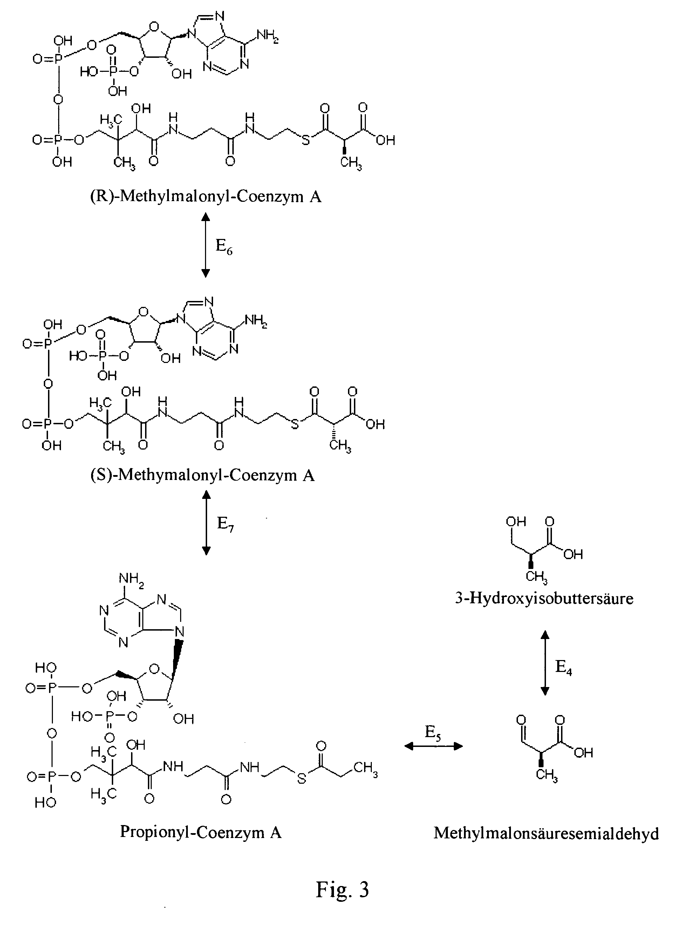

Gemäß einer zweiten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden, ist es bevorzugt, dass die Zelle, gegebenenfalls zusätzlich zur gesteigerten Aktivität des Enzyms E₁, eine im Vergleich zu ihrem Wildtyp gesteigerte Aktivität mindestens eines der folgenden Enzyme E₄ bis E₇ aufweist (siehe die Figur 3):
- eines Enzyms E₆, welches die Umsetzung von (R)-Methylmalonyl-Coenzym A zu (S)-Methylmalonyl-Coenzym A katalysiert;
- eines Enzyms E₇, welches die Umsetzung von (S)-Methylmalonyl-Coenzym A zu Propionyl-Coenzym A katalysiert;
- eines Enzyms E₅, welches die Umsetzung von Propionyl-Coenzym A zu Methylmalonatsemialdehyd katalysiert;
- eines Enzyms E₄, welches die Umsetzung von Methylmalonatsemialdehyd zu 3-Hydroxyisobuttersäure katalysiert.

Erfindungsgemäß besonders bevorzugt werden Zellen eingesetzt, bei denen die Aktivität folgender Enzyme oder Enzymkombinationen gesteigert ist: E₄, E₅, E₆, E₇, E₄E₅, E₄E₆, E₄E₇, E₅E₆, E₅E₇, E₆E₇, E₄E₅E₆, E₄E₅E₇, E₄E₆E₇, E₅E₆E₇ und E₄E₅E₆E₇, wobei E₄E₅E₆E₇ am meisten bevorzugt ist.

In diesem Zusammenhang ist es besonders bevorzugt, dass das Enzym
- E₆: eine Methylmalonyl-Coenzym A-Epimerase (EC 5.1.99.1),
- E₇: eine Methylmalonyl-Coenzym A-Decarboxylase (EC 4.1.1.41),
- E₅: eine Methylmalonatsemialdehyd-Dehydrogenase (EC 1.2.1.27), und
- E₄: eine 3-Hydroxyisobutyrat-Dehydrogenase (EC 1.1.1.31) oder eine 3-Hydroxyacyl-Coenzym A-Dehydrogenase (EC 1.1.1.35)
ist.

Bevorzugt Enzyme E₄ sind dabei diejenigen, die bereits vorstehend im Zusammenhang mit der ersten Variante der ersten bevorzugten Ausführungsform des erfindungsgemäßen Verfahrens beschrieben worden sind.

Das Enzym E₆ wird vorzugsweise vom mcee-Gen kodiert. Eine geeignete Methylmalonyl-Coenzym A-Decarboxylase (Enzym E₇) ist beispielsweise in Biochmistry, Vol. 39 (2000), Seiten 4.630-4.639 durch Benning et al. beschrieben.
Geeignete Gene für das Enzym E₅ sind vorzugsweise ausgewählt aus der Gruppe bestehend aus aldh6a1, cg17896, t22c12.10, ald6, putA1, mmsA, mmsA-1, mmsA-2, mmsA-3, mmsA-4, msdA, iolA und iolAB.

Geeignete Gene für das Enzym E₇ sind vorzugsweise ausgewählt aus der Gruppe bestehend aus mmdA, bcc, oadB, oadB2, oadB3, SC1C2.16, SC1G7.10, pccB1, accA2, mmdB, mmdC und ppcB.

Die Nukleotidsequenzen der vorstehend genannten Gene für die Enzyme E₅, E₆ und E₇ können unter anderem auch der KEGG-Datenbank entnommen werden.

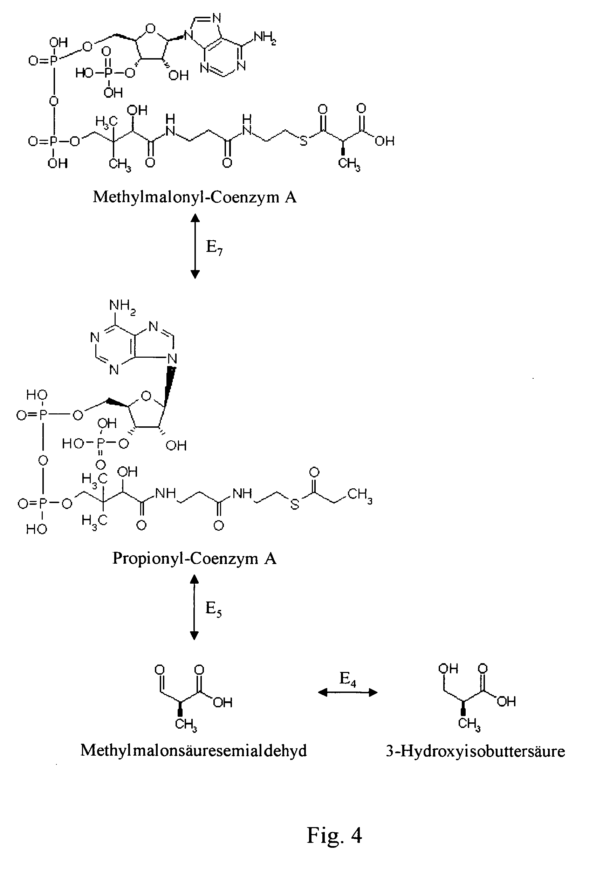

Gemäß einer dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden, ist es bevorzugt, dass die Zelle, gegebenenfalls zusätzlich zur gesteigerten Aktivität des Enzyms E₁, eine im Vergleich zu ihrem Wildtyp gesteigerte Aktivität mindestens eines der folgenden Enzyme E₄, E₅ und E₇ aufweist (siehe die Figur 4):
- eines Enzyms E₇, welches die Umsetzung von Methylmalonyl-Coenzym A zu Propionyl-Coenzym A katalysiert;
- eines Enzyms E₅, welches die Umsetzung von Propionyl-Coenzym A zu Methylmalonatsemialdehyd katalysiert;
- eines Enzyms E₄, welches die Umsetzung von Methylmalonatsemialdehyd zu 3-Hydroxyisobuttersäure katalysiert.

Dieser Weg entspricht im Wesentlichen der zweiten Variante der ersten bevorzugten Ausführungsform des erfindungsgemä-βen Verfahrens, wobei jedoch im Unterschied zur zweiten Variante die Herstellung von Propionyl-CoA unmittelbar aus Methylmalonyl-Coenzym A erfolgt. Bevorzugte Enzyme und Gene für die Enzyme E₄, E₅ und E₇ sind diejenigen Gene bzw. Enzyme, die bereits vorstehend im Zusammenhang mit der zweiten Variante genannt worden sind.

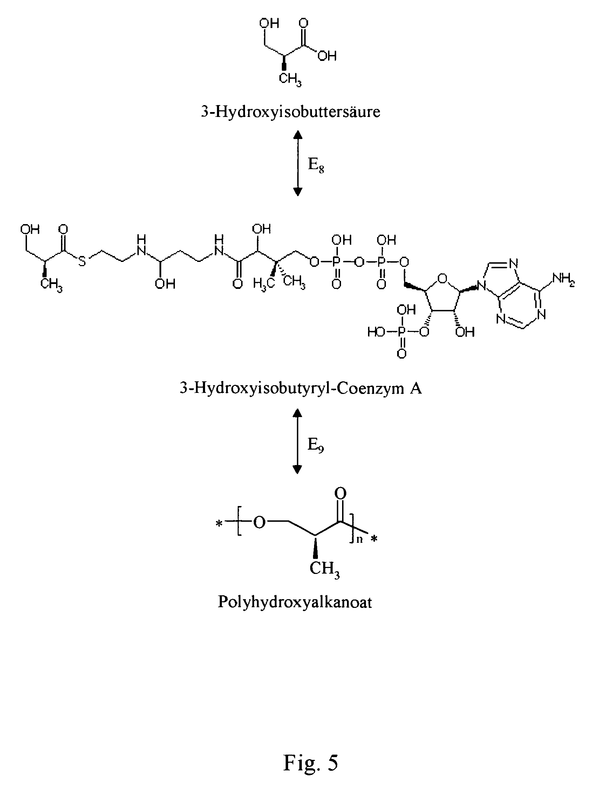

Weiterhin kann es gemäß der ersten besonderen Ausführungsform des erfindungsgemäßen Verfahrens (und auch gemäß aller nachfolgend noch beschriebenen Ausführungsformen) auch bevorzugt sein, eine gentechnisch veränderte Zelle einzusetzen, die in der Lage ist, die gebildete 3-Hydroxyisobuttersäure in ein Polyhydroxyalkanoat umzuwandeln. Solche Polyhydroxyalkanoate werden von vielen Mikroorganismen in Form von stark lichtbrechenden Granula intrazellulär abgelagert. In diesem Zusammenhang ist es insbesondere bevorzugt, dass die in dem erfindungsgemäßen Verfahren eingesetzte, gentechnisch veränderte Zelle eine im Vergleich zu ihrem Wildtyp gesteigerte Aktivität mindestens eines der, vorzugsweise der beiden folgenden Enzyme E₈ und E₉ aufweist (siehe die Figur 5):
- eines Enzyms E₈, welches die Umsetzung von 3-Hydroxyisobuttersäure zu 3-Hydroxyisobutyryl-Coenzym A katalysiert;
- eines Enzyms E₉, welches die Umsetzung von 3-Hydroxyisobutyryl-Coenzym A zu einem auf 3-Hydroxyisobuttersäure basierenden Polyhydroxyalkanoat katalysiert.

In diesem Zusammenhang ist es besonders bevorzugt, dass das Enzym
- E₈: eine 3-Hydroxyisobutyryl-CoA-Hydrolase (EC 3.1.2.4) und
- E₉: eine Polyhydroxyalkanoat-Synthase
ist.

Wie bereits vorstehend erläutert, entsteht bei der ersten bevorzugten Ausführungsform des erfindungsgemäßen Verfahrens die 3-Hydroxyisobuttersäure oder die auf 3-Hydroxyisobuttersäure basierenden Polyhydroxyalkanoate aus Succinyl-Coenzym A als Zwischenprodukt und aus Methylmalonat-Semialdehyd als Vorprodukt. Dabei kann es grundsätzlich sinnvoll sein, neben einer Beeinflussung einer oder mehrerer der vorstehend genannten Enzymaktivitäten E₁ bis E₉ auch solche Enzymaktivitäten zu beeinflussen, welche zu einer Erhöhung der Bildung von Succinyl-Coenzym A in der Zelle führen.

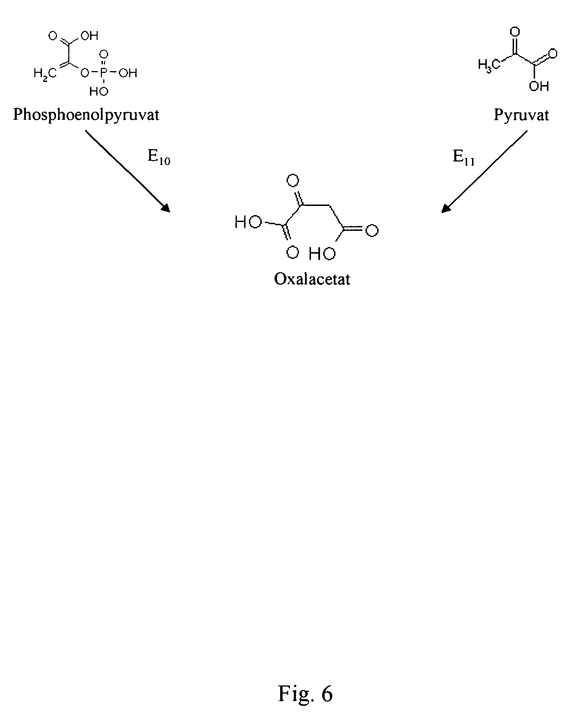

Sofern gemäß der ersten besonderen Ausführungsform der ersten Variante des erfindungsgemäßen Verfahrens die Bildung der 3-Hydroxyisobuttersäure oder des auf 3-Hydroxyisobuttersäure basierenden Polyhydroxyalkanoates über Succinyl-Coenzym A als Zwischenprodukt und Methylmalonat-Semialdehyd als Vorprodukt aus Kohlenhydraten oder Glycerin erfolgt, ist es gemäß einer besonderen Ausgestaltung der vorstehend beschriebenen ersten, zweiten oder dritten Alternative des erfindungsgemäßen Verfahrens bevorzugt, dass die eingesetzte, gentechnisch veränderte Zelle eine im Vergleich zu ihrem Wildtyp gesteigerte Aktivität mindestens eines der, vorzugsweise der beiden folgenden Enzyme E₁₀ und E₁₁ aufweist (siehe die Figur 6):
- eines Enzyms E₁₀, welches die Umsetzung von Phosphoenolpyruvat zu Oxalacetat katalysiert;
- eines Enzyms E₁₁, welches die Umsetzung von Pyruvat zu Oxalacetat katalysiert.

In diesem Zusammenhang ist es besonders bevorzugt, dass das Enzym
- E₁₀: eine Phosphoenolpyruvat-Carboxylase (EC 4.1.1.31) und
- E₁₁: eine Pyruvat-Carboxylase (EC 6.4.1.1)
ist.

Das Enzym E₁₀ wird vorzugsweise von den Genen ausgewählt aus der Gruppe umfassend f12m16.21, f14n22.13, k15m2.8, ppc, clpA, pepC, capP, cgl1585, pepC, pck, ppc und pccA kodiert, wobei das ppc-Gen besonders bevorzugt ist. Erfindungsgemäß bevorzugte Phosphoenolpyruvat-Carboxylasen sind insbesondere auch in US 4,757,009, US 4,980,285, US 5,573,945, US 6,872,553 und US 6,599,732 beschrieben.

Das Enzym E₁₁ wird vorzugsweise von den Genen ausgewählt aus der Gruppe umfassend pc, pcx, cg1516, cg1516, pyc-1, pyc-2, aar162Cp, pyr1, accC-2, pycA, pycA2, pca, cgl0689, pyc, pycB, accC, oadA, acc und accC1 kodiert, wobei das pyc-Gen besonders bevorzugt ist. Erfindungsgemäß bevorzugte Pyruvat-Carboxylasen sind insbesondere auch in US 6,455,284, US 6,171,833, US 6,884,606, US 6,403,351, US 6,852,516 und US 6,861,246 beschrieben. Eine weitere, erfindungsgemäß besonders bevorzugte Pyruvat-Carboxylase ist diejenige Mutante, die in "A novel methodology employing Corynebacterium glutamicum genome information to generate a new L-lysine-producing mutant.", Ohnishi J et al., Applied Microbiology and Biotechnology, Vol. 58 (2), Seiten 217-223 (2002) beschrieben ist.

Die Nukleotidsequenzen geeigneter Gene der Enzyme E₁₀ und E₁₁ können der KEGG-Datenbank, der NCBI-Datenbank oder EMBL-Datenbank entnommen werden.

Von der Oxalacetat-Zwischenstufe gibt es mehrere Möglichkeiten, um zum Succinyl-Coenzym A zu gelangen, welches dann über Methylmalonyl-Coenzym A mittels der drei eingangs genannten Varianten zu 3-Hydroxyisbuttersäure umgesetzt werden kann.

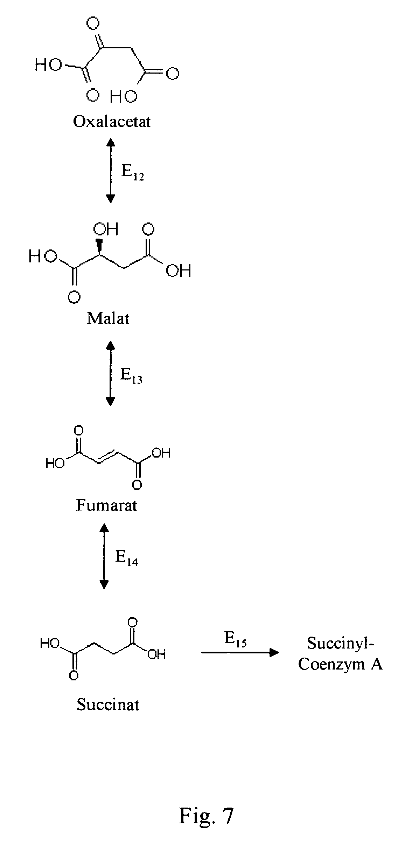

Ein erster Weg führt über Fumarat als Zwischenprodukt. In diesem Fall ist es gemäß einer ersten besonderen Ausgestaltung der vorstehend beschriebenen ersten, zweiten oder dritten Alternative des erfindungsgemäßen Verfahrens, bei der eine gentechnisch veränderte Zelle eingesetzt wird, in der Methylmalonat-Semialdehyd als Vorprodukt und Succinyl-Coenzym A als Zwischenprodukt gebildet werden, bevorzugt, dass die Zelle, gegebenenfalls zusätzlich zu einer gesteigerten Aktivität des Enzyms E₁₀ oder E₁₁, eine im Vergleich zu ihrem Wildtyp gesteigerte Aktivität mindestens eines der folgenden Enzyme E₁₂ bis E₁₅ aufweist (siehe die Figur 7) :
- eines Enzyms E₁₂, welches die Umsetzung von Oxalacetat zu Malat katalysiert;
- eines Enzyms E₁₃, welches die Umsetzung von Malat zu Fumarat katalysiert;
- eines Enzyms E₁₄, welches die Umsetzung von Fumarat zu Succinat katalysiert;
- eines Enzyms E₁₅, welches die Umsetzung von Succinat zu Succinyl-Coenzym A katalysiert.

Erfindungsgemäß besonders werden dabei Zellen eingesetzt, bei denen die Aktivität folgender Enzyme oder Enzymkombinationen gesteigert ist: E₁₂, E₁₃, E₁₄, E₁₅, E₁₂E₁₃, E₁₂E₁₄, E₁₂E₁₅, E₁₃E₁₄, E₁₃E₁₅, E₁₄E₁₅, E₁₂E₁₃E₁₄, E₁₂E₁₃E₁₅, E₁₂E₁₄E₁₅, E₁₃E₁₄E₁₅, E₁₂E₁₃E₁₄E₁₅, wobei E₁₂E₁₃E₁₄E₁₅ am meisten bevorzugt ist.

In diesem Zusammenhang ist es besonders bevorzugt, dass das Enzym
- E₁₂: eine Malat-Dehydrogenase (EC 1.1.1.37) oder eine Malat-Chinon-Oxidoreduktase (1.1.99.16),
- E₁₃: eine Fumarat-Hydratase (EC 4.2.1.2),
- E₁₄: eine Succinat-Dehydrogenase (EC 1.3.99.1 oder EC 1.3.5.1) oder eine Succinat-Chinon-Oxidoreduktase (1.3.5.1), und
- E₁₅: eine Succinat-Coenzym A-Ligase (EC 6.2.1.4 oder EC 6.2.1.5)
ist.

Das Enzym E₁₂ wird vorzugsweise von Genen ausgewählt aus der Gruppe umfassend mdh1, mdh2, mor1, cg10748, cg10749, cg5362, mdh-1, f46e10.10, f19p19.13, f12m16.14, t30l20.4, k15m2.16, f1p2.70, f17i14.150, mnl12.18, mik19.17, mdh3, adl164cp, adr152cp, adr252wp, mdhA, mdhC, mdhB, ybiC, mdh, yiaK, ybiC, allD, citH, yjmC, citH, cgl2380, ldh, sqdB, mqo, yojH, mqoA, mqoB, mqo1, mqo2, mqo3, mqo4 und cgl2001 kodiert, wobei das mqo-Gen und das mdh-Gen besonders bevorzugt sind.

Das Enzym E₁₃ wird vorzugsweise von Genen ausgewählt aus der Gruppe umfassend fh, fh1, sc4094, sc4095, t30b22.19, k3k7.11, acr013Cp, fum1, fum2, fum3, fum4, fumH, fumA, fumB, fumC, fumC1, fumC2, fum, ttdA, ttdB, fumB-alpha, fumB-beta, citG, citB, fumX, fum-1 und fum-2 kodiert, wobei das fum-Gen besonders bevorzugt ist.

Das Enzym E₁₄ wird vorzugsweise von Genen ausgewählt aus der Gruppe umfassend sdh1, sdh2, sdh3, sdh4, sdh5, sdh6, osm1, osm2, sdhA, sdhB, sdhC, sdhD, frdA, frdB, frdC, frdD, ifcA-1, ifcA-2, sdhB-1, sdhB-2, frdC2, cgl0370, cgl0371, cgl0372, scm10.10c, scm10.11c, scm10.12c, sc5g8.25c, sc5g8.26c, scbac-31e11.02c, scbac31e11.02c, sc4b10.10c, s-dhA2, sdhB2, sdhA1, sdhB1, qcrB2, sdhA3, sdhB3, frdB1 und frdB2 kodiert, wobei die Gene sdhA, sdhB und sdhC besonders bevorzugt sind.

Das Enzym E₁₅ wird vorzugsweise von Genen ausgewählt aus der Gruppe umfassend suclg1, suclg2, loc434885, cg10622, dmel-CG6255, f11a3.3, f8115.30, mkd15.11, lsc1, lsc2, a-el211wp, afr134cp, scsA, scsB, sucC und sucD kodiert.
Die Nukleotidsequenzen geeigneter Gene der Enzyme E₁₂ bis E₁₅ können wiederum auch der KEGG-Datenbank, der NCBI-Datenbank oder EMBL-Datenbank entnommen werden.

Sofern die Aktivität eines oder mehrerer der Enzyme E₁₂ bis E₁₅ erhöht ist, kann es sich auch als vorteilhaft erweisen, dass die Zelle eine im Vergleich zu ihrem Wildtyp verminderte Aktivität eines der folgenden Enzyme E₁₆ bis E₂₃ aufweist:
- eines Enzyms E₁₆, welches die Umsetzung von Oxalacetat zu Citrat katalysiert;
- eines Enzyms E₁₇, welches die Umsetzung von Malat zu Oxalacetat katalysiert;
- eines Enzyms E₁₈, welches die Umsetzung von Succinyl-Coenzym A zu Succinat katalysiert,
- eines Enzyms E₁₉, welches die Umsetzung von Oxalacetat zu Phosphoenolpyruvat katalysiert,
- eines Enzyms E₂₀, welches die Umsetzung von Oxalacetat zu Pyruvat katalysiert,
- eines Enzyms E₂₁, welches die Umsetzung von Oxalacetat zu Aspartat katalysiert,
- eines Enzyms E₂₂, welches die Umsetzung von Malat zu Pyruvat katalysiert,
- eines Enzyms E₂₃, welches die Umsetzung von Pyruvat zu Acetat katalysiert,

Erfindungsgemäß besonders bevorzugte Zellen sind dabei diejenigen, bei denen die Aktivität folgender Enzyme oder Enzymkombinationen vermindert ist: E₁₆, E₁₇, E₁₈, E₁₉, E₂₀, E₂₁, und E₁₆E₁₇E₁₈E₁₉E₂₀E₂₁E₂₂E₂₃.

In diesem Zusammenhang ist es besonders bevorzugt, dass das Enzym
- E₁₆: eine Citrat-Synthase (EC 2.3.3.1 oder EC 2.3.3.8),
- E₁₇: eine Malat-Oxidase (EC 1.1.3.3),
- E₁₈: eine Succinyl-CoA-Hydrolase (EC 3.1.2.3),
- E₁₉: eine Phosphoenolpyruvat-Carboxykinase (EC 4.1.1.49 oder 4.1.1.32),
- E₂₀: eine Oxalacetat-Decarboxylase (EC 4.1.1.3),
- E₂₁: eine Aspartat-Transaminase (EC 2.6.1.1),
- E₂₂: eine Malat-Dehydrogenase (EC 1.1.1.38, EC 1.1.1.39 oder EC 1.1.1.40),
- E₂₃: eine Pyruvat-Dehydrogenase (EC 1.2.1.51),
ist.

Das Enzym E₁₆ wird vorzugsweise von Genen ausgewählt aus der Gruppe umfassend glt, cs, csl, cg3861, cts-1, f7f19.21, f4i1.16, t20n10.90, t20n10.100, t2O9.80, cit1, cit2, cit3, aar004cp, agr002wp, cshA, gltA, citZ, cit, prpC, cisY, cis, mmgD, citA, gltA1, gltA2, gltA3, cgl0829, prpC1, scd10.20, citA1, citA2, citA3, acly, cg8322, f5e6.2, k7jJ8.14 und citE kodiert, wobei gltA am meisten bevorzugt ist.

Das Enzym E₁₉ wird vorzugsweise von Genen ausgewählt aus der Gruppe umfassend pckA, pck1, pck2, cg10924, cg17725, cg17725, pckG, ppcK, cgl2863, pck und 2sck36.02 kodiert. Das Enzym E₂₀ wird vorzugsweise von Genen ausgewählt aus der Gruppe umfassend oadA, oadB, oadC, oadG, oag3, eda, dcoA, oadA1, oadA2, pycB und mmdB kodiert.

Das Enzym E₂₁ wird vorzugsweise von Genen ausgewählt aus der Gruppe umfassend myn8.7, gltl, adr290wp, gltB, gltD, glt1, gls1, gltA, glt, glxD, gltD1, gltD2, gdh2, agl040Cp, gdhA1, gdhA, gdhA2, gluD, gluD1, gluD2, rocG, ypcA, gudB, t11i18.2, t2i1.150, mrg7.13, f19c24.7, gdh, gdh1, gdh2, gdh3, got1, got2, cg4233, cg8430, f23n19.17, f13j11.16, t26c19.9, f7f1.18, F10N7.200, t16l1.170, f15n18.110, t20d1.70, aat1, aat2, abl038wp, afr211cp, agx1, bna4, aatA, aatB, ybdL, aspC, yfbQ, aat, avtA1, avtA2, tyrB, avtA, avtB, argD1, argD2, aspB1, aspB2, aspB3, aspB, aspC1, aspC2, aspC3, aspC4, RS05143, aspAT, ywfG, yhdR, argD, mtnV, alaT, hisC, avtA1, avtA2, avtA3, cgl0240, cgl1103, cgl2599, cgl2844, 2sck36.07c, sc9e12.21, sc2h4.04c, tyrB, gtp, gtp1, gtp2, cg1640, f20d23.34, f26f24.16, f24j13.15, t10d10.20 und agr085wp kodiert, wobei aspC, aatA, gdh, gudB, gdhA, gltB und gltD besonders bevorzugt sind.

Das Enzym E₂₁ wird vorzugsweise von Genen ausgewählt aus der Gruppe umfassend myn8.7, glt1, adr290wp, gltB, gltD, glt1, gls1, gltA, glt, glxD, gltD1, gltD2, gdh2, agl040Cp, gdhAl, gdhA, gdhA2, gluD, gluD1, gluD2, rocG, ypcA,

Das Enzym E₂₂ wird vorzugsweise von Genen ausgewählt aus der Gruppe umfassend me, me1, me2, me3, mae, mae1, mae2, sfcA, sfcA1, maeA, maeB, tme, yqkJ, ywkA, yqkJ, malS, ytsJ, mleA, mleS, mez, sce59.10c, 2sc7g11.23, malS1, malS2, dme, maeB1, maeB2, mdh, mdh1, mdh2, dmel_cg10120, dmel_cg10120, dmel-cg5889, f19k16.27, f6f22.7, t22p22.60, f18a17.1, mod1, tme, mao, cgl3007, malS und malE kodiert.

Das Enzym E₂₃ wird vorzugsweise von Genen ausgewählt aus der Gruppe umfassend me, me1, me2, me3, mae, mae1, mae2, sfcA, sfcA1, maeA, maeB, tme, yqkJ, ywkA, yqkJ, malS, ytsJ, mleA, mleS, mez, sce59.10c, 2sc7g11.23, malS1, malS2, dme, maeB1, maeB2, mdh, mdh1, mdh2, dmel_cg10120, dmel_cg10120, dmel_cg5889, f19k16.27, f6f22.7, t22p22.60, f18a17.1, mod1, tme, mao, cgl3007, malS und malE kodiert.

Weiterhin ist es erfindungsgemäß bevorzugt, dass in dem Fall, in dem die gesteigerte Bereitstellung von Succinyl-Coenzym A in der Zelle mittels des vorstehend beschriebenen Weges (Oxalacetat → Malat → Fumarat → Succinyl-Coenzym A) erfolgt, in der Zelle auch die Bereitstellung von Reduktionsäquivalenten gezielt zu steigern.

Eine Möglichkeit der Steigerung von Reduktionsäquivalenten besteht darin, den oxidativen Pentosephosphat-Weg zu steigern. In diesem Zusammenhang ist es besonders bevorzugt, dass die Aktivität der Glucose-6-Phosphat-Dehydrogenase (EC 1.1.1.49) und/oder der 6-Phosphogluconat-Dehydrogenase (EC 1.1.1.44), welche vorzugsweise vom gnd-Gen kodiert wird, erhöht wird und gegebenenfalls gleichzeitig die Glykolyse gehemmt wird, beispielsweise durch Abschwächung der Aktivität der Glucose-6-Phosphat-Isomerase, wie dieses in WO-A-01/07626 beschrieben ist. Neben oder anstelle der gezielten Förderung des Pentosephosphat-Weges kann es weiterhin bevorzugt sein, Reduktionsäquivalente dadurch bereitzustellen, dass den Zellen Ethanol als Kohlenstoffquelle angeboten wird und in den Zellen die Umsetzung des Ethanols zu Acetaldehyd mittels Alkohol-Dehydrogenasen (EC 1.1.1.1, EC 1.1.1.2, EC 1.1.1.71 oder EC 1.1.99.8) und die weitere Umsetzung des Acetaldehyds zu Acetyl-Coenzym A mittels Acetaladehyd-Dehydrogenasen (EC 1.2.1.10) zu fördern. Geeignete Gene für Alkohol-Dehydrogenasen und Acetaldehyd-Dehydrogenasen können wiederum aus dem Fachmann bekannten Gen-Datenbanken, wie etwa der KEGG-Datenbank, der NCBI-Datenbank oder EMBL-Datenbank, entnommen werden.

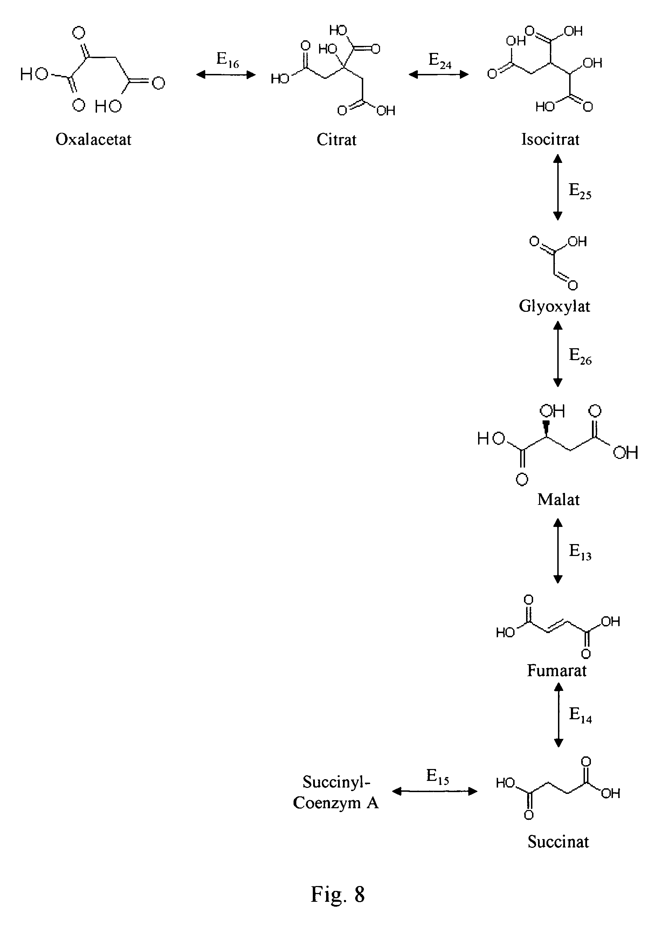

Ein zweiter Weg vom Oxalacetat zum Succinyl-Coenzym A führt über Citrat als Zwischenprodukt. In diesem Fall ist es gemäß einer zweiten besonderen Ausgestaltung der vorstehend beschriebenen ersten, zweiten oder dritten Alternative des erfindungsgemäßen Verfahrens bevorzugt, eine gentechnisch veränderte Zelle einzusetzen, die gegebenenfalls zusätzlich zu einer gesteigerten Aktivität des Enzyms E₁₀ oder E₁₁, eine im Vergleich zu ihrem Wildtyp gesteigerte Aktivität mindestens eines der folgenden Enzyme E₁₃ bis E₁₆ und E₂₄ bis E₂₆ aufweist (siehe die Figur 8):
- eines Enzyms E₁₆, welches die Umsetzung von Oxalacetat zu Citrat katalysiert;
- eines Enzyms E₂₄, welches die Umsetzung von Citrat zu Isocitrat katalysiert;
- eines Enzyms E₂₅, welches die Umsetzung von Isocitrat zu Glyoxalat und Succinat katalysiert;
- eines Enzyms E₂₆, welches die Umsetzung von Glyoxalat zu Malat katalysiert;
- eines Enzyms E₁₃, welches die Umsetzung von Malat zu Fumarat katalysiert;
- eines Enzyms E₁₄, welches die Umsetzung von Fumarat zu Succinat katalysiert;
- eines Enzyms E₁₅, welches die Umsetzung von Succinat zu Succinyl-Coenzym A katalysiert.

Erfindungsgemäß besonders bevorzugte Zellen sind dabei diejenigen, bei denen die Aktivität folgender Enzyme oder Enzymkombinationen erhöht ist: E₁₃, E₁₄, E₁₅, E₁₆, E₂₄, E₂₅, E₂₆, E₁₃E₁₄, E₁₃E₁₅, E₁₃E₁₆, E₁₃E₂₄, E₁₃E₂₅, E₁₃E₂₆, E₁₄E₁₅, E₁₄E₁₆, E₁₄E₂₄, E₁₄E₂₅, E₁₄E₂₆, E₁₅E₁₆, E₁₅E₂₄, E₁₅E₂₅, E₁₅E₂₆ und E₁₃E₁₄E₁₅E₁₆ E₂₄E₂₅E₂₆, wobei E₁₃E₁₄E₁₅E₁₆E₂₄E₂₅E₂₆ am meistem bevorzugt ist.

In diesem Zusammenhang ist es besonders bevorzugt, dass das Enzym
- E₁₃: eine Fumarat-Hydratase (EC 4.2.1.2),
- E₁₄: eine Succinat-Dehydrogenase (EC 1.3.99.1 oder EC 1.3.5.1) oder eine Succinat-Chinon-Oxidoreduktase (1.3.5.1),
- E₁₅: eine Succinat-Coenzym A-Ligase (EC 6.2.1.4 oder EC 6.2.1.5),
- E₁₆: eine Citrat-Synthase (EC 2.3.3.1 oder EC 2.3.3.8),
- E₂₄: eine Aconitat-Hydratase (EC 4.2.1.3),
- E₂₅: eine Iscocitrat-Lyase (EC 4.1.3.1) und
- E₂₆: eine Malat-Synthase (EC 2.3.3.9),
ist.

Bevorzugt Gene für die Enzyme E₁₃ bis E₁₆ sind diejenigen, die bereits vorstehend im Zusammenweg mit dem ersten Weg vom Oxalacetat zum Succinyl-Coenzym A beschrieben worden sind.

Das Enzym E₂₄ wird vorzugsweise von Genen ausgewählt aus der Gruppe umfassend aco1, aco2, ratireb, dmel-CG4706, dmel-CG4900, dmel-cg6342, cg9244, t3p4.5, f10m23.310, f4b14.100, ad1032Wp, afr629wp, acnA, acnB, acnC, acnD, rpfA, acnA1, acnA2, acnM, citB, leuC, cgl1540, sacA, can, und aco kodiert, wobei acnA und acnB besonders bevorzugt sind.

Das Enzym E₂₅ wird vorzugsweise von Genen ausgewählt aus der Gruppe umfassend msd21.4, icl1, icl2, adl066cp, agl057wp, aceA, icl, aceAa, aceAb, cgl0097 und cgl2331 kodiert, wobei aceA besonders bevorzugt ist. Gemäß einer besonderen Ausführungsform sind solche Gene bevorzugt, welche für eine auf der Gen-Ebene oder Protein-Ebene deregulierte Isocitrat-Lyase kodieren.

Das Enzym E₂₆ wird vorzugsweise von Genen ausgewählt aus der Gruppe umfassend med24.5, mlsS1, acr268cp, masA, glcB, aceB, mls, glcB-1, glcB-2, cg12329, masZ, aceB1, aceB2 und mas kodiert, wobei das aceB-Gen besonders bevorzugt ist.

Auch hier können die Nukleotidsequenzen geeigneter Gene der Enzyme E₂₄ bis E₂₆ der KEGG-Datenbank, der NCBI-Datenbank oder EMBL-Datenbank entnommen werden.

Wenn die Bereitstellung von Oxalacetat aus Phosphoenolpyruvat oder aus Pyruvat durch die Steigerung der Aktivität des Enzyms E₁₀ oder E₁₁ gefördert wird, kann das bei der Spaltung des Isocitrats durch die Iscocitrat-Lyase neben Glyoxalat gebildete Succinat ebenfalls für die Bildung von Succinyl-Coenzym A genutzt werden. Weiterhin kann es bei diesem zweiten Weg vom Oxalacetat zum Succinat vorteilhaft sein, die Aktivität eines Enzyms E₂₇, welches die Umsetzung von Isocitrat zu 2-Oxoglutarat katalysiert und bei dem es sich vorzugsweise um eine Isocitrat-Dehydrogenase (EC 1.1.1.41 oder EC 1.1.1.42) handelt, zu vermindern. Vorzugsweise handelt es sich bei der Isocitrat-Dehydrogenase um ein Enzym, welches von einem Gen ausgewählt aus der Gruppe umfassend idh1, idh2, cg7176, cg7176, cg7176, f20d21.16, f12p19.10, t15n1.80, idp1, idp2, idp3, aal022Wp, aer061Cp, idhC, idhM, icdA, icd, idh, icd1, icd2, leuB, citC, citC, cgl0664, leuB2, idh3A, idg3B, idh3G, cg12233, dmel-CG5028, dmel-CG6439, f6p23.14, f23e12.180, f8d20.160, f12e4.20, adl223wp und afr137cp kodiert wird, wobei icdA und citC besonders bevorzugt sind.

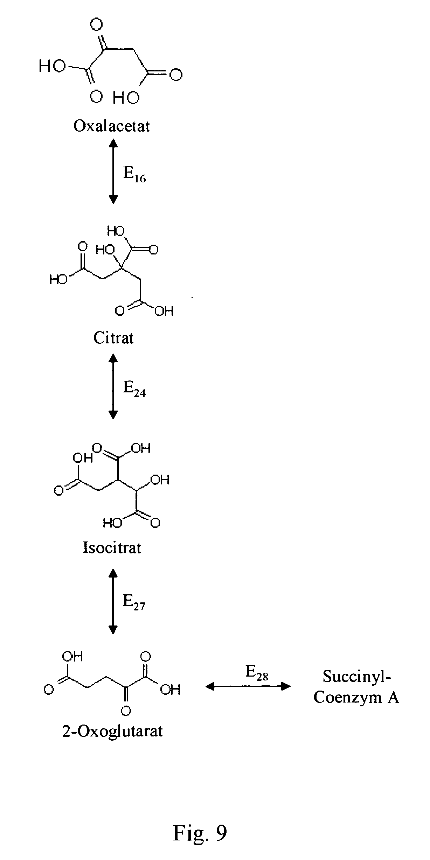

Ein dritter Weg vom Oxalacetat zum Succinyl-Coenzym A führt über 2-Oxoglutarat als Zwischenprodukt. In diesem Fall ist es gemäß einer dritten besonderen Ausgestaltung der vorstehend beschriebenen ersten, zweiten oder dritten Alternative des erfindungsgemäßen Verfahrens bevorzugt, eine gentechnisch veränderte Zelle einzusetzen, die gegebenenfalls zusätzlich zu einer gesteigerten Aktivität des Enzyms E₁₀ oder E₁₁, eine im Vergleich zu ihrem Wildtyp gesteigerte Aktivität mindestens eines der folgenden Enzyme E₁₆, E₂₄, E₂₇ und E₂₈ aufweist (siehe die Figur 9) :
- eines Enzyms E₁₆, welches die Umsetzung von Oxalacetat zu Citrat katalysiert;
- eines Enzyms E₂₄, welches die Umsetzung von Citrat zu Isocitrat katalysiert;
- eines Enzyms E₂₇, welches die Umsetzung von Isocitrat zu 2-Oxoglutarat katalysiert;
- eines Enzyms E₂₈, welches die Umsetzung von 2-Oxoglutarat zu Succinyl-Coemzym A katalysiert.

Erfindungsgemäß besonders bevorzugte Zellen sind dabei diejenigen, bei denen die Aktivität folgender Enzyme oder Enzymkombinationen erhöht ist: E₁₆, E₂₄, E₂₇, E₂₈, E₁₆E₂₄, E₁₆E₂₇, E₁₃E₂₈, E₂₄E₂₇, E₂₄E₂₈, E₂₇E₂₈, E₁₆E₂₄E₂₇, E₁₆E₂₄E₂₈, E₂₄E₂₇E₂₈ und E₁₆E₂₄E₂₇E₂₈, wobei E₁₆E₂₄E₂₇E₂₈ am meistem bevorzugt ist.

In diesem Zusammenhang ist es besonders bevorzugt, dass das Enzym
- E₁₆: eine Citrat-Synthase (EC 2.3.3.1 oder EC 2.3.3.8),
- E₂₄: eine Aconitat-Hydratase (EC 4.2.1.3),
- E₂₇: eine Isocitrat-Dehydrogenase (EC 1.1.1.41 oder EC 1.1.1.42) und
- E₂₈: eine 2-Oxoglutarat-Synthase (EC 1.2.7.3)
ist.

Bevorzugt Gene für die Enzyme E₁₆, E₂₄ und E₂₇ sind diejenigen, die bereits vorstehend im Zusammenhang mit dem ersten und zweiten Weg vom Oxalacetat zum Succinyl-Coenzym A beschrieben worden sind beschrieben worden sind.

Das Enzym E₂₈ wird vorzugsweise von Genen ausgewählt aus der Gruppe umfassend korA, korB, kor D, korA1, korA2, korB1, korB2, oorA, oorB, oorC, oorD, oforA, oforB, porA, porB, porA1, porA2, porA3, porA4, porG, porG1, porG2, porB1, porB2, porB3, SCD20.12c, SCD20.13c, SCAH10.34c, SCAH10.35c, korG, orA, orB, korG1 und korG2 kodiert. Weiterhin kann es sich bei E₂₈ auch um einen Dehydrogenase-Komplex aus mehreren Untereinheiten handeln, die unterschiedliche enzymatische Aktivitäten aufweisen. Insbesondere kann es sich um einen Dehydrogenase-Komplex umfassend eine Oxoglutarat-Dehydrogenase (EC 1.2.4.2), eine Dihydrolipoyl-Dehydrogenase (EC 1.8.1.4) und eine Dihydrolipoyllysin-Rest-Succinyl-Transferase (EC 2.3.1.61). Die Oxoglutarat-Dehydrogenase (EC 1.2.4.2) wird dabei vorzugsweise von Genen ausgewählt aus der Gruppe umfassend ogdh, ghdhl, loc239017, mgc68800, mgc80496, cg11661, t22e16.70, mpA24.10, kgd1, aer374cp, sucA, odhA, kgdA und cgl1129 kodiert, wobei sucA und odhA besonders bevorzugt sind. Die Dihydrolipoyl-Dehydrogenase (EC 1.8.1.4) wird vorzugsweise von Genen ausgewählt aus der Gruppe umfassend dld, dldprov, dldh, cg7430, t2j15.6, k14a17.6, at3g17240, mgd8.7lpd1, afr512wp, dld1, lpd, tb03.26j7.650, tb04.3m17.450, tb927.8.7380, tb08.10k10.200, lpdA, lpdG, lpdV, lpd3, acoD, lpdA1, lpdA2, lpdA3, odhL, pdhD, pdhD1, pdhD2, pdhD3, pdhD42, lpdAch1, lpdAch2, lpdAc, acoL, bfmbC, bkdD, cgl0366, cgl0688, scm1.17c, pdhL, sck13.11, lpdB2 und dld1 kodiert, wobei lpd besonders bevorzugt ist. Die Dihydrolipoyllysin-Rest-Succinyl-Transferase (EC 2.3.1.61) wird dabei vorzugsweise von Genen ausgewählt aus der Gruppe umfassend dlst, dlst-prov, mgc89125, dmel_CG5214, f10m23.250, k13p22.8, kgd2agl200wp, kgd2, odhB, sucB, aceF, kgdB, sucB1, sucB2, pdhC, dlaT, kgd, sc5F7.20 und sc4B10.24c kodiert, wobei sucB und odhB besonders bevorzugt sind.

Die Nukleotidsequenzen geeigneter Gene des Enzyms E₂₈ bzw. der vorstehend genannten Untereinheiten des Enzyms E₂₈ können wiederum der KEGG-Datenbank, der NCBI-Datenbank oder EMBL-Datenbank entnommen werden.

Die vorstehend beschriebenen Wege vom Oxalacetat zum Succinyl-Coenzym A gehen von Phosphoenolpyruvat oder von Pyruvat als Substratvorstufen aus. In diesem Zusammenhang kann es weiterhin bevorzugt sein, die Zellen gentechnisch derart zu verändern, dass sie aus Kohlehydraten und/oder aus Glycerin besonders große Mengen an Pyruvat oder Phosphoenolpyruvat bereitstellen können.

Sofern die Zellen Glycerin als Nährstoffquelle verwerten können, ist es bevorzugt, dass die in dem erfindungsgemäßen Verfahren eingesetzte, gentechnisch veränderte Zelle eine im Vergleich zu ihrem Wildtyp gesteigerte Aktivität mindestens eines, vorzugsweise aller folgenden Enzyme E₂₉ bis E₄₂ aufweist:
- eines Enzyms E₂₉, welches die Diffusion von Glycerin in die Zelle hinein erleichtert,
- eines Enzyms E₃₀, welches die Umsetzung von Glycerin zu Glycerin-3-Phosphat katalysiert,
- eines Enzyms E₃₁, welches die Umsetzung von Glycerin-3-Phosphat zu Dihydroxyacetonphosphat katalysiert,
- eines Enzyms E₃₂, welches die Übertragung von Schwefel auf den Schwefelsakzeptor Thioredoxin 1 katalysiert,
- eines Enzyms E₃₃, welches die Hydrolyse von Phospholipiden unter Bildung von Alkoholen und Glycerin katalysiert,
- eines Enzyms E₃₄, welches den Transport von Glycerin-3-Phosphat in die Zelle hinein im Austausch gegen Phosphat katalysiert;
- eines Enzyms E₃₅, welches die Umsetzung von Dihydroxyacetonphosphat zu Glycerinaldehyd-3-Phosphat katalysiert,
- eines Enzyms E₃₆, welches die Umsetzung von Glycerinal-dehyd-3-Phosphat zu 1,3-Biphosphoglycerat katalysiert,
- eines Enzyms E₃₇, welches die Umsetzung von 1,3-Biphosphoglycerat zu 3-Phosphoglycerat katalysiert,
- eines Enzyms E₃₈, welches die Umsetzung von 3-Phosphoglycerat zu 2-Phosphoglycerat katalysiert,
- eines Enzyms E₃₉, welches die Umsetzung von 2-Phosphoglycerat zu Phosphoenolpyruvat katalysiert,
- eines Enzyms E₄₀ welches die Umsetzung von Phosphoenolpyruvat zu Pyruvat katalysiert,
- eines Enzyms E₄₁, welches die Umsetzung von Glycerin zu Dihydroxyaceton katalysiert,
- eines Enzyms E₄₂, welches die Umsetzung von Dihydroxyaceton zu Dihydroxyacetonphosphat katalysiert.

Erfindungsgemäß besonders bevorzugte Zellen sind dabei diejenigen, bei denen die Aktivität folgender Enzyme oder Enzymkombinationen vermindert ist: E₂₉, E₃₀, E₃₁, E₃₂, E₃₃, E₃₄, E₃₅, E₃₆, E₃₇, E₃₈, E₃₉, E₄₀, E₄₁, E₄₂, E₂₉E₃₀, E₂₉E₃₁, E₂₉E₃₂, E₂₉E₃₃, E₂₉E₃₄, E₂₉E₃₅, E₂₉E₃₆, E₂₉E₃₇, E₂₉E₃₈, E₂₉E₃₉, E₂₉E₄₀, E₂₉E₄₁, E₂₉E₄₂, E₃₀E₃₁, E₃₀E₃₂, E₃₀E₃₃, E₃₀E₃₄, E₃₀E₃₅, E₃₀E₃₆, E₃₀E₃₇, E₃₀E₃₈, E₃₀E₃₉, E₃₀E₄₀, E₃₀E₄₁, E₃₀E₄₂, E₃₁E₃₂, E₃₁E₃₃, E₃₁E₃₄, E₃₁E₃₅, E₃₁E₃₆, E₃₁E₃₇, E₃₁E₃₈, E₃₁E₃₉, E₃₁E₄₀, E₃₁E₄₁, E₃₁E₄₂, E₃₂E₃₃, E₃₂E₃₄, E₃₂E₃₅, E₃₂E₃₆, E₃₂E₃₇, E₃₂E₃₈, E₃₂E₃₉, E₃₂E₄₀₁ E₃₂E₄₁, E₃₂E₄₂, E₃₃E₃₄, E₃₃E₃₅, E₃₃E₃₆, E₃₃E₃₇, E₃₃E₃₈, E₃₃E₃₉, E₃₃E₄₀, E₃₄E₄₁, E₃₃E₄₂, E₃₄E₃₅, E₃₄E₃₆, E₃₄E₄₇, E₃₄E₃₈, E₃₄E₃₉, E₃₄E₄₀, E₃₄E₄₁, E₃₄E₄₂, E₃₅E₃₆, E₃₅E₃₇, E₃₅E₃₈, E₃₅E₃₉, E₃₅E₄₀, E₃₅E₄₁, E₃₅E₄₂, E₃₆E₃₇, E₃₆E₃₈, E₃₆E₃₉, E₃₆E₄₀, E₃₆E₄₁, E₃₆E₄₂, E₃₇E₃₈, E₃₇E₃₉, E₃₇E₄₀, E₃₇E₄₁, E₃₇E₄₂, E₃₆E₃₉, E₃₉E₄₀, E₃₉E₄₁, E₃₉E₄₂, E₄₀E₄₁, E₄₀E₄₂, E₄₁E₄₂ und E₂₉E₃₀E₃₁E₃₂E₃₃E₃₄E₃₅E₃₆E₃₇E₃₈E₃₉-E₄₀E₄₁E₄₂.

In diesem Zusammenhang ist es insbesondere bevorzugt, dass das Enzym
- E₂₉: ein Aquaglyceroporin (Glycerol-Facilitator), der vorzugsweise vom glpF-Gen kodiert wird,
- E₃₀: eine Glycerin-Kinase (EC 2.7.1.30), die vorzugsweise vom glpK-Gen kodiert wird,
- E₃₁: eine Glycerin-3-Phosphat-Dehydrogenase (EC 1.1.99.5), vorzugsweise eine FAD-abhängige Glycerin-3-Phosphat-Dehydrogenase, wobei die Glycerin-3-Phosphat-Dehydrogenase vorzugsweise von dem glpA-Gen, dem glpB-Gen, dem glpC-Gen oder dem glpD-Gen, besonders bevorzugt dem glpD-Gen kodiert wird,
- E₃₂: eine Schwefeltransferase, welche von dem glpE-Gen kodiert wird,
- E₃₃: eine Glycerinphosphodiesterase (EC 3.1.4.46), welche vorzugsweise vom glpQ-Gen kodiert wird,
- E₃₄: eine Glycerin-3-Phosphat-Permease, welche vorzugsweise vom glpT-Gen kodiert wird
- E₃₅: eine Triosephosphat-Isomerase (EC 5.3.1.1),
- E₃₆: eine Glyceraldehyd-3-Phosphat-Deydrogenase (EC 1.2.1.12),
- E₃₇: eine Phosphoglycerat-Kinase (EC 2.7.2.3),
- E₃₈: eine Phosphoglycerat-Mutase (EC 5.4.2.1),
- E₃₉: eine Enolase (EC 4.2.1.11),
- E₄₀: eine Pyruvat-Kinase (EC 2.7.1.40),
- E₄₁: eine Glycerin-Dehydrogenase (EC 1.1.1.6), die vorzugsweise vom gldA-Gen kodiert wird, und
- E₄₂: eine Dihydroxyaceton-Kinase (EC 2.7.1.29), die vorzugsweise vom dhaK-Gen kodiert wird,
ist.

Die Gensequenzen der vorstehend genannten Enzyme können wiederum aus den dem Fachmann bekannten Gen-Datenbanken, insbesondere der KEGG-Datenbank, der NCBI-Datenbank oder EMBL-Datenbank, entnommen werden.

Des Weiteren sind das Glyceraldehyd-3-Phosphat-Dehydrogenase kodierende gap-Gen (Eikmanns (1992), Journal of Bacteriology 174: 6076-6086), das für die Triosephosphat-Isomerase codierende tpi-Gen (Eikmanns (1992), Journal of Bacteriology 174: 6076-6086) und das für die 3-Phosphoglycerat-Kinase codierende pgk-Gen (Eikmanns (1992), Journal of Bacteriology 174: 6076-6086) auch aus anderen Quellen bekannt.

Mit den bekannten Genen der Enzyme E₂₉ bis E₄₂ lassen sich gentechnisch veränderte Zellen, in denen mindestens eine, besonders bevorzugt mindestens zwei, darüber hinaus bevorzugt mindestens drei und am meisten bevorzugt alle Aktivitäten der Enzyme E₂₉ bis E₄₂ durch die eingangs im Zusammenhang mit dem Enzym E₁ beschriebenen Techniken (Mutation des Enzyms oder Steigerung der Enzymexpression) erhöht worden ist, herstellen. Diese Zellen sind in der Lage, in Gegenwart von Glycerin als einziger Kohlenstoffquelle (oder aber zusammen mit Kohlehydraten als weitere Kohlenstoffquelle) kultiviert werden zu können.

Neben der Erhöhung einer oder mehrerer der Enzymaktivitäten E₂₉ bis E₄₂ kann es, wenn die Zelle Glycerin als Kohlenstoffquelle zu verwerten vermag, auch vorteilhaft sein, wenn in den im erfindungsgemäßen Verfahren eingesetzten Zellen folgende Gene vorzugsweise heterolog exprimiert werden:
- das glpG-Gen oder das 3925-Gen,
- das glpX-Gen,
- das dhaR-Gen, das ycgU-Gen oder das b1201-Gen
- das fsa-Gen, das mipB-Gen, das ybiZ-Gen oder das B0825-Gen
- das talC-Gen, das fsaB-Gen, das yijG-Gen oder das b3946-Gen.

Die Nukleotidsequenzen dieser Gene können wiederum der KEGG-Datenbank, der NCBI-Datenbank oder EMBL-Datenbank entnommen werden entnommen werden.

Sofern die Zellen Kohlenhydrate als Nährstoffquelle verwerten können, ist es bevorzugt, dass die in dem erfindungsgemäßen Verfahren eingesetzte Zelle eine im Vergleich zu ihrem Wildtyp gesteigerte Aktivität mindestens eines, vorzugsweise aller folgenden Enzyme E₄₃ bis E₄₅ und E₃₆ bis E₄₀ aufweist:
- eines Enzyms E₄₃, welches die Umsetzung von α-D-Glukose-6-Phosphat zu β-D-Fruktose-6-Phosphat katalysiert,
- eines Enzyms E₄₄, welches die Umsetzung von β-D-Fruktose-6-Phosphat zu β-D-Fruktose-1,6-Biphosphat katalysiert,
- eines Enzyms E₄₅, welches die Umsetzung von β-D-Fruktose-1,6-Biphosphat zu Glycerinaldehyd-3-Phosphat und Dihydroxyacetonphosphat katalysiert,
- eines Enzyms E₃₆, welches die Umsetzung von Glycerinaldehyd-3-Phosphat zu 1,3-Biphosphoglycerat katalysiert,
- eines Enzyms E₃₇, welches die Umsetzung von 1,3-Biphosphoglycerat zu 3-Phosphoglycerat katalysiert,
- eines Enzyms E₃₈, welches die Umsetzung von 3-Phosphoglycerat zu 2-Phosphoglycerat katalysiert,
- eines Enzyms E₃₉, welches die Umsetzung von 2-Phosphoglycerat zu Phosphoenolpyruvat katalysiert, und
- eines Enzyms E₄₀, welches die Umsetzung von Phosphoenolpyruvat zu Pyruvat katalysiert.

Erfindungsgemäß besonders bevorzugte gentechnisch veränderte Zellen sind dabei diejenigen, bei denen die Aktivität folgender Enzyme oder Enzymkombinationen gesteigert ist: E₃₆, E₃₇, E₃₈, E₃₉, E₄₀, E₄₃, E₄₄, E₄₅, E₃₆E₃₇, E₃₆E₃₈, E₃₆E₃₉, E₃₆E₄₀, E₃₆E₄₃, E₃₆E₄₄, E₃₆E₄₅, E₃₇E₃₈, E₃₇E₃₉, E₃₇E₄₀, E₃₇E₄₃, E₃₇E₄₄, E₃₇E₄₅ᵣ E₃₈E₃₉, E₃₈E₄₀, E₃₈E₄₃, E₃₈E₄₄, E₃₈E₄₅, E₃₉E₄₀, E₃₉E₄₃, E₃₉E₄₄, E₃₉E₄₅, E₄₀E₄₃, E₄₀E₄₄, E₄₀E₄₅, E₄₃E₄₄, E₄₃E₄₅, E₄₄E₄₅ und E₃₆E₃₇E₃₈E₃₉₋E₄₀E₄₃E₄₄E₄₅.

In diesem Zusammenhang ist es insbesondere bevorzugt, dass das Enzym
- E₄₃: eine Glucose-6-Phosphat-Isomerase (EC 5.3.1.9),
- E₄₉: eine 6-Phosphofrukto-Kinase (EC 2.7.1.11),
- E₄₅: eine Fruktose-Bisphosphat-Aldolase (EC 4.1.2.13),
- E₃₆: eine Glyceraldehyd-3-Phosphat-Deydrogenase (EC 1.2.1.12),
- E₃₇: eine Phosphoglycerat-Kinase (EC 2.7.2.3),
- E₃₈: eine Phosphoglycerat-Mutase (EC 5.4.2.1),
- E₃₉: eine Enolase (EC 4.2.1.11) und
- E₄₀: eine Pyruvat-Kinase (EC 2.7.1.40),
ist.

Die Nukleotidsequenzen dieser Gene können wiederum der KEGG-Datenbank, der NCBI-Datenbank oder EMBL-Datenbank entnommen werden entnommen werden.

Weiterhin ist es, wenn die Zelle Kohlenhydrate als Kohlenstoffquelle zu verwerten vermag, bevorzugt, dass neben der Aktivität der vorstehend genannten Enzyme E₄₃ bis E₄₅ und E₃₆ bis E₄₀ auch die Aufnahme von Glukose in die Zellen erhöht wird, beispielsweise durch die Erhöhung der Aktivität von Enzymen des Phosphotransferase-Systems, insbesondere derjenigen Enzyme, die von ptsI-, ptsH- und ptsM-Genen kodiert werden, oder durch die Verstärkung der Glukokinase (EC 2.7.1.2), welche vorzugsweise durch das glk-Gen kodiert wird. In diesem Zusammenhang wird insbesondere auf US 6, 680, 187, US 6, 818, 432, US 6, 913, 910 und US 6,884,614 verwiesen.

Auch kann es im Falle von Kohlenhydraten als Kohlenstoffquelle vorteilhaft sein, gezielt den Pentosephosphat-Weg zu fördern, beispielsweise durch Erhöhung der Aktivität der Glucose-6-Phosphat-Dehydrogenase (EC 1.1.1.49) und der 6-Phosphogluconat-Dehydrogenase (EC 1.1.1.44), welche vorzugsweise vom gnd-Gen kodiert wird, und gegebenenfalls gleichzeitig die Glykolyse zu hemmen, beispielsweise durch Abschwächung der Aktivität der Glucose-6-Phosphat-Isomerase, wie dieses in WO-A-01/07626 beschrieben ist.

Sofern die Zellen 3-Hydroxyisobuttersäure oder auf 3-Hydroxyisobuttersäure basierende Polyhydroxyalkanoate gemäß der besonderen Ausgestaltung des erfindungsgemäßen Verfahrens, bei der eine gentechnisch veränderte Zelle eingesetzt wird, in der Methylmalonat-Semialdehyd als Vorprodukt und Succinyl-Coenzym A als Zwischenprodukt gebildet werden, über Oxalacetat und Pyruvat als Zwischenprodukte bilden, kann es weiterhin bevorzugt sein, die Aktivität mindestens eines, vorzugsweise alle nachfolgenden Enzymaktivitäten in der Zelle zu vermindern:
- eines Enzyms, welches die Umsetzung von Oxaloacetat zu Phosphoenolpyruvat katalysiert, wie etwa die Phosphoenylpyruvat-Carboxy-Kinase (EC 4.1.1.49) (siehe auch DE-A-199 50 409),
- eines Enzyms, welches die Umsetzung von Pyruvat zu Acetat katalysiert, wie etwa die Pyruvat-Oxidase (EC 1.2.2.2) (siehe auch DE-A-199 51 975),
- eines Enzyms, welches die Umsetzung von α-D-Glukose-6-Phosphat zu β-D-Fruktose-6-Phosphat katalysiert (siehe auch US 09/396,478),
- eines Enzyms, welches die Umsetzung von Pyruvat zu Lactat katalysiert, wie etwa die 1-Lactat-Dehydrogenase (EC 1.1.1.27) oder die Lactat-Malat-Transhydrogenase (EC 1.1.99.7),
- eines Enzyms, welches die Umsetzung von Pyruvat zu Acetyl-Coenzym A katalysiert, wie etwa die Pyruvat-Dehydrogenase (EC 1.2.1.51),
- eines Enzyms, welches die Umsetzung von Pyruvat zu Acetylphosphat katalysiert, wie etwa die Pyruvat-Oxidase (EC 1.2.3.3),
- eines Enzyms, welches die Umsetzung von Pyruvat zu Acetat katalysiert, wie etwa die Pyruvat-Dehydrogenase (EC 1.2.2.2),
- eines Enzyms, welches die Umsetzung von Pyruvat zu Phosphoenol-Pyruvat katalysiert, wie etwa die Phosphoenolpyruvat-Synthase (EC 2.7.9.2) oder die Pyruvat-Phosphat-Dikinase (EC 2.7.9.1),
- eines Enzyms, welches die Umsetzung von Pyruvat zu Alanin katalysieren, wie etwa die Alanin-Transaminase (2.6.1.2) oder die Alanin-Oxosäure-Transaminase (EC 2.6.1.12) katalysiert, und/oder
- eines Enzyms, welches Pyruvat zu Acetolactat umsetzt, wie etwa die Acetohydroxysäure-Synthase (EC 2.2.1.6).

Erfindungsgemäß besonders bevorzugte Zellen, welche 3-Hydroxyisobuttersäure oder auf 3-Hydroxyisobuttersäure basierende Polyhydroxyalkanoate über Succinyl-Coenzym A als Zwischenprodukt aus Kohlenhydraten als Kohlenstoffquelle bilden können und in denen eine oder mehrere der vorstehend genannten Enzymaktivitäten, insbesondere eine der Enzymaktivitäten E₁ bis E₄₅, darüber hinaus bevorzugt die Enzymaktivitäten E₁, E₁E₂E₃E₄, E₁E₄E₅E₆E₇ oder und E₁E₄E₅E₇ erhöht werden können, sind diejenigen Mikroorganismen, die durch Bennett et al., Metab. Eng. (2005), 7 (3), Seiten 229 bis 239, Bennett et al., Biotechnol. Bioeng. (2005), 90 (6), Seiten 775 bis 779, Bennett et al., Biotechnol. Prog. (2005), 21 (2), Seiten 358 bis 365, Bennett et al. (2005), Appl. Microbiol. Biotechnol., 67 (4), Seiten 515 bis 523, Vemuri et al. (2002), Applied and Environmental Microbiology 68 (4), Seiten 1.715 bis 1.727 und in US 6,455,284 beschrieben sind.

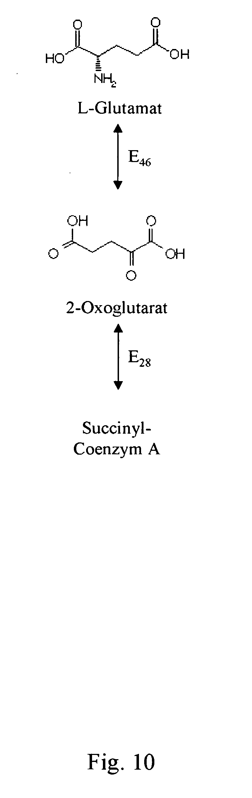

Erfolgt gemäß der ersten besonderen Ausführungsform des erfindungsgemäßen Verfahrens die Bildung der 3-Hydroxyisobuttersäure oder des auf 3-Hydroxyisobuttersäure basierenden Polyhydroxyalkanoates über Succinyl-Coenzym A als Zwischenprodukt aus L-Glutamat als Kohlenstoffquelle, so ist es gemäß einer weiteren besonderen Ausführungsform des erfindungsgemäßen Verfahrens, bei der eine gentechnisch veränderte Zelle eingesetzt wird, in der Methylmalonat-Semialdehyd als Vorprodukt und Succinyl-Coenzym A als Zwischenprodukt gebildet werden, erfindungsgemäß weiterhin bevorzugt, dass die eingesetzte Zelle eine im Vergleich zu ihrem Wildtyp gesteigerte Aktivität mindestens eines der, vorzugsweise der beiden folgenden Enzyme E₂₈ und E₄₆ aufweist (siehe die Figur 10):
- eines Enzyms E₄₆, welches die Umsetzung von L-Glutamat zu 2-Oxoglutarat katalysiert;
- eines Enzyms E₂₈, welches die Umsetzung von 2-Oxoglutarat zu Succinyl-Coemzym A katalysiert.

In diesem Zusammenhang ist es besonders bevorzugt, dass das Enzym
- E₄₆: eine Glutamat-Synthase (EC 1.4.1.13 oder EC 1.4.1.14), eine Glutamat-Dehydrogenase (EC 1.4.1.2, EC 1.4.1.3 oder EC 1.4.1.4) oder eine Aspartat-Transaminase (EC 2.6.1.1 oder EC 2.6.1.2) und
- E₂₈: eine 2-Oxoglutarat-Synthase (EC 1.2.7.3)
ist.

Als Enzym E₂₈ sind diejenigen bevorzugt, die bereits Eingangs als bevorzugte Enzyme E₂₈ genannt worden sind.

Das Enzym E₄₆ wird vorzugsweise von den Genen ausgewählt aus der Gruppe umfassend myn8., glt1, adr290wp, gltB, gltD, yeiT, aegA, ygfT, gltD-1, gltD-2, glt1, glt2, gls1, gltA, glt, glxD, gltA, yerD, cg10184, cgl0185, sc3c9.12, gdh1, gdh2, ag140cp, gdhA, gdhA1, gdhA2, gluD, rocG, ypcA, gudB, gluD, gdhA, gdhA2, gdh, gdhA-1, gdhA2-2, gdhA-3, gluD1, gluD2, glud1-prov, glud1a, t11I18.2, t2I1.150, mrg7.13, got1, got2, caspat, got2-prov, xr406-prov, 406-prov, cg4233, cg4233, cg8430, cg8430, f23n19,17, f13j11.16, t26c19.9, f7f1.18, f10n7.200, t1611.170, f15n18.110, t20d1.70, aat, aat1, aat2, abl038wp, afr211cp, agx1, bnA4, aatA, aatB, ybdL, aspC, yfbQ, ydcR, avtA2, aspC-1, aspC-2, aspC-3, aspC-4, aspB, aspB-1, aspB-2, aspB-3, aspB-4, argD1, argD2, aatAc, ywfG, mtnV, alaT, avtA1, avtA2, avtA3, cgl0240, cgl1103,cgl2599, cgl2844, dapC, 2sck36.07c, sc9e12.21, sc2h4.04c, aspB1, aspB2, aspB3, tyrB, gpt, gpt1, gpt2, mgc82097, cg1640, c32f10.8, f20d23.34, f26f24.16, f24j13.15, t10d10.20 und agrwp.

Die Nukleotidsequenzen dieser Gene können wiederum der KEGG-Datenbank, der NCBI-Datenbank oder EMBL-Datenbank entnommen werden entnommen werden.

Gemäß einer zweiten besonderen Ausführungsform des erfindungsgemäßen Verfahrens, bei der eine gentechnisch veränderte Zelle eingesetzt wird, in der die Bildung von 3-Hydroxyisobuttersäure oder von auf 3-Hydroxyisobuttersäure basierenden Polyhydroxyalkanoaten über Methylmalonat-Semialdehyd als Vorprodukt erfolgt, ist es bevorzugt, dass die Bildung der 3-Hydroxyisobuttersäure oder des auf 3-Hydroxyisobuttersäure basierenden Polyhydroxyalkanoates über Propionyl-Coenzym A als Zwischenprodukt erfolgt, wobei die eingesetzte Zelle vorzugsweise Kohlenhydrate, Glycerin, Methan oder Methanol als Kohlenstoffquelle zu verwerten vermag. Dabei gibt es verschiedene Wege, um vom Propionyl-Coenzym A zur 3-Hydroxyisobuttersäure bzw. zu auf 3-Hydroxyisobuttersäure basierenden Polyhydroxyalkanoaten zu gelangen.

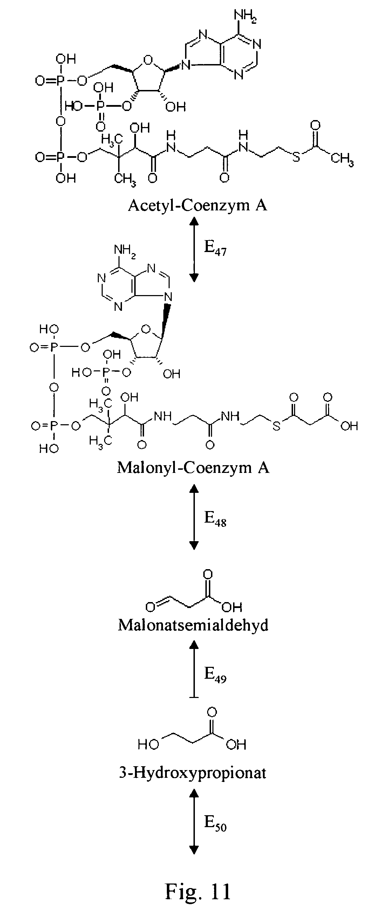

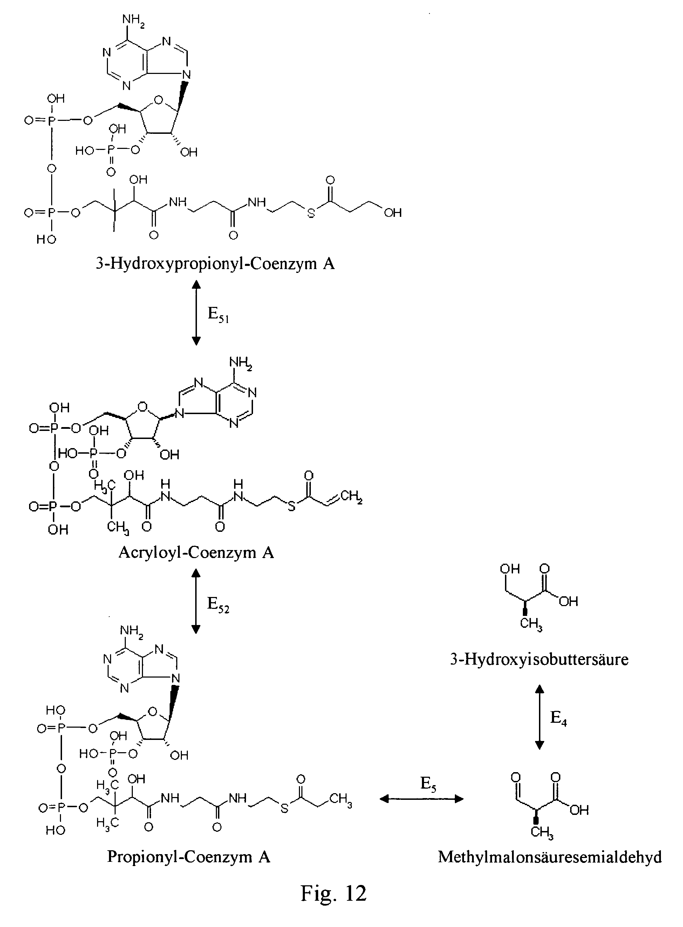

Gemäß einer ersten Alternative dieser zweiten besonderen Ausführungsform des erfindungsgemäßen Verfahrens erfolgt die Bildung des Zwischenproduktes Propionyl-Coenzym A über Acetyl-Coenzym A als weiteres Zwischenprodukt. In diesem Zusammenhang ist es insbesondere bevorzugt, dass die eingesetzte gentechnisch veränderte Zelle eine im Vergleich zu ihrem Wildtyp gesteigerte Aktivität mindestens eines der folgenden Enzyme E₄, E₅ und E₄₇ bis E₅₂ aufweist (siehe die Figuren 11 und 12):
- eines Enzyms E₄₇, welches die Umsetzung von Acetyl-Coenzym A zu Malonyl-Coenzym A katalysiert;
- eines Enzyms E₄₈, welches die Umsetzung von Malonyl-Coenzym A zu Malonatsemialdehyd katalysiert;
- eines Enzyms E₄₉, welches die Umsetzung von Malonatsemialdehyd zu 3-Hydroxypropionat katalysiert;
- eines Enzyms E₅₀, welches die Umsetzung von 3-Hydroxypropionat zu 3-Hydroxypropionyl-Coenzym A katalysiert;
- eines Enzyms E₅₁, welches die Umsetzung von 3-Hydroxypropionyl-Coenzym A zu Acryloyl-Coenzym A katalysiert;
- eines Enzyms E₅₂, welches die Umsetzung von Acryloyl-Coenzym A zu Propionyl-Coenzym A katalysiert;
- eines Enzyms E₅, welches die Umsetzung von Propionyl-Coenzym A zu Methylmalonatsemialdehyd katalysiert;
- eines Enzyms E₄, welches die Umsetzung von Methylmalonatsemialdehyd zu 3-Hydroxyisobutyrat katalysiert.

Erfindungsgemäß besonders bevorzugt werden gentechnisch veränderte Zellen eingesetzt, bei denen die Aktivität folgender Enzyme oder Enzymkombinationen gesteigert ist: E₄₇, E₄₈, E₄₉, E₅₀, E₅₁, E₅₂, E₄, E₅ und E₄₇E₄₈E₄₉E₅₀E₅₁E₅₂E₄E₅.

Weiterhin ist es in diesem Zusammenhang insbesondere bevorzugt, dass das Enzym
- E₄: eine 3-Hydroxyisobutyrat-Dehydrogenase (EC 1.1.1.31) oder eine 3-Hydroxyacyl-Coenzym A-Dehydrogenase (EC 1.1.1.35),
- E₅: eine Methylmalonatsemialdehyd-Dehydrogenase (EC 1.2.1.27),
- E₄₇: eine Malonyl-Coenzym A-Decarboxylase (EC 4.1.1.9), eine Malonat-Coenzym A-Transferase (EC 2.8.3.3), eine Methylmalonyl-Coenzym A-Carboxytransferase (EC 2.1.3.1) oder eine Acetyl-Coenzym A-Carboxylase (EC 6.4.1.2),
- E₄₈: eine Malonatsemialdehyd-Dehydrogenase (EC 1.2.1.18),
- E₄₉: eine 3-Hydroxypropionat-Dehydrogenase (EC 1.1.1.59),
- E₅₀: eine 3-Hydroxyisobutyryl-Coenzym A Hydrolase (EC 3.1.2.4),
- E₅₁: eine Enoyl-Coenzym A-Hydratase (EC 4.2.1.17) und
- E₅₂: eine Acyl-Coenzym A-Dehydrogenase (EC 1.3.99.3),
ist.

Bevorzugt Gene für die Enzyme E₄ und E₅ sind diejenigen, die bereits vorstehend im Zusammenhang mit der ersten besonderen Ausführungsform der erfindungsgemäßen Zelle beschrieben worden sind.

Das Enzym E₄₇ wird vorzugsweise von Genen ausgewählt aus der Gruppe umfassend mlycd, t19b17.4, tb08.2904.110, matA, acac, acaca, acacb, f5j5.21, f15c21.2, t8p21.5, acc1, aar071wp, accA, accB, accC, accD, accC1, accC2, mmdA, fabG, accD1, accD2, accD3, cgl0831, accBC, dtsR1, accDA, scc24.16c und cgl1327 kodiert, wobei accA, accC und accD am meisten bevorzugt sind.

Das Enzym E₄₈ wird vorzugsweise vom iolD-Gen kodiert.

Das Enzym E₅₁ wird vorzugsweise von Genen ausgewählt aus der Gruppe umfassend echS1, ehhadh, hadha, echs1-prov, cg4389, cg4389, cg6543, cg6984, cg8778, ech-1, ech-2, ech-3, ech-4, ech-5, ech-6, ech-7, FCAALL.314, fcaall.21, fox2, eci1,-eci2, paaF, paaG, yfcX, fadB, faoA, rpfF, phaA, phaB, echA1, echA2, echA3, echA4, echA5, echA6, echA7, echA8, e-chA9, echA9, echA10, echA11, echA12, echA13, echA14, e-chA15, echA16, echA17, echA18, echA19, echA20, echA21, fad-1, fad-2, fad-3, dcaE, hcaA, fadJ, rsp0671, rsp0035, rsp0648, rsp0647, rs03234, rs03271, rs04421, rs04419, rs02820, rs02946, paaG1, paaG2, paaG3, ech, pksH, ydbS, eccH1, eccH2, pimF, fabJ1, fabJ2, caiD2, ysiB, yngF, yusL, fucA, cgl0919, scf41.23, scd10.16, sck13.22, scp8.07c, stbac16h6.14, sc5f2a.15, sc6a5.38, hbd-1, hbd-2, hdb-3, hdb-4, hdb-5, hdb-6, hdb-7, hdb-8, hdb-9, hdb-10, fad-1, fad-2, fad-3, fad-4, fad-5, paaF-1, paaF-2, paaF-3, paaF-4, paaF-5, paaF-6, paaF-7 und crt kodiert.

Das Enzym E₅₂ wird vorzugsweise von Genen ausgewählt aus der Gruppe umfassend acadl, acadm, acad10, acad11, acadmprov, acadl-prov, mgc81873, cg12262, cg4703, cg4860, f3e22.5, afl213wp, acdC, fadE13, acd-1, acd-2, acd-3, acd-4, acd-5, acd-6, acd-7, acd-8, acd-9, acd-10, acd-11, acd-12, acd, fadE1, fadE2, fadE3, fadE4, fadE5, fadE6, fadE7, fadE13, fadE14, fadE15, fadE16, fadE17, fadE18, fadE19, fadE20, fadE21, fadE22, fadE23, fadE26, fadE27, fadE30, fadE31, fadE33, fadE35, fadE38, fadE45, fadE, caiA, aidB, RSp0036, RS03588, mmgC, acdA-3, bcd, acdA, acdH1, acdH2, acdH3, aidB, acdI und acdH kodiert.

Die Nukleotidsequenzen geeigneter Gene für die Enzyme E₄₇ bis E₅₂, insbesondere auch der Enzyme E₄₉ und E₅₀, können der KEGG-Datenbank, der NCBI-Datenbank oder EMBL-Datenbank entnommen werden.

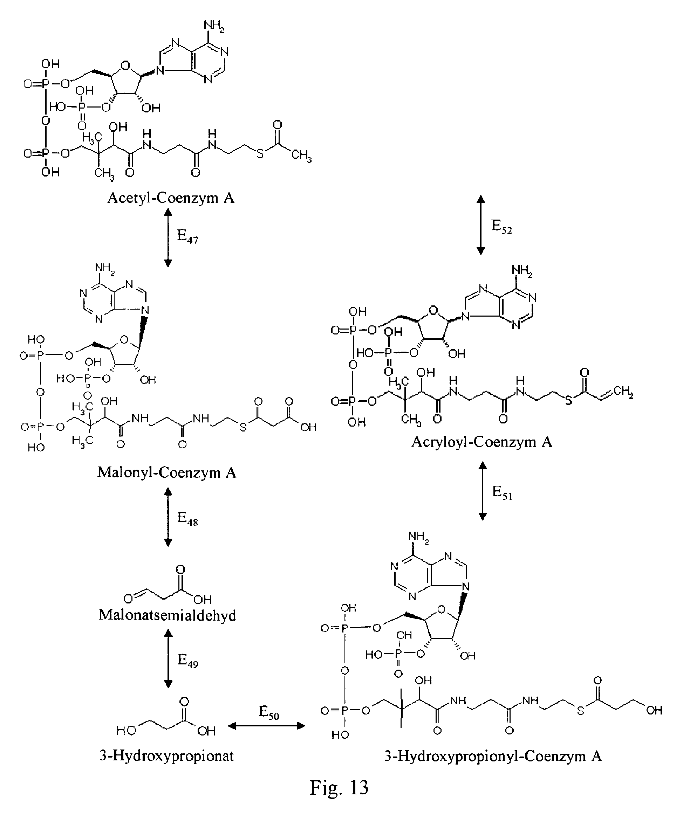

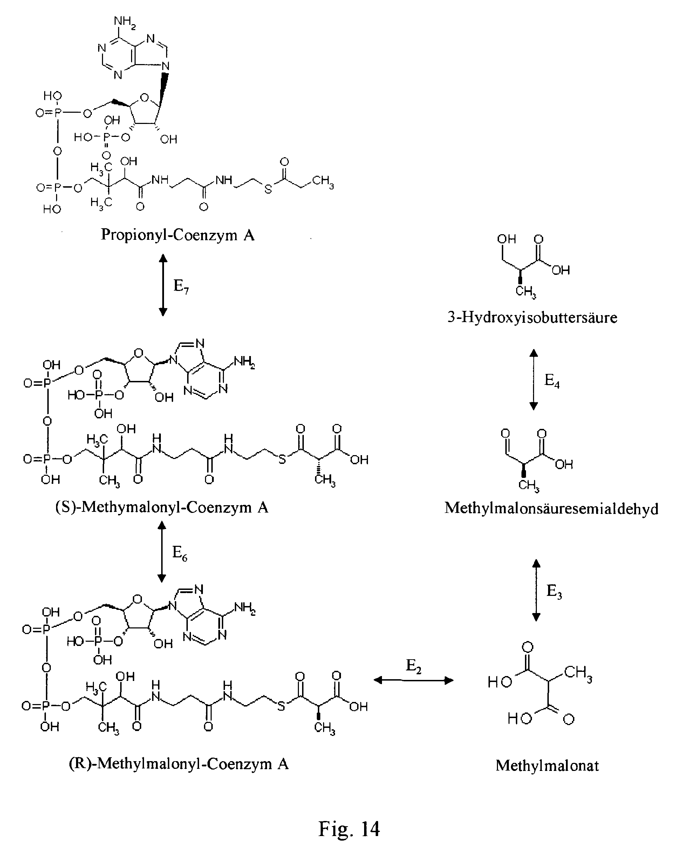

Gemäß einer zweiten Alternative dieser zweiten besonderen Ausführungsform des erfindungsgemäßen Verfahrens erfolgt die Bildung des Zwischenproduktes Propionyl-Coenzym A ebenfalls über Acetyl-Coenzym A als weiteres Zwischenprodukt, wobei gemäß dieser Alternative das Propionyl-Coenzym A nicht direkt, sondern über Methylmalonyl-Coenzym A zum Methylmalonatsemialdehyd umgesetzt wird. In diesem Zusammenhang ist es insbesondere bevorzugt, dass die eingesetzte gentechnisch veränderte Zelle eine im Vergleich zu ihrem Wildtyp gesteigerte Aktivität mindestens eines der folgenden Enzyme E₂ bis E₄, E₆, E₇ und E₄₇ bis E₅₂ aufweist (siehe die Figuren 13 und 14):
- eines Enzyms E₄₇, welches die Umsetzung von Acetyl-Coenzym A zu Malonyl-Coenzym A katalysiert;
- eines Enzyms E₄₈, welches die Umsetzung von Malonyl-Coenzym A zu Malonatsemialdehyd katalysiert;
- eines Enzyms E₄₉, welches die Umsetzung von Malonatsemialdehyd zu 3-Hydroxypropionat katalysiert;
- eines Enzyms E₅₀, welches die Umsetzung von 3-Hydroxypropionat zu 3-Hydroxypropionyl-Coenzym A katalysiert;
- eines Enzyms E₅₁, welches die Umsetzung von 3-Hydroxypropionyl-Coenzym A zu Acryloyl-Coenzym A katalysiert;
- eines Enzyms E₅₂, welches die Umsetzung von Acryloyl-Coenzym A zu Propionyl-Coenzym A katalysiert;
- eines Enzyms E₇, welches die Umsetzung von Propionyl-Coenzym A zu (S)-Methylmalonyl-Coenzym A katalysiert;
- eines Enzyms E₆, welches die Umsetzung von (S)-Methylmalonyl-Coenzym A zu (R)-Methylmalonyl-Coenzym A katalysiert;
- eines Enzyms E₂, welches die Umsetzung von (R)-Methylmalonyl-Coenzym A zu Methylmalonat katalysiert;
- eines Enzyms E₃, welches die Umsetzung von Methylmalonat zu Methylmalonatsemialdehyd katalysiert;
- eines Enzyms E₄, welches die Umsetzung von Methylmalonatsemialdehyd zu 3-Hydroxyisobutyrat katalysiert.

Erfindungsgemäß besonders bevorzugt werden gentechnisch veränderte Zellen eingesetzt, bei denen die Aktivität folgender Enzyme oder Enzymkombinationen gesteigert ist: E₂, E₃, E₄, E₆, E₇, E₄₇, E₄₈, E₄₉, E₅₀, E₅₁, E₅₂ und E₂E₃E₄E₆E₇E₄₇E₄₆E₄₈E₄₉E₅₀E₅₂.

Bevorzugte Enzyme und Gene dieser Enzyme sind diejenigen Gene und Enzyme, die bereits vorstehend im Zusammenhang mit den Enzymen E₂ bis E₄, E₆, E₇ und E₄₇ bis E₅₂ genannt worden sind.

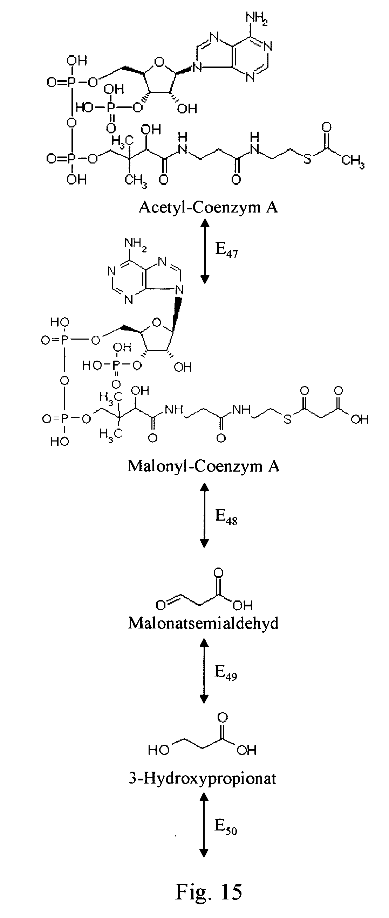

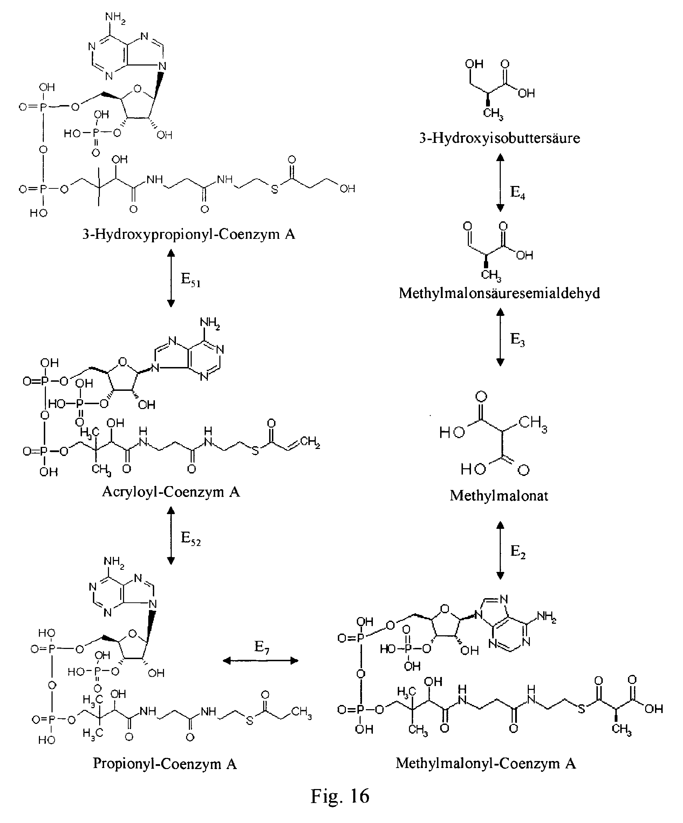

Gemäß einer dritten Alternative dieser ersten Alternative der zweiten besonderen Ausführungsform des erfindungsgemäßen Verfahrens erfolgt die Bildung des Zwischenproduktes Propionyl-Coenzym A ebenfalls über Acetyl-Coenzym A als weiteres Zwischenprodukt, wobei gemäß dieser Alternative das Propionyl-Coenzym A ebenfalls nicht direkt, sondern über (R)-Methylmalonyl-Coenzym A (und nicht über (S)-Methylmalonyl-Coenzym A) zum Methylmalonatsemialdehyd umgesetzt wird. In diesem Zusammenhang ist es insbesondere bevorzugt, dass die eingesetzte, gentechnisch veränderte Zelle eine im Vergleich zu ihrem Wildtyp gesteigerte Aktivität mindestens eines der folgenden Enzyme E₂ bis E₄, E₇ und E₄₇ bis E₅₂ aufweist (siehe die Figuren 15 und 16):
- eines Enzyms E₄₇, welches die Umsetzung von Acetyl-Coenzym A zu Malonyl-Coenzym A katalysiert;
- eines Enzyms E₄₈, welches die Umsetzung von Malonyl-Coenzym A zu Malonatsemialdehyd katalysiert;
- eines Enzyms E₄₉, welches die Umsetzung von Malonatsemialdehyd zu 3-Hydroxypropionat katalysiert;
- eines Enzyms E₅₀, welches die Umsetzung von 3-Hydroxypropionat zu 3-Hydroxypropionyl-Coenzym A katalysiert;
- eines Enzyms E₅₁, welches die Umsetzung von 3-Hydroxypropionyl-Coenzym A zu Acryloyl-Coenzym A katalysiert;
- eines Enzyms E₅₂, welches die Umsetzung von Acryloyl-Coenzym A zu Propionyl-Coenzym A katalysiert;
- eines Enzyms E₇, welches die Umsetzung von Propionyl-Coenzym A zu Methylmalonyl-Coenzym A katalysiert;
- eines Enzyms E₂, welches die Umsetzung von Methylmalonyl-Coenzym A zu Methylmalonat katalysiert;
- eines Enzyms E₃, welches die Umsetzung von Methylmalonat zu Methylmalonatsemialdehyd katalysiert;
- eines Enzyms E₄, welches die Umsetzung von Methylmalonatsemialdehyd zu 3-Hydroxyisobutyrat katalysiert.

Erfindungsgemäß besonders bevorzugt werden gentechnisch veränderte Zellen eingesetzt, bei denen die Aktivität folgender Enzyme oder Enzymkombinationen gesteigert ist: E₂, E₃, E₄, E₇, E₄₇, E₄₈, E₄₉, E₅₀, E₅₁, E₅₂ und
E₂E₃E₄E₇E₄₇E₄₈E₄₉E₅₀E₅₁E₅₂.

Bevorzugte Enzyme und Gene auch dieser Enzyme sind wiederum diejenigen Gene und Enzyme, die bereits vorstehend im Zusammenhang mit den Enzymen E₂ bis E₄, E₇ und E₄₇ bis E₅₂ genannt worden sind.

Gemäß einer vierten Alternative der zweiten besonderen Ausführungsform des erfindungsgemäßen Verfahrens erfolgt die Bildung des Zwischenproduktes Propionyl-Coenzym A ebenfalls über Acetyl-Coenzym A als weiteres Zwischenprodukt, wobei gemäß dieser Alternative Acetoacetyl-Coenzym A als Zwischenprodukt gebildet wird. In diesem Zusammenhang kann es bevorzugt sein, dass die eingesetzte, gentechnisch veränderte Zelle eine im Vergleich zu ihrem Wildtyp gesteigerte Aktivität mindestens eines der folgenden Enzyme E₈ und E₅₃ bis E₆₁ aufweist
- eines Enzyms E₅₃, welches die Umsetzung von zwei Acetyl-Coenzym A-Einheiten zu Acetoacetyl-Coenzym A katalysiert;
- eines Enzyms E₅₉, welches die Umsetzung von Acetoacetyl-Coenzym A zu 3-Hydroxybutanoyl-Coenzym A katalysiert;
- eines Enzyms E₅₅, welches die Umsetzung von 3-Hydroxybutanoyl-Coenzym A zu Crotonyl-Coenzym A katalysiert;
- eines Enzyms E₅₆, welches die Umsetzung von Crotonyl-Coenzym A zu Butyryl-Coenzym A katalysiert;
- eines Enzyms E₅₇, welches die Umsetzung von Butyryl-Coenzym A zu Ethylmalonyl-Coenzym A katalysiert;
- eines Enzyms E₅₈, welches die Umsetzung von Ethylmalonyl-Coenzym A zu Methylsuccinyl-Coenzym A katalysiert;
- eines Enzyms E₅₉, welches die Umsetzung von Methylsuccinyl-Coenzym A Isobutyryl-Coenzym A katalysiert;
- eines Enzyms E₆₀, welches die Umsetzung von Isobutyryl-Coenzym A zu Methacrylyl-Coenzym A katalysiert;
- eines Enzyms E₆₁, welches die Umsetzung von Methacrylyl-Coenzym A zu 3-Hydroxyisobutyryl-Coenzym A katalysiert;
- eines Enzyms E₈, welches die Umsetzung von 3-Hydroxyisobutyryl-Coenzym A zu 3-Hydroxyisobutyrat katalysiert.

Erfindungsgemäß besonders bevorzugte gentechnisch veränderte Zellen sind dabei diejenigen, bei denen die Aktivität folgender Enzyme oder Enzymkombinationen gesteigert ist: E₈, E₅₃, E₅₄, E₅₅, E₅₆, E₅₇, E₅₈, E₅₉, E₆₀, E₆₁ und E₈E₅₃E₅₄E₅₅E₅₆E₅₇E₅₈E₅₉E₆₀E₆₁.

Dieser Stoffwechselweg und die an diesem Stoffwechselweg beteiligten Enzyme sind beispielsweise in Korotkova et al., *Journal of Bacteriology* (2002), Seiten 1.750 bis 1.758 beschrieben.

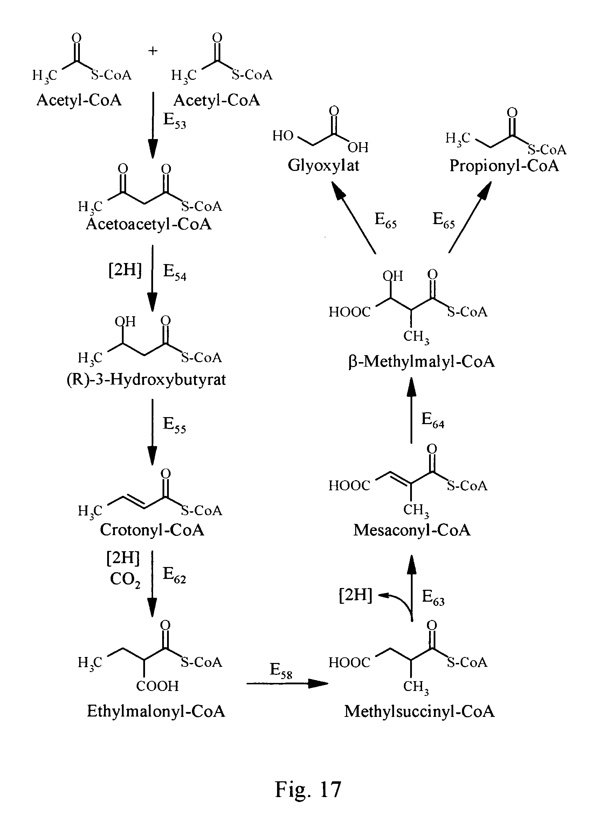

Gemäß einer fünften Alternative der zweiten besonderen Ausführungsform des erfindungsgemäßen Verfahrens erfolgt die Bildung des Zwischenproduktes Propionyl-Coenzym A wiederum über Acetyl-Coenzym A als weiteres Zwischenprodukt, wobei gemäß dieser Abwandlung ebenfalls Acetoacetyl-Coenzym A als weiteres Zwischenprodukt gebildet wird, wobei jedoch in diesem Fall aus Crotonyl-Coenzym A direkt Ethylmalonyl-Coenzym A gebildet wird. In diesem Zusammenhang kann es bevorzugt sein, dass die Zelle eine im Vergleich zu ihrem Wildtyp gesteigerte Aktivität mindestens eines der folgenden Enzyme E₈, E₅₃ bis E₅₅, E₅₈ und E₆₂ bis E₆₅ aufweist (siehe die Figur 17):
- eines Enzyms E₅₃, welches die Umsetzung von zwei Acetyl-Coenzym A-Einheiten zu Acetoacetyl-Coenzym A katalysiert;
- eines Enzyms E₅₄, welches die Umsetzung von Acetoacetyl-Coenzym A zu 3-Hydroxybutyryl-Coenzym A katalysiert;
- eines Enzyms E₅₅, welches die Umsetzung von 3-Hydroxybutyryl-Coenzym A zu Crotonyl-Coenzym A katalysiert;
- eines Enzyms E₆₂, welches die Umsetzung von Crotonyl-Coenzym A zu Ethylmalonyl-Coenzym A katalysiert
- eines Enzyms E₅₈, welches die Umsetzung von Ethylmalonyl-Coenzym A zu Methylsuccinyl-Coenzym A katalysiert;
- eines Enzyms E₆₃, welches die Umsetzung von Methylsuccinyl-Coenzym A zu Mesaconyl-Coenzym A katalysiert;
- eines Enzyms E₆₄, welches die Umsetzung von Mesaconyl-Coenzym A zu β-Methylmalyl-Coenzym A katalysiert;
- eines Enzyms E₆₅, welches die Umsetzung von β-Methylmalyl-Coenzym A zu Glyoxylat und Propionyl-Coenzym A katalysiert.

Aus dem Propionyl-Coenzym A kann dann auf die vorstehend beschriebenen Art und Weisen (Erhöhung der Aktivität eines oder mehrerer der Enzyme E₇, E₂, E₃ und E₄, Erhöhung der Aktivität eines oder mehrerer der Enzyme E₇, E₆, E₂, E₃ und E₄ oder Erhöhung der Aktivität eines der oder beider Enzyme E₄ und E₅) 3-Hydroxyisobuttersäure gebildet werden.

In diesem Zusammenhang ist es besonders bevorzugt, dass das Enzym
- E₅₃: eine β-Kethothiolase (EC 2.3.1.9),
- E₅₄: Acetoacetyl-Coenzym A-Reduktase (eine EC 1.1.1.36),
- E₅₅: eine Enoyl-Coenzym A-Hydratase (EC 4.2.1.17),
- E₆₂: eine Crotonyl-Coenzym A-Decarboylase,
- E₅₈: eine Ethylmalonyl-Coenzym A-Mutase (EC 5.4.99.2),
- E₆₃: eine Methylsuccinyl-Coenzym A-Dehydrogenase,
- E₆₄: eine Mesaconyl-Coenzym A-Hydratase, und
- E₆₅: eine β-Methylmalyl/L-Malyl-Coenzym A-Lyase
ist.

Das Enzym E₅₃ wird vorzugsweise von Genen ausgewählt aus der Gruppe bestehend aus acat1, acat2, loc484063, loc489421, mgc69098, mgc81403, mgc81256, mgc83664, kat-1, erg10, ygeF, atoB, fadAx, phbA-1, phbA-2, atoB-2, pcaF, pcaF-2, phb-A, bktB, phaA, tioL, thlA, fadA, paaJ, phbAf, pimB, mmgA, yhfS, th1, vraB, th1, mvaC, thiL, paaJ, fadA3, fadA4, fadA5, fadA6, cgl12392 catF, sc8f4.03, thiL1, thiL2, acaB1, acaB2, acaB3 oder acaB4 kodiert, wobei acat1, acat2, atoB und phbA sowie das entsprechende Gen aus *Rhodobacter sphaeroides* besonders bevorzugt sind.

Das Enzym E₅₄ wird vorzugsweise von Genen ausgewählt aus der Gruppe bestehend aus phbB, fabG, phbN1, phbB2 oder cgl12444 kodiert, wobei phbB besonders sowie das entsprechende Gen aus *Rhodobacter sphaeroides* besonders bevorzugt sind.

Das Enzym E₅₅ wird vorzugsweise von Genen ausgewählt aus der Gruppe bestehend aus echS1, ehhadh, hadha, echs1-prov, cg4389, cg4389, cg6543, cg6984, cg8778, ech-1, ech-2, ech-3, ech-4, ech-5, ech-6, ech-7, FCAALL.314, fcaall.21, fox2, eci1, eci2, paaF, paaG, yfcX, fadB, faoA, rpfF, phaA, phaB, echA1, echA2, echA3, echA4, echA5, echA6, echA7, echA8, e-chA9, echA9, echA10, echA11, echA12, echA13, echA14, e-chA15, echA16, echA17, echA18, echA19, echA20, echA21, fad-1, fad-2, fad-3, dcaE, hcaA, fadJ, rsp0671, rsp0035, rsp0648, rsp0647, rs03234, rs03271, rs04421, rs04419, rs02820, rs02946, paaG1, paaG2, paaG3, ech, pksH, ydbS, eccH1, eccH2, pimF, fabJ1, fabJ2, caiD2, ysiB, yngF, yusL, fucA, cgl0919, scf41.23, scd10.16, sck13.22, scp8.07c, stbac16h6.14, sc5f2a.15, sc6a5.38, hbd-1, hbd-2, hdb-3, hdb-4, hdb-5, hdb-6, hdb-7, hdb-8, hdb-9, hdb-10, fad-1, fad-2, fad-3, fad-4, fad-5, paaF-1, paaF-2, paaF-3, paaF-4, paaF-5, paaF-6, paaF-7 und crt kodiert, wobei das entsprechende Gen aus *Rhodobacter sphaeroides* besonders bevorzugt ist.

Geeignete Gene für das Enzym E₅₈ sind ausgewählt aus der Gruppe bestehend aus mut, mutA, mutB, sbm, sbmA, sbmB, sbm5, bhbA, mcmA, mcmA1, mcmA2, mcmB, mcm1, mcm2, mcm3, icmA, meaA1 und meaA2, wobei auch hier das entsprechende Gen aus *Rhodobacter sphaeroides* besonders bevorzugt ist.

Als Enzym E₅₂ wird vorzugsweise ein Enzym aus *Rhodobacter sphaeroides* eingesetzt, welches von DNA-Sequenz mit der Seq.-ID.-Nr. 05 kodiert wird und welches die Aminosäuresequenz gemäß Seq.-ID.-Nr. 06 aufweist.

Bevorzugte Gene für die Enzyme E₆₃, E₆₄ und E₆₅ sind insbesondere die Gene für diese Enzyme aus *Rhodobacter sphaeroides.*

Weitere Beispiele für Nukleotidsequenzen der vorstehend genannten Gene können unter anderem auch der KEGG-Datenbank, der NCBI-Datenbank oder EMBL-Datenbank entnommen werden.

Wie bereits vorstehend erläutert, entsteht bei der ersten Alternative der zweiten bevorzugten Ausführungsform des erfindungsgemäßen Verfahrens die 3-Hydroxyisobuttersäure oder die auf 3-Hydroxyisobuttersäure basierenden Polyhydroxyalkanoate über Propionyl-Coenzym A und Acetyl-Coenzym A als Zwischenprodukte. Dabei kann es grundsätzlich sinnvoll sein, neben einer Beeinflussung einer oder mehrerer der vorstehend genannten Enzymaktivitäten E₂ bis E₈ und E₄₇ bis E₆₅ auch solche Enzymaktivitäten zu beeinflussen, welche zu einer Erhöhung der Bildung von Acetyl-Coenzym A in der Zelle führen.

Wenn die 3-Hydroxyisobuttersäure aus Kohlehydraten oder aus Glycerin als Kohlenstoffquelle gebildet wird, so kann es bevorzugt sein, dass die Zelle eine gesteigerte Aktivität eines Enzyms E₆₆ aufweist, welches die Umsetzung von Pyruvat zu Acetyl-Coenzym A katalysiert. Bei diesem Enzym E₆₆ handelt es sich vorzugsweise um eine Pyruvat-Dehydrogenase (EC 1.2.1.51).

Wenn die 3-Hydroxyisobuttersäure aus C₁-Kohlenstoffquellen wie etwa Methan oder Methanol gebildet wird, so kann es bevorzugt sein, dass die Zelle eine im Vergleich zu ihrem Wildtyp gesteigerte Aktivität mindestens eines der Enzyme E₆₇ bis E₇₁ aufweist:
- eines Enzyms E₆₇, welches die Umsetzung von Methan zu Methanol katalysiert;
- eines Enzyms E₆₈, welches die Umsetzung von Methanol zu Formaldehyd katalysiert;
- eines Enzyms E₆₉, welches die Umsetzung von Formaldehyd zu 5,10-Methylenetetrahydrofolat katalysiert;
- eines Enzyms E₇₀, welches die Umsetzung von 5,10-Methylen-etetrahydrofolate zu 5-Methyltetrahydrofolat katalysiert;
- eines Enzyms E₇₁, welches die Umsetzung von 5-Methyltetrahydrofolat zu Acetyl-Coenzym A katalysiert.

In diesem Zusammenhang ist es besonders bevorzugt, dass das Enzym
- E₆₇: eine Methan-Monooxigenase (EC 1.14.13.25),
- E₆₈: eine Methanol-Dehydrogenase (EC 1.1.1.244),
- E₆₉: eine Methylmalonatsemialdehyd-Dehydrogenase (EC 1.2.1.27),
- E₇₀: eine Methylenetetrahydrofolat-Reductase (EC 1.5.1.20),
- E₇₁: eine Kohlenmonoxid-Dehydrogenase (EC 1.2.99.2)
ist.

Die Nukleotidsequenzen geeigneter Gene für die Enzyme E₆₃ bis E₆₇ können der KEGG-Datenbank, der NCBI-Datenbank oder EMBL-Datenbank entnommen werden.

Gemäß einer dritten besonderen Ausführungsform des erfindungsgemäßen Verfahrens, bei der eine gentechnisch veränderte Zelle eingesetzt wird, in der die Bildung von 3-Hydroxyisobuttersäure oder von auf 3-Hydroxyisobuttersäure basierenden Polyhydroxyalkanoaten über Methylmalonat-Semialdehyd als Vorprodukt erfolgt, ist es bevorzugt, dass die Bildung der 3-Hydroxyisobuttersäure oder des auf 3-Hydroxyisobuttersäure basierenden Polyhydroxyalkanoates über Acryloyl-Coenzym -A als Zwischenprodukt erfolgt, wobei die eingesetzte Zelle vorzugsweise Kohlenhydrate, Glycerin oder Glutamat als Kohlenstoffquelle zu verwerten vermag.

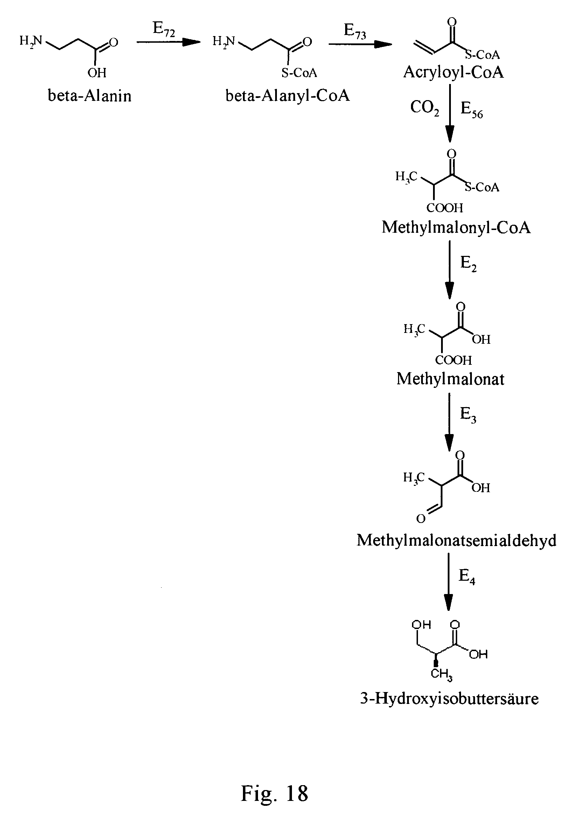

Im Zusammenhang mit der dritten besonderen Ausführungsform des erfindungsgemäßen Verfahrens ist es insbesondere bevorzugt, wenn die eingesetzte, gentechnisch veränderte Zelle eine im Vergleich zu ihrem Wildtyp gesteigerte Aktivität mindestens eines der nachfolgenden Enzyme E₂ bis E₄, E₆₂, E₇₂ und E₇₃ aufweist (siehe die Figur 18):
- eines Enzyms E₇₂, welches die Umsetzung von Beta-Alanin zu Beta-Alanyl-Coenzym A katalysiert,
- eines Enzyms E₇₃, welches die Umsetzung von Beta-Alanyl-Coenzym A zu Acrylyl-Coenzym A katalysiert,
- eines Enzyms E₆₂, welches die Umsetzung von Acrylyl-Coenzym A zu Methylmalonyl-Coenzym A (oder die Umsetzung von Crotonyl-Coenzym A zu Ethylmalonyl-Coenzym A) katalysiert,
- eines Enzyms E₂, welches die Umsetzung von Methylmalonyl-Coenzym A zu Methylmalonat katalysiert;
- eines Enzyms E₃, welches die Umsetzung von Methylmalonat zu Methylmalonat-Semialdehyd katalysiert;
- eines Enzyms E₄, welches die Umsetzung von Methylmalonat-Semialdehyd zu 3-Hydroxyisobuttersäure katalysiert.

Erfindungsgemäß besonders bevorzugte Zellen sind dabei diejenigen, bei denen die Aktivität folgender Enzyme oder Enzymkombinationen gesteigert ist: E₆₂E₂, E₆₂E₃, E₆₂E₄, E₆₂E₂E₃ und E₇₂E₇₃E₆₂E₂E₃E₄. Auch im Zusammenhang mit der vierten besonderen Ausführungsform des erfindungsgemäßen Verfahrens kann es vorteilhaft sein, dass eine gentechnisch veränderte Zelle eingesetzt wird, in der ein Enzym überexprimiert wird, welches mindestens zwei der vorstehend beschriebenen Reaktionsschritte zu katalysieren vermag. So kann auch hier beispielsweise ein Enzym eingesetzt werden, welches sowohl die Aktivität des Enzyms E₂ als auch die des Enzyms E₃ aufweist, wie beispielsweise die Malonyl-Coenzym A-Reduktase aus *Sulfolobus tokodaii,* welche von der DNA-Sequenz mit der Seq.-ID.-Nr. 03 kodiert wird und welche die Aminosäuresequenz gemäß Seq.-ID.-Nr. 04 aufweist. Weiterhin ist es im Zusammenhang mit der vierten besonderen Ausführungsform der erfindungsgemäßen Zelle grundsätzlich auch möglich, eine Zelle einzusetzen, die bereits in der Lage ist, besonders große Mengen an Acrylyl-Coenzym A zu bilden, einzusetzen.

In diesem Zusammenhang ist es besonders bevorzugt, dass das Enzym
- E₇₂: eine Coenzym A-Transferase (EC 2.8.3.1) oder Coenzym A-Synthetase, vorzugsweise eine Coenzym A-Transferase,
- E₇₃: eine Beta-Alanyl-Coenzym A-Ammonium-Lysase (EC 4.3.1.6),
- E₆₂: eine Crotonyl-Coenzym A-Decarboxylase
- E₂: eine Methylmalonyl-Coenzym A-Hydrolase (EC 3.1.2.17),
- E₃: eine Aldehyd-Dehydrogenase (EC 1.2.1.3) oder eine Aldehyd-Oxidase (EC 1.2.3.1) und
- E₄: eine 3-Hydroxyisobutyrat-Dehydrogenase (EC 1.1.1.31) oder eine 3-Hydroxyacyl-Coenzym A-Dehydrogenase (EC 1.1.1.35)
ist.

Bevorzugte Enzyme E₇₂ mit einer CoA-Transferaseaktivität sind diejenigen aus *Megasphaera elsdenii, Clostridium propionicum, Clostridium kluyveri* und auch aus *Escherichia Co*li. Als Beispiele für eine eine CoA-Transferase kodierende DNA-Sequenz sei an dieser Stelle die in der WO-A-03/062173 mit der SEQ ID NO: 24 bezeichnete Sequenz aus *Megasphaera elsdenii* genannt. Des Weiteren bevorzugte Enzyme sind diejenigen Varianten der CoA-Transferase, die in der WO-A-03/062173 beschrieben werden.

Geeignete Enzyme E₇₃ mit einer Beta-Alanyl-Coenzym A-Ammonium-Lyase-Aktivität sind beispielsweise diejenigen aus *Clostridium propionicum.* DNA-Sequenzen, welche für ein solches Enzym kodieren, können beispielsweise aus *Clostridium propionicum,* wie im Beispiel 10 der WO-A-03/062173 beschrieben, erhalten werden. Die DNA-Sequenz, welche für die Beta-Alanyl-Coenzym A-Ammonium-Lyase aus *Clostridium propionicum* kodiert, ist in der WO-A-03/062173 als SEQ ID NO: 22 angegeben.

Als Enzym E₆₂ wird vorzugsweise wiederum die Crotonyl-Coenzym A-Decarboxylase aus *Rhodobacter sphaeroides* eingesetzt, welche von DNA-Sequenz mit der Seq.-ID.-Nr. 05 kodiert wird und welche die Aminosäuresequenz gemäß Seq.-ID.-Nr. 06 aufweist. Dieses Enzym ist nicht nur in der Lage, Crotonyl-Coenzym A zu Ethylmalonyl-Coenzym A, sondern auch Acrylyl-Coenzym A zu Methylmalonyl-Coenzym A umzusetzen.

Geeignete Gene für die Enzyme E₂ bis E₄ wurden bereits im Zusammenhang mit der ersten Variante des erfindungsgemäßen Verfahrens genannt, wobei es auch im Zusammenhang mit der zweiten Variante bevorzugt ist, als Gen für das Enzym E₃ das vorstehend beschriebene Gen aus *Sulfolobus tokodaii* besonders bevorzugt ist.

Gemäß einer besonders bevorzugten Variante der dritten besonderen Ausführungsform des erfindungsgemäßen Verfahrens wird eine gentechnisch veränderte Zelle eingesetzt, welche im Vergleich zu ihrem Wildtyp zumindest eine gesteigerte Aktivität der Enzyms E₂ und E₆₂ bzw. der Enzyme E₂, E₃ und E₆₂ aufweist, wobei das Enzym E₂ bzw. die Enzyme E₂ und E₃ von einer DNA-Sequenz gemäß SEQ.-ID-Nr. 03 und das Enzym E₆₂ von einer DNA-Sequenz gemäß SEQ.-ID-Nr. 05 kodiert wird. In diesem Zusammenhang ist es bevorzugt, wenn die gesteigerte Aktivität dieser beiden Enzyme dadurch erzielt wird, dass die Polypeptide mit SEQ.-ID-Nr. 04 und der SEQ.-ID-Nr. 06 oder aber dass Aminosäure-Sequenzen, die eine Identität von mindestens 50 %, vorzugsweise mindestens 55 %, darüber hinaus bevorzugt mindestens 60 %, darüber hinaus bevorzugt mindestens 65 % und am meisten bevorzugt mindestens 70 % zu den Aminosäure-Sequenz gemäß SEQ.-ID-Nr.: 04 bzw. SEQ.-ID-Nr. 06 aufweisen, in der Zelle überexpremiert werden. Dabei können diese beiden DNA-Sequenzen in das Genom der Zelle integriert sein oder auf einem Vektor im Inneren der Zelle vorliegen.

Im Zusammenhang mit der vorstehend beschriebenen, dritten besonderen Ausführungsform des erfindungsgemäßen Verfahrens kann es weiterhin vorteilhaft sein, wenn die eingesetzte, gentechnisch veränderte Zelle neben der Erhöhung der Aktivität des Enzyms E₆₂ und/oder der Aktivität des Enzyms E₂ bzw. der Enzyme E₂ und E₃ mindestens eine, vorzugsweise beide der folgenden Eigenschaften aufweist:
- eine im Vergleich zu ihrem Wildtyp gesteigerte Aktivität eines Enzyms E₁₁, welches die Umsetzung von Pyruvat zu Oxalacetat katalysiert oder eines Enzyms E₇₄, welches die Umsetzung von Phosphoenolpyruvat zu Oxalacetat katalysiert, vorzugsweise jedoch eines Enzyms E₁₁, welches die Umsetzung von Pyruvat zu Oxalacetat katalysiert sowie
- eine gesteigerte Aktivität eines Enzyms E₇₅, welches die Umsetzung von Aspartat zu Beta-Alanin katalysiert,

Bei dem Enzym E11 handelt es sich vorzugsweise um eine Carboxylase, besonders bevorzugt um eine Pyruvat-Carboxylase (EC-Nummer 6.4.1.1), welche die Umsetzung von Pyruvat zu Oxalacetat katalysiert. Eine in diesem Zusammenhang besonders bevorzugte Pyruvat-Carboxylase ist diejenige Mutante, die in *"*A novel methodology employing Corynebacterium glutamicum genome information to generate a new L-lysine-producing mutant.", Ohnishi J et al., Applied Microbiology and Biotechnology, Vol. 58 (2), Seiten 217-223 (2002) beschrieben ist. Bei dieser Mutation wurde die Aminosäure Prolin an Position 458 durch Serin ersetzt.

Bei dem Enzym E75 handelt es sich vorzugsweise um eine Decarboxylase, besonders bevorzugt um eine Glutamat-Decarboxylase oder eine Aspartat-Decarboxylase, wobei eine 1-Aspartat-1-Decarboxylase (EC-Nummer 4.1.1.11) am meisten bevorzugt ist, welche von dem panD-Gen kodiert wird. Die Aspartat-Decarboxylase katalysiert die Umsetzung von Aspartat zu Beta-Alanin. Gene für die Aspartat-Decarboxylase (panD-Gene) unter anderem aus Escherichia coli (FEMS Microbiology Letters, 143, Seiten 247-252 (1996)), "Photorhabdus luminescens subsp. Laumondii, Mycobacterium bovis subsp. Bovis") sowie aus zahlreichen anderen Mikroorganismen wurden bereits kloniert und sequenziert. Insbesondere die Nukleotidsequenz des panD-Gens aus Corynebacterium glutamicum ist in der DE-A-198 55 313 beschrieben. Grundsätzlich können panD-Gene jeder nur denkbaren Herkunft, gleichgültig ob aus Bakterien, Hefen oder Pilzen, verwendet werden. Weiterhin können alle Allele des panD-Gens verwendet werden, insbesondere auch diejenigen, die sich aus der Degeneriertheit des genetischen Codes oder durch funktionsneutrale Sinnmutationen (sense mutations) ergeben. Eine neben der Aspartat-Decarboxylase aus Corynebacterium glutamicum erfindungsgemäß besonders bevorzugte Aspartat-Decarboxylase ist die Escherichiacoli-Mutante DV9 (Vallari und Rock, Journal of Bacteriology, 164, Seiten 136-142 (1985)). Die Herstellung rekombinanter Zellen, in denen sowohl die Aktivität der Pyruvat-Carboxylase als auch die Aktivität der Aspartat-Decarboxylase erhöht ist, ist in DE-A-10 2005 048 818 beschrieben.

Gemäß einer zweiten Variante des erfindungsgemäßen Verfahrens erfolgt die Bildung von 3-Hydroxyisobuttersäure oder von auf 3-Hydroxyisobuttersäure basierenden Polyhydroxyalkanoaten über 3-Hyroxyisobutyryl-Coenzym A als Vorprodukt.

Wenn in dem erfindungsgemäßen Verfahren eine Zelle eingesetzt wird, in der die Bildung von 3-Hydroxyisobuttersäure oder von auf 3-Hydroxyisobuttersäure basierenden Polyhydroxyalkanoaten gemäß der zweiten Variante über 3-Hydroxyisobutyryl-Coenzym A als Vorprodukt erfolgt, so ist es gemäß einer ersten besonderen Ausführungsform bevorzugt, dass die Bildung der 3-Hydroxyisobuttersäure oder des auf 3-Hydroxyisobuttersäure basierenden Polyhydroxyalkanoates über Isobutyryl-Coenzym A als Zwischenprodukt erfolgt, wobei die Zelle vorzugsweise Kohlenhydrate, Glycerin oder L-Valin als Kohlenstoffquelle zu verwerten vermag.

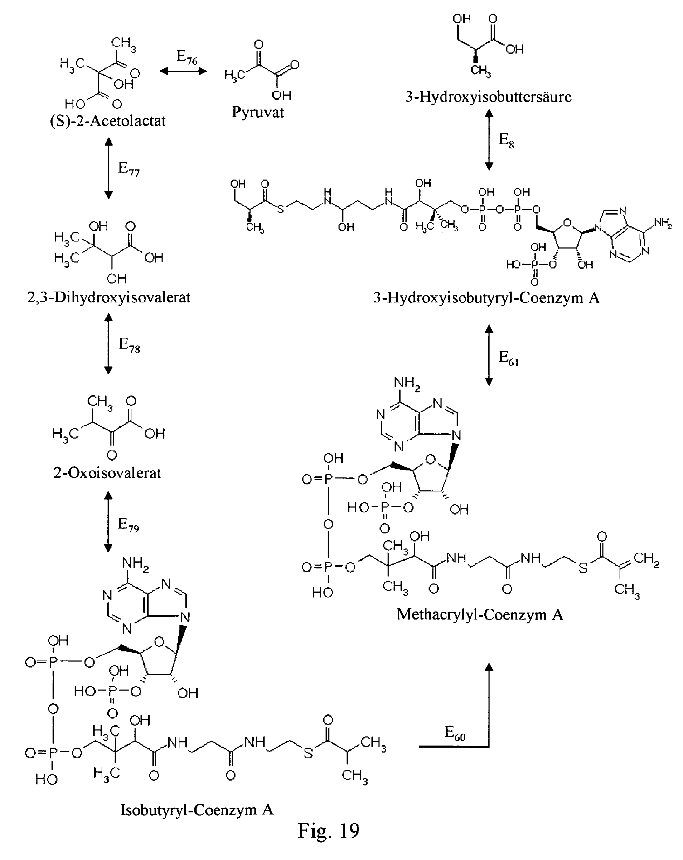

Sofern Kohlenhydrate oder Glycerin als Kohlenstoffquelle dienen, ist es gemäß einer ersten Alternative dieser ersten besonderen Ausführungsform der zweiten Variante des erfindungsgemäßen Verfahrens bevorzugt, eine gentechnisch veränderte Zelle einzusetzen, welche eine im Vergleich zu ihrem Wildtyp gesteigerte Aktivität mindestens eines der folgenden Enzyme E₇₆ bis E₇₉, E₆₀, E₆₁ und E₈ aufweist (siehe Figur 19):
- eines Enzyms E₇₆, welches die Umsetzung von Pyruvat zu 2-Acetolactat katalysiert;
- eines Enzyms E₇₇, welche die Umsetzung von 2-Acetolactat zu 2,3-Dihydroxyisolvalerat katalysiert;
- eines Enzyms E₇₈, welches die Umsetzung von 2,3-Dihydroxyisolvalerat zu 2-Oxoisovalerat katalysiert;
- eines Enzyms E₇₉, welches die Umsetzung von 2-Oxoisovalerat zu Isobutyryl-Coenzym A katalysiert;
- eines Enzyms E₆₀, welches die Umsetzung von Isobutyryl-Coenzym A zu Methacrylyl-Coenzym A katalysiert;
- eines Enzyms E₆₁, welches die Umsetzung von Methacrylyl-Coenzym A zu 3-Hydroxyisobutyryl-Coenzym A katalysiert;
- eines Enzyms Es, welches die Umsetzung von 3-Hydroxyisobutyryl-Coenzym A zu 3-Hydroxyisobutyrat katalysiert.

Erfindungsgemäß besonders bevorzugt werden gentechnisch veränderte Zellen eingesetzt, bei denen die Aktivität folgender Enzyme oder Enzymkombinationen gesteigert ist: E₈, E₆₀, E₆₁, E₇₆, E₇₇, E₇₈, E₇₉ und E₈E₆₀E₆₁E₇₆E₇₇E₇₈E₇₉.

In diesem Zusammenhang ist es besonders bevorzugt, dass das Enzym
- E₈: eine 3-Hydroxyisobutyryl-Coenzym A-Hydrolase (EC 3.1.2.4),
- E₇₆: eine Acetolactat-Synthase (EC 2.2.1.6),
- E₇₇: eine Dihydroxyisovalerat-Dehydrogenase (EC 1.1.1.86),
- E₇₈: eine 2,3-Dihydroxyisovalerat-Dehydratase (EC 4.2.1.9),
- E₇₉: eine 2-Oxoisovalerat-Dehydrogenase (EC 1.2.1.25 oder EC 1.2.4.4),
- E₆₀: eine Acyl-Coenzym A-Dehydrogenase (EC 1.3.99.3), eine Butyryl-Coenzym A-Dehydrogenase (EC 1.3.99.2) oder eine 2-Methylacyl-Coenzym A-Dehydrogenase (EC 1.3.99.12), und
- E₆₁: eine Enoyl-Coenzym A-Hydratase (EC 4.2.1.17)
ist.

Bevorzugt Enzyme E₈, E₆₀ und E₆₁ sind diejenigen, die bereits vorstehend beschrieben worden sind.

Das Enzym E₇₆ wird vorzugsweise von Genen ausgewählt aus der Gruppe umfassend ilvbl, t8p19.70, ilv1, ilv2, ilv6, aal021wp, ael305cp, ilvI, ilvH, ilvN, ilvB, ilvM, ilvG, ilvN, budB, ilvN-1, ilvN-2, atrC, ilvX, iolD, budB, alsS, ilvK, ilvB1, ilvB2, ilvB3, ilvN1, ilvN2, cgl1271, cgl1272, iolD und scc57A.40c kodiert.

Das Enzym E₇₇ wird vorzugsweise von Genen ausgewählt aus der Gruppe umfassend f14p22.200, ilv5, acl198Wp, ilvC, ilvY, ilvC-1, ilvC-2, ilvC-3 und cgl1273 kodiert, wobei das ilvC-Gen am meisten bevorzugt ist.

Das Enzym E₇₈ wird vorzugsweise von Genen ausgewählt aus der Gruppe umfassend f14o13.18, ilv3, acl117wp, ilvD, cgl1268 ilvD1 und ilvD2 kodiert, wobei ilvD am meisten bevorzugt ist.

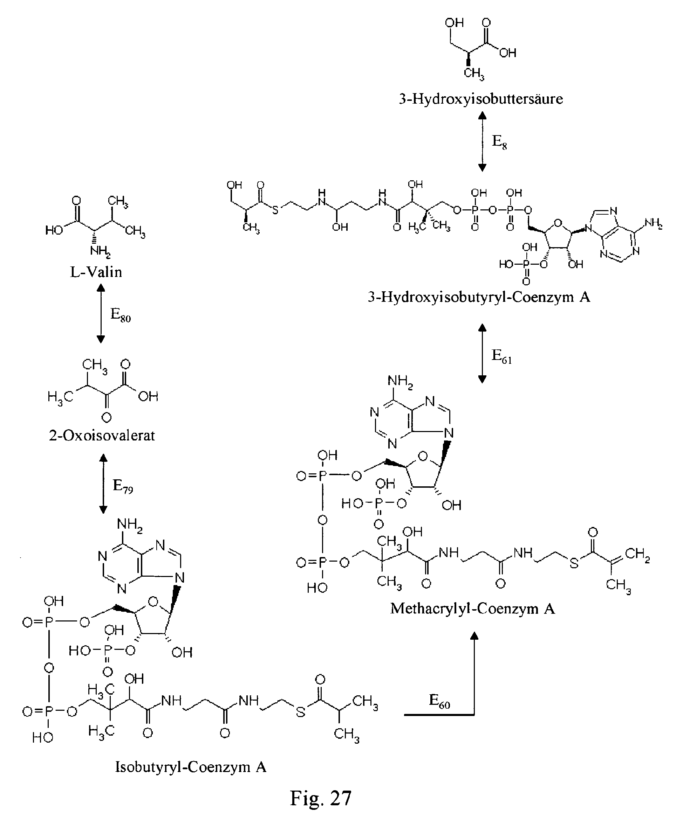

Sofern L-Valin als Kohlenstoffquelle dient, ist es gemäß einer zweiten Abwandlung der ersten besonderen Ausführungsform der zweiten Alternative des erfindungsgemäßen Verfahrens, bei der eine gentechnisch veränderte Zelle eingesetzt wird, in der die Bildung von 3-Hydroxyisobuttersäure oder von auf 3-Hydroxyisobuttersäure basierenden Polyhydroxyalkanoaten über 3-Hydroxyisobutyryl-Coenzym A als Vorprodukt und Isobutyryl-Coenzym A als Zwischenprodukt erfolgt, bevorzugt, dass diese eingesetzte Zelle eine im Vergleich zu ihrem Wildtyp gesteigerte Aktivität mindestens eines der folgenden Enzyme E₇₉, E₈₀, E₆₀, E₆₁ und E₈ aufweist (siehe die Figur 20):
- eines Enzyms E₈₀, welches die Umsetzung von L-Valin zu 2-Oxoisovalerat katalysiert;
- eines Enzyms E₇₉, welches die Umsetzung von 2-Oxoisovalerat zu Isobutyryl-Coenzym A katalysiert;
- eines Enzyms E₆₀, welches die Umsetzung von Isobutyryl-Coenzym A zu Methacrylyl-Coenzym A katalysiert;
- eines Enzyms E₆₁, welches die Umsetzung von Methacrylyl-Coenzym A zu 3-Hydroxyisobutyryl-Coenzym A katalysiert;
- eines Enzyms E₈, welches die Umsetzung von 3-Hydroxyisobutyryl-Coenzym A zu 3-Hydroxyisobutyrat katalysiert.

Erfindungsgemäß besonders bevorzugt werden gentechnisch veränderte Zellen eingesetzt, bei denen die Aktivität folgender Enzyme oder Enzymkombinationen gesteigert ist: E₈, E₆₀, E₆₁, E₇₉, E₈₀ und E₈E₆₀E₆₁E₇₉E₈₀.

In diesem Zusammenhang ist es besonders bevorzugt, dass das Enzym
- E₈: eine 3-Hydroxyisobutyryl-Coenzym A-Hydrolase (EC 3.1.2.4),
- E₆₀: eine Acyl-Coenzym A-Dehydrogenase (EC 1.3.99.3), eine Butyryl-Coenzym A-Dehydrogenase (EC 1.3.99.2) oder eine 2-Methylacyl-Coenzym A-Dehydrogenase (EC 1.3.99.12),
- E₆₁: eine Enoyl-Coenzym A-Hydratase (EC 4.2.1.17),
- E₇₉: eine 2-Oxoisovalerat-Dehydrogenase (EC 1.2.1.25 oder EC 1.2.4.4), und
- E₈₀: eine Aminosäure-Transferase (EC 2.6.1.42)
ist.

Bevorzugte Enzyme E₈, E₆₀, E₆₁ und E₇₉ sind diejenigen, die bereits vorstehend beschrieben worden sind.

Das Enzym E₈₀ wird vorzugsweise von Genen ausgewählt aus der Gruppe umfassend bcat1, bcat2, t27I1.8, t27i1.9, f2j10.5, f2j10.4, t12h1.16, mmb12.20, t9c5.3, mpa24.13, bat1, bat2, adl384wp, eca39, bcaA, ilvE, ilvE1, ilvE2, ilvE3, ywaA, ybgE, bcaT und cgl2204 kodiert, wobei ilvE besonders bevorzugt ist.

Die Nukleotidsequenzen geeigneter Gene das Enzym E₈₀ können im wiederum der KEGG-Datenbank, der NCBI-Datenbank oder EMBL-Datenbank entnommen werden.

Im Zusammenhang mit dieser zweiten Alternative der ersten besonderen Ausführungsform der zweiten Variante des erfindungsgemäßen Verfahrens kann es weiterhin vorteilhaft sein, eine gentechnisch veränderte Zelle einzusetzen, in der die Aktivität eines Enzyms E₄, welches die Umsetzung von Methylmalonatsemialdehyd zu 3-Hydroxyisobuttersäure katalysiert, vermindert ist, wobei es sich bei diesem Enzym E₄ vorzugsweise um eine 3-Hydroxyisobutyrat-Dehydrogenase (EC 1.1.1.31) oder um eine 3-Hydroxyacyl-Coenzym A-Dehydrogenase (EC 1.1.1.35) handelt.

Weiterhin kann es gemäß der zweiten Abwandlung der ersten besonderen Ausführungsform der zweiten Variante des erfindungsgemäßen Verfahrens, bei der eine gentechnisch veränderte Zelle eingesetzt wird, in der die Bildung von 3-Hydroxyisobuttersäure oder von auf 3-Hydroxyisobuttersäure basierenden Polyhydroxyalkanoaten über 3-Hydroxyisobutyryl-Coenzym A als Vorprodukt und Isobutyryl-Coenzym A als Zwischenprodukt und ausgehend von L-Valin als Kohlenstoffquelle erfolgt, bevorzugt sein, solche Zellen einzusetzen, die bereits große Mengen an L-Valin bilden können. In Betracht kommen hier insbesondere diejenigen Zellen, die in Applied Environmental Microbiology, Vol. 73 (7) (2007), Seiten 2.079-2.084 von Blombach et al. beschrieben wurden.

Gemäß einer besonderen Ausführungsform des erfindungsgemäßen Verfahrens ist es weiterhin bevorzugt, dass die eingesetzte gentechnisch veränderte Zelle eine gegenüber ihrem Wildtyp gesteigerte Expression des glb0-Gens aufweist. Weiterhin kann es unter Umständen bevorzugt sein, dass die eingesetzte gentechnisch veränderte Zelle eine im Vergleich zu ihrem Wildtyp verminderte Aktivität des Citrat-Transportproteins, welches durch das dctA-Gen oder das citP-Gen kodiert wird, aufweist.

Die in dem erfindungsgemäßen Verfahren eingesetzte, gentechnisch veränderte Zelle ist vorzugsweise erhältlich durch ein Verfahren, umfassend den Verfahrensschritt der Erhöhung der Aktivität mindestens eines der vorstehend beschriebenen Enzyme, vorzugsweise eines oder mehrer der Enzyme
- E₁ bis E₄,
- E₁, E₄, E₅, E₆ und E₇,
- E₁, E₄, E₅ und E₇,
- E₄, E₅ und E₄₇ bis E₅₂,
- E₂ bis E₄, E₆, E₇ und E₄₇ bis E₅₂,
- E₂ bis E₄, E₇ und E₄₇ bis E₅₂,
- E₈ und E₅₃ bis E₆₁,
- E₈, E₅₃ bis E₅₅, E₅₈ und E₆₂ bis E₆₄,
- E₂ bis E₃, E₆₂, E₇₂ und E₇₃,
- E₈, E₆₀, E₆₁ und E₇₆ bis E₇₉, oder
- E₈, _{E60}, _{E61}, E₇₉ und E₈₀
in der Zelle, wobei das Erhöhen der Enzymaktivität vorzugsweise durch die eingangs beschriebenen Verfahren erfolgt.

Im Verfahrensschritt IA) des erfindungsgemäßen Verfahrens können die gentechnisch veränderten Zellen kontinuierlich oder diskontinuierlich im *batch*-Verfahren (Satzkultivierung) oder im *fed-batch*-Verfahren (Zulaufverfahren) oder *repeated-fed-batch*-Verfahren (repetitives Zulaufverfahren) zum Zwecke der Produktion von 3-Hydroxyisobutyrat oder von auf 3-Hydroxyisobutyrat basierenden Polyhydroxyalkanoaten mit dem Nährmedium in Kontakt und somit kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Als Kohlenstoffquelle können Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Methanol, Kohlenwasserstoffe wie Methan, Aminosäuren wie L-Glutamat oder L-Valin oder organische Säuren wie z. B. Essigsäure verwendet werden. Diese Stoffe können einzeln oder als Mischung verwendet werden. Besonders bevorzugt ist der Einsatz von Kohlehydraten, insbesondere Monosaccharide, Oligosaccharide oder Polysaccharide, wie dies in US 6,01,494 und US 6,136,576 beschrieben ist, von C₅-Zuckern oder von Glycerin.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen, wie z. B. Luft, in die Kultur eingetragen. Die Temperatur der Kultur liegt normalerweise bei 20 °C bis 45 °C und vorzugsweise bei 25 °C bis 40 °C. Insbesondere bei der Verwendung von Zellen, welche Glycerin als Substrat umwandeln können, kann es bevorzugt sein, als Zellen solche Zellen einzusetzen, die in der US 6,803,218 beschrieben werden. In diesem Fall können die Zellen bei Temperaturen im Bereich von 40 bis 100 °C kultiviert werden.

Die Aufreinigung der 3-Hydroxyisobuttersäure aus der Nährlösung erfolgt vorzugsweise kontinuierlich, wobei es in diesem Zusammenhang weiterhin bevorzugt ist, auch die Herstellung der 3-Hydroxyisobuttersäure durch Fermentation kontinuierlich durchzuführen, so dass der gesamte Prozess von der Herstellung der 3-Hydroxyisobuttersäure bis zur deren Aufreinigung aus der Fermentationsbrühe kontinuierlich durchgeführt werden kann. Zur kontinuierlichen Aufreinigung der Herstellung der 3-Hydroxyisobuttersäure aus der Fermentationsbrühe wird diese kontinuierlich über eine Vorrichtung zur Abtrennung der bei der Fermentation eingesetzten Mikroorganismen, vorzugsweise über einen Filter mit einer Ausschlussgröße in einem Bereich von 20 bis 200 kDa geführt, in dem eine Fest/Flüssig-Trennung stattfindet. Denkbar ist auch der Einsatz einer Zentrifuge, einer geeigneten Sedimentationsvorrichtung oder eine Kombination dieser Vorrichtungen, wobei es besonders bevorzugt ist, zumindest einen Teil der Mikroorganismen zunächst durch Sedimentation abzutrennen und anschließend die von den Mikroorganismen teilweise befreite Fermentationsbrühe einer Ultrafiltration oder Zentrifugationsvorrichtung zuzuführen.

Das hinsichtlich seines 3-Hydroxyisobuttersäure-Anteils angereicherte Fermentationserzeugnis wird nach der Abtrennung der Mikroorganismen einer vorzugsweise mehrstufigen Trennanlage zugeführt. In dieser Trennanlage sind mehrere hintereinander geschaltete Trennstufen vorgesehen, aus denen jeweils Rückführleitungen ausmünden, die zum Fermentationstank zurückgeführt sind. Weiterhin führen aus den jeweiligen Trennstufen Ableitungen heraus. Die einzelnen Trennstufen können nach dem Prinzip der Elektrodialyse, der Umkehrosmose, der Ultrafiltration oder der Nanofiltration arbeiten. In der Regel handelt es sich um Membran-Trenneinrichtungen in den einzelnen Trennstufen. Die Auswahl der einzelnen Trennstufen ergibt sich aus Art und Umfang der Gärungsnebenprodukte und Substratreste.

Neben der Abtrennung der 3-Hydroxyisobuttersäure mittels Elektrodialyse, Umkehrosmose, Ultrafiltration oder Nanofiltration, in deren Verlauf als Endprodukt eine wässrige 3-Hydroxyisobuttersäure-Lösung erhalten wird, kann die 3-Hydroxyisobuttersäure auch durch Extraktionsverfahren aus der von Mikroorganismen befreiten Fermentationslösung abgetrennt werden, wobei in diesem Fall letztendlich die reine 3-Hydroxyisobuttersäure erhalten werden kann. Zur Abtrennung der 3-Hydroxyisobuttersäure durch Extraktion können der Fermentationslösung beispielsweise Ammoniumverbindungen oder Amine zugesetzt werden, um ein Ammoniumsalz der 3-Hydroxyisobuttersäure zu bilden. Dieses Ammoniumsalz kann dann aus der Fermentationslösung abgetrennt werden, in dem ein organisches Extraktionsmittel zugesetzt und die so erhaltene Mischung anschließend erhitzt wird, wodurch das Ammoniumsalz sich in der organischen Phase anreichert. Aus dieser Phase kann die 3-Hydroxyisobuttersäure dann unter Erhalt der reinen 3-Hydroxyisobuttersäure beispielsweise durch weitere Extraktionsschritte isoliert werden. Genauere Einzelheiten bezüglich dieses Trennverfahrens sind der WO-A-02/090312 zu entnehmen, deren Offenbarungsgehalt hinsichtlich der Abtrennung von Hydroxycarbonsäuren aus Fermentationslösungen hiermit als Referenz eingeführt wird und einen Teil der Offenbarung der vorliegenden Anmeldung bildet.

Je nach Art und Weise der Abtrennung der 3-Hydroxyisobuttersäure aus der Fermentationslösung wird entweder eine wässrige 3-Hydroxyisobuttersäure-Lösung, beinhaltend 2 bis 90 Gew.-%, vorzugsweise 7,5 bis 50 Gew.-% und besonders bevorzugt 10 bis 25 Gew.-% an 3-Hydroxyisobuttersäure, oder aber reine 3-Hydroxyisobuttersäure erhalten.

Des Weiteren kann die im Schritt IA) des erfindungsgemäßen Verfahrens hergestellte 3-Hydroxyisobuttersäure vor, während oder nach der Aufreinigung noch neutralisiert werden, wobei hierzu Basen wie etwa Kalziumhydroxid oder Natriumhydroxid eingesetzt werden können.

Im Verfahrensschritt IB) des erfindungsgemäßen Verfahrens wird die 3-Hydroxyisobuttersäure unter Bildung von Methacrylsäure dehydratisiert, wobei hierzu entweder die aus der Fermenationslösung isolierte, reine 3-Hydroxyisobuttersäure oder aber die bei der Aufarbeitung der Fermenationslösung isolierte wässrige 3-Hydroxyisobuttersäure-Lösung eingesetzt werden kann, wobei diese gegebenenfalls noch vor der Dehydratisierung beispielsweise durch Destillation, gegebenenfalls in Gegenwart eines geeigneten Schleppmittels, aufkonzentriert wird.

Die Dehydratisierung kann grundsätzlich in flüssiger Phase oder in der Gasphase durchgeführt werden. Weiterhin ist es erfindungsgemäß bevorzugt, dass die Dehydratisierung in Gegenwart eines Katalysators erfolgt, wobei die Art des eingesetzten Katalysators davon abhängig ist, ob eine Gasphasen- oder eine Flüssigphasenreaktion durchgeführt wird.

Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x in Kontakt steht. Als Träger x kommen alle dem Fachmann als geeignet erscheinenden Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und Gesamtporenvolumen in einem Bereich von 0,1 bis 1,5 ml/g besonders bevorzugt. Zudem ist es bevorzugt, dass die als Träger x geeigneten Feststoffe eine Oberfläche im Bereich von 0,001 bis 1000 m²/g, vorzugsweise im Bereich von 0,005 bis 450 m²/g und darüber hinaus bevorzugt im Bereich von 0,01 bis 300 m²/g nach BET-Test gemäß DIN 66131 aufweisen. Als Träger für den Dehydratisierungskatalysator kann zum einen ein Schüttgut, das einen mittleren Teilchendurchmesser im Bereich von 0,1 bis 40 mm, vorzugsweise im Bereich von 1 bis 10 mm und darüber hinaus bevorzugt im Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x, die auch Dehydratisierungskatalysatoreigenschaften aufweisen können, eignen sich insbesondere natürliche oder synthetische silikatische Stoffe, wie insbesondere Mordenit, Montmorillonit, saure Zeolithe; mit ein-, zwei oder mehrbasigen anorganischen Säuren, insbesondere Phosphorsäure, oder saure Salze anorganischer Säuren belegte Trägerstoffe, wie oxidische oder silikatische Stoffe, beispielsweise Al₂O₃, TiO₂; Oxide und Mischoxide, wie beispielsweise γ-Al₂O₃ und ZnO-Al₂O₃-Mischoxide der Heteropolysäuren.

Es entspricht einer erfindungsgemäßen Ausführungsform, dass der Träger x mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x als auch als Dehydratisierungskatalysator wirkende Verbindungsklasse beinhalten Silizium/Aluminium/Phosphoroxide. Bevorzugte basische sowohl als Dehydratisierungskatalysator als auch als Träger x fungierende Stoffe beinhalten Alkali, Erdalkali, Lanthan, Lanthanoide oder eine Kombination von mindestens zwei davon in ihrer oxidischen Form. Derartige saure oder basische Dehydratisierungskatalysatoren sind sowohl bei der Degussa AG als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x durch Eintauchen bzw. Imprägnieren immobilisiert werden.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

Zudem ist es bevorzugt, dass in dem erfindungsgemäßen Verfahren ein Dehydratisierungskatalysator mit einem H₀-Wert im Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von - 3 bis -8,2 eingesetzt wird. Der H₀-Wert entspricht der Säurefunktion nach Hämmert und lässt sich durch die sogenannte Amin-Titration und Verwendung von Indikatoren oder durch Absorption einer gasförmigen Base ermitteln (siehe "Studies in Surface Science and Catalytics", Vol. 51, 1989: "New solid Acids and Bases, their catalytic Properties", K. Tannabe et al.

Gemäß einer besonderen Ausführungsform des erfindungsgemäßen Verfahrens wird als saurer Feststoffkatalysator ein mit einer anorganischen Säure, vorzugsweise mit Phosphorsäure oder mit Supersäuren wie etwa sulfatisiertem oder phosphatisiertem Zirkoniumoxid in Kontakt gebrachter porenförmiger Trägerkörper eingesetzt, der vorzugsweise zu mindestens 90 Gew.-%, darüber hinaus bevorzugt zu mindestens 95 Gew.-% und am meisten bevorzugt zu mindestens 99 Gew.-% auf einem Siliziumoxid, vorzugsweise auf SiO₂, basiert. Das in Kontakt bringen des porenförmigen Trägerkörpers mit der anorganischen Säure erfolgt vorzugsweise durch das Imprägnieren des Trägerkörpers mit der Säure, wobei diese vorzugsweise in einer Menge in einem Bereich von 10 bis 70 Gew.-%, besonders bevorzugt in einem Bereich 20 bis 60 Gew.-% und darüber hinaus bevorzugt in einem Bereich von 30 bis 50 Gew.-%, bezogen auf das Gewicht des Trägerkörpers, mit diesem in Kontakt gebracht und anschließend getrocknet wird. Im Anschluss an die Trocknung wird der Trägerkörper zur Fixierung der anorganischen Säure erhitzt, vorzugsweise auf eine Temperatur in einem Bereich von 300 bis 600 °C, darüber hinaus bevorzugt in einem Bereich von 400 bis 500 °C.

Gemäß einer besonderen Ausführungsform des erfindungsgemäßen Verfahrens wird die Dehydratisierung in der Gasphase durchgeführt. Dabei können übliche Apparaturen, wie sie dem Fachmann für Gasphasenreaktion bekannt sind, beispielsweise Röhrenreaktoren, eingesetzt werden. Besonders bevorzugt ist der Einsatz von Rohrbündelwärmeaustauschern sowie von Reaktoren, welche Thermobleche als Wärmeaustauscher beinhalten.

Gemäß einer Ausführungsform der Gasphasendehydratisierung wird reine 3-Hydroxyisobuttersäure in einen Reaktor umfassend einen der vorstehend genannten Festbettkatalysatoren eingebracht. Gemäß einer anderen Ausführungsform wird die 3-Hydroxyisobuttersäure in Form einer wässrigen Lösung beinhaltend 2 bis 80 Gew.-%, besonders bevorzugt 5 bis 50 Gew.-% und darüber hinaus bevorzugt 10 bis 25 Gew.-% an 3-Hydroxyisobuttersäure, jeweils bezogen auf das Gesamtgewicht der wässrigen Lösung, in den Reaktor eingebracht. Die Druck- und Temperaturbedingungen im Inneren des Reaktors werden so gewählt, dass die 3-Hydroxyisobuttersäure bzw. die wässrigen Lösung bei ihrem Eintritt in den Reaktor in gasförmiger Form vorliegt. Die Dehydratisierung in der Gasphase erfolgt vorzugsweise im Temperaturbereich zwischen 200 und 400°C, besonders bevorzugt zwischen 250 und 350°C. Der Druck im Inneren des Reaktors liegt bei der Gasphasendehydratisierung vorzugsweise in einem Bereich von 0,1 bis 50 bar, besonders bevorzugt in einem Bereich von 0,2 bis 10 bar und am meisten bevorzugt in einem Bereich von 0,5 bis 5 bar.

Die Menge an in den Reaktor eingebrachter 3-Hydroxyisobuttersäure liegt bei der Gasphasendehydratisierung vorzugsweise in einem Bereich von 10 bis 100 Vol-%, besonders bevorzugt in einem Bereich von 20 bis 100 Vol-% und am meisten bevorzugt in einem Bereich von 30 bis 100 Vol-%.

Gemäß einer anderen besonderen Ausführungsform des erfindungsgemäßen Verfahrens wird die Dehydratisierung in der Flüssigphase durchgeführt. Die Flüssigphasendehydratisierung kann ebenfalls in allen dem Fachmann bekannten Apparaturen durchgeführt werden, in denen ein Fluid auf eine gewünschte Reaktionstemperatur erhitzt werden kann, wobei die Apparatur mit einem Druck beaufschlagt werden kann, der ausreicht, um die Reaktionskomponenten unter den gewünschten Temperaturbedingungen flüssig zu halten.

Gemäß einer besonderen Ausführungsform des erfindungsgemäßen Verfahrens umfasst das Verfahren der Flüssigphasendehydratisierung einen ersten Verfahrensschritt, bei dem reine 3-Hydroxyisobuttersäure oder eine wässrige Lösung beinhaltend 5 bis 100 Gew.-%, besonders bevorzugt 20 bis 100 Gew.-% und am meisten bevorzugt 50 bis 100 Gew.-% an 3-Hydroxyisobuttersäure, bezogen auf das Gesamtgewicht der wässrigen Lösung, in einen Reaktor eingebracht wird. Die Druck- und Temperaturbedingungen im Inneren des Reaktors werden so gewählt, dass die 3-Hydroxyisobuttersäure bzw. die wässrigen Lösung bei ihrem Eintritt in den Reaktor in flüssiger Form vorliegt. Gemäß einer besonderen Ausführungsform des erfindungsgemäßen Verfahrens, bei dem eine Dehydratisierung in der Flüssigphase durchgeführt wird, wird die 3-Hydroxyisobuttersäure bzw. die wässrige Lösung im Inneren des Dehydratisierungsreaktors derart über ein Katalysatorfestbett geführt, dass die flüssige Phase über die Oberfläche der Katalysatorpartikel rieselt. Eine derartige Vorgehensweise kann beispielsweise in einem Rieselbettreaktor durchgeführt werden.

Die Dehydratisierung in der Flüssigphase erfolgt vorzugsweise im Temperaturbereich zwischen 200 und 350 °C, besonders bevorzugt zwischen 250 und 300 °C. Der Druck im Inneren des Reaktors liegt bei der Flüssigphasendehydratisierung vorzugsweise in einem Bereich von 1 bis 50 bar, besonders bevorzugt in einem Bereich von 2 bis 25 bar und am meisten bevorzugt in einem Bereich von 3 bis 10 bar.

Die Katalyse der Dehydratisierung kann sowohl bei der Gasphasendehydratisierung als auch bei der Flüssigphasendehydratisierung homogen oder heterogen erfolgen.

Bei der homogenen Katalyse wird der Katalysator, bei dem es sich dann vorzugsweise um eine anorganische Säure wie etwa Phosphorsäure oder Schwefelsäure handelt, zunächst mit der reinen 3-Hydroxyisobuttersäure oder mit der wässrigen Lösung beinhaltend die 3-Hydroxyisobuttersäure in Kontakt gebracht. Anschließend wird die so erhaltene Zusammensetzung in den Reaktor eingebracht und unter den gewünschten Druck-und Temperaturbedingungen in Methacrylsäure überführt. Denkbar ist auch, die anorganische Säure unabhängig von der 3-Hydroxyisobuttersäure bzw. der wässrigen Lösung in den Reaktor einzubringen. In diesem Fall weist der Reaktor mindestens zwei Zuleitungen, eine für die 3-Hydroxyisobuttersäure bzw. die wässrigen Lösung beinhaltend die 3-Hydroxyisobuttersäure und eine für den Katalysator, auf. Wird die Dehydratisierung in flüssiger Phase in einem Rieselbettreaktor durchgeführt, so ist es bevorzugt, dass der Katalysator zusammen mit der 3-Hydroxyisobuttersäure bzw. der wässrigen Lösung beinhaltend die 3-Hydroxyisobuttersäure im Kopfbereich des Reaktors eingebracht wird.

Bei der heterogenen Katalyse befindet sich der Katalysator in Form eines festen Substrates im Reaktionsraum, beispielsweise in Form einer Festbettschüttung, in Form von mit Katalysator beschichteten Platten, vorzugsweise Thermoblechen, welche im Inneren des Reaktors angeordnet sind oder aber in Form von mit Katalysator beschichteten Reaktorwänden. Mögliche Reaktoren sind beispielsweise in der DE-A-198 48 208, DE-A-100 19 381 und EP-A-I 234 612 beschrieben. Im Falle der heterogenen Katalyse sind als Katalysatoren mit anorganischen Säuren in Kontakt gebrachte, vorzugsweise imprägnierte porenförmiger Trägerkörper bevorzugt. Die 3-Hydroxyisobuttersäure bzw. die wässrige Lösung beinhaltend die 3-Hydroxyisobuttersäure wird dann in dampfförmiger oder flüssiger Form mit der Oberfläche des festen Katalysatormaterials in Kontakt gebracht.

Gemäß einer besonders bevorzugten Ausführungsform des erfindungsgemäßen Verfahrens wird die Dehydratisierung der 3-Hydroxyisobuttersäure in flüssiger Phase bei einem Druck in einem Bereich von 200 bis 500 mbar, bei einer Temperatur in einem Bereich von 200 bis 230 °C und in Gegenwart von Alkalimetall-Ionen als Katalysator durchgeführt.

Als Reaktionsgemisch, welches im Anschluss an die Dehydratisierung erhalten wird, wird entweder eine wässrige Methacrylsäure-Lösung, die keine Katalysatorbestandteile enthält (eine solche wird im Falle einer heterogen katalysierten Dehydratisierung erhalten) oder aber eine wässrige Methacrylsäure-Lösung, welche Katalysatoren beinhaltet (eine solche wird im Falle einer homogen katalysierten Dehydratisierung erhalten) erhalten. Weiterhin kann die wässrige Methacrylsäure-Lösung in flüssiger Form (sofern die Dehydratisierung in der Flüssigphase erfolgt ist) oder gasförmig (sofern die Dehydratisierung in der Gasphase erfolgt ist) vorliegen.

Die so erhaltene Methacrylsäure-Lösung kann gemäß einer besonderen Ausführungsform des erfindungsgemäßen Verfahrens ohne weitere Aufarbeitung gegebenenfalls der Veresterung zugeführt werden. Dabei wird die Methacrylsäure-Lösung mit entsprechenden Alkoholen, wie etwa Methanol, Ethanol, 1-Propanol, 2-Propanol oder 1-Butanol sowie geeigneten, dem Fachmann bekannten Veresterungkatalysatoren, wie etwa konzentrierten Säuren, unter Erhitzen in Kontakt gebracht und die Methacrylsäure auf diese Weise in die entsprechenden Ester überführt. Vorteilhaft kann es jedoch sein, die Methacrylsäure vor der Veresterung noch aufzureinigen, wobei zur Aufreinigung grundsätzlich jedes dem Fachmann bekanntes Aufreinigungsverfahren angewendet werden kann, welches herkömmlicherweise zur Aufreinigung verunreinigter, durch katalytische Gasphasenoxidation von Propylen erhaltener (Meth)Acrylsäure Anwendung findet.

Wurde die Dehydratisierung in der Gasphase durchgeführt, so ist es bevorzugt, dass die Methacrylsäure zunächst unter Erhalt einer wässrigen Methacrylsäure-Lösung kondensiert wird. Dabei kann grundsätzlich jedes dem Fachmann bekannte Kondensationsverfahren eingesetzt werden, beispielsweise eine fraktionierte Kondensation, wie sie in WO-A-2004/035514, WO-A-03/014172 oder EP-A-EP 1 163 201 beschrieben ist, oder durch eine Totalkondensation, wie sie in der EP-A-0 695 736 beschrieben ist. Denkbar ist auch, bei der Kondensation zusätzliches Lösungsmittel, insbesondere Wasser, zuzusetzen, um die Methacrylsäure möglichst vollständig zu absorbieren.

Die nach der Kondensation erhaltene, wässrige Methacrylsäure-Lösung oder aber die im Falle der Flüssigphasendehydratisierung erhaltene wässrige Methacrylsäure-Lösung kann dann in weiteren Aufreinigungsschritten von Wasser und anderen Verunreinigungen befreit werden. Dabei kann zunächst das Wasser in Gegenwart eines Schleppmittels durch Azeotropdestillation entfernt werden, wie dies beispielsweise in der DE-A-198 53 064 beschrieben ist. Denkbar ist auch der Einsatz hochsiedender organischer Lösungsmittel zur Absorption der Methacrylsäure, wie dies beispielsweise in der EP-A-0 974 574 offenbart ist. Neben diesen destillativen Verfahren können auch Membranen zur Entwässerung eingesetzt werden, wie dies etwa in DE-A-44 01 405 vorgeschlagen wird. Denkbar ist weiterhin eine Aufreinigung der im Falle der Flüssigphasendehydratisierung gewonnen oder durch Kondensation erhaltenen wässrigen Methacrylsäure-Lösung durch Kristallisationsverfahren.

Die nach dem Entwässern erhaltene Methacrylsäure kann in weiteren Verfahrensschritten noch weiter aufgereinigt werden. So können noch enthaltene, hochsiedende Verunreinigungen durch weitere Destillationsschritte entfernt werden. Besonders bevorzugt ist es jedoch, wenn die nach dem Entwässern erhaltene Methacrylsäure durch Kristallisationsverfahren weiter aufgereinigt wird, wie dies etwa in DE-A-101 49 353 beschrieben ist.

Die so erhaltene, aufgereinigte Methacrylsäure kann dann gegebenenfalls der Veresterung unterzogen werden.

Einen Beitrag zur Lösung der eingangs genannten Aufgaben leistet weiterhin ein Verfahren zur Herstellung von Methacrylsäure oder Methacrylsäureestern, umfassend die Verfahrensschritte
IIA) Herstellung von auf 3-Hydroxyisobuttersäure basierenden Polyhydroxyalkanoaten durch das vorstehend beschriebene Verfahren,
IIB) Spaltung der auf 3-Hydroxyisobuttersäure basierenden Polyhydroxyalkanoate unter Bildung von 3-Hydroxyisobuttersäure sowie gegebenenfalls Neutralisation der 3-Hydroxyisobuttersäure und/oder Aufreinigung der 3-Hydroxyisobuttersäure,
IIC) Dehydratisierung der 3-Hydroxyisobuttersäure unter Bildung von Methacrylsäure sowie gegebenenfalls Veresterung des Methacrylates bzw. der Methacrylsäure.

Einen Beitrag zur Lösung der eingangs genannten Aufgaben leistet auch ein Verfahren zur Herstellung von Polymethacrylsäure oder Polymethacrylsäureestern, umfassend die Verfahrensschritte
IIIA) Herstellung von Methacrylsäure durch das vorstehend beschriebene Verfahren,
IIIB) radikalische Polymerisation der Methacrylsäure,
wobei gegebenenfalls die Carboxylgruppen der Methacrylsäure vor oder nach der radikalischen Polymerisation zumindest teilweise verestert werden können.

Die vorliegende Erfindung wird nun anhand nicht limitierender Figuren und Beispiele näher erläutert.

Es zeigt die Figur 1 die durch das Enzym E₁ katalysierte Umsetzung von Succinyl-Coenzym A zu Methylmalonyl-Coenzym A.

Es zeigt die Figur 2 die durch die Enzyme E₂ bis E₄ katalysierte Umsetzung von Methylmalonyl-Coenzym A zu 3-Hydroxyisobuttrsäure gemäß der ersten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 3 die durch die Enzyme E₄, E₆ und E₇ katalysierte Umsetzung von (R)-Methylmalonyl-Coenzym A zu 3-Hydroxyisobuttrsäure gemäß der zweiten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 4 die durch die Enzyme E₄, E₅ und E₇ katalysierte Umsetzung von Methylmalonyl-Coenzym A zu 3-Hydroxyisobuttrsäure gemäß der dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 5 die durch die Enzyme E₈ und E₉ katalysierte Umsetzung von 3-Hydroxyisobuttrsäure zu einem Polyhydroxyalkanoat.

Es zeigt die Figur 6 die durch die Enzyme E₁₀ oder E₁₁ katalysierte Umsetzung von Phosphoenolpyruvat bzw. Pyruvat zu Oxalacetat gemäß einer besonderen Ausgestaltung der ersten, zweiten oder dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 7 die durch die Enzyme E₁₂ bis E₁₅ katalysierte Umsetzung von Oxalacetat zu Succinyl-Coenzym A gemäß einer ersten besonderen Ausgestaltung der ersten, zweiten oder dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 8 die durch die Enzyme E₁₃ bis E₁₆ und E₂₄ bis E₂₆ katalysierte Umsetzung von Oxalacetat zu Succinyl-Coenzym A gemäß einer zweiten besonderen Ausgestaltung der ersten, zweiten oder dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 9 die durch die Enzyme E₁₆, E₂₄, E₂₇ und E₂₈ katalysierte Umsetzung von Oxalacetat zu Succinyl-Coenzym A gemäß einer dritten besonderen Ausgestaltung der ersten, zweiten oder dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 10 die durch die Enzyme E₄₆ und E₂₈ katalysierte Umsetzung von L-Glutamat zu Succinyl-Coenzym A gemäß einer weiteren besonderen Ausgestaltung der ersten, zweiten oder dritten Alternative des erfindungsgemäßen Verfahrens, bei der eine gentechnisch veränderte Zelle eingesetzt wird, in der Succinyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigen die Figuren 11 und 12 die durch die Enzyme E₄, E₅ und E₄₇ bis E₅₂ katalysierte Umsetzung von Acetyl-Coenzym A zu 3-Hydroxyisobuttersäure gemäß einer ersten Alternative der zweiten besonderen Ausführungsform des erfindungsgemäßen Verfahrens, bei der eine gentechnisch veränderte Zelle eingesetzt wird, in der Propionyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigen die Figuren 13 und 14 die durch die Enzyme E₂ bis E₄, E₆, E₇ und E₄₇ bis E₅₂ katalysierte Umsetzung von Propionyl-Coenzym A zu 3-Hydroxyisobuttersäure gemäß einer zweiten Alternative der zweiten besonderen Ausführungsform des erfindungsgemäßen Verfahrens, bei der eine gentechnisch veränderte Zelle eingesetzt wird, in der Propionyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigen die Figuren 15 und 16 die durch die Enzyme E₂ bis E₄, E₇ und E₄₇ bis E₅₂ katalysierte Umsetzung von Propionyl-Coenzym A zu 3-Hydroxyisobuttersäure gemäß einer dritten Alternative der zweiten besonderen Ausführungsform des erfindungsgemäßen Verfahrens, bei der eine gentechnisch veränderte Zelle eingesetzt wird, in der Propionyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 17 die durch die Enzyme E₅₃ bis E₅₅, E₅₈ und E₅₂ bis E₆₅ katalysierte Umsetzung von zwei Einheiten Acetyl-Coenzym A zu Glyoxylat und Propionyl-Coenzym A gemäß einer fünften Alternative der zweiten besonderen Ausführungsform des erfindungsgemäßen Verfahrens, bei der eine gentechnisch veränderte Zelle eingesetzt wird, in der Propionyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 18 die durch die Enzyme E₂ bis E₄, E₆₂, E₇₂ und E₇₃ katalysierte Umsetzung von Beta-Alanin zu 3-Hydroxyisobuttersäure gemäß einer dritten besonderen Ausführungsform des erfindungsgemäßen Verfahrens, bei der eine gentechnisch veränderte Zelle eingesetzt wird, in der Acrylyl-Coenzym A als Zwischenprodukt und Methylmalonat-Semialdehyd als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 19 die durch die Enzyme E₇₆ bis E₇₉, E₆₀, E₆₁ und E₈ katalysierte Umsetzung von Pyruvat zu 3-Hydroxyisobuttersäure gemäß einer ersten Alternative der ersten besonderen Ausführungsform der zweiten Variante des erfindungsgemäßen Verfahrens, bei der eine gentechnisch veränderte Zelle eingesetzt wird, in der Isobutyryl-Coenzym A als Zwischenprodukt und 3-Hydroxyisobutyryl-Coenzym A als Vorprodukt bei der Herstellung von 3-Hydroxyisobuttersäure oder von auf 3-Hydroxyisobuttersäure basierenden Polyhydroxyalkanoaten gebildet werden.

Es zeigt die Figur 20 die durch die Enzyme E₈, E₆₀, E₆₁, E₇₉ und E₈₀ katalysierte Umsetzung von L-Valin zu 3-Hydroxyisobuttersäure gemäß einer zweiten Alternative der ersten besonderen Ausführungsform der zweiten Variante des erfindungsgemäßen Verfahrens, bei der eine gentechnisch veränderte Zelle eingesetzt wird, in der Isobutyryl-Coenzym A als Zwischenprodukt und 3-Hydroxyisobutyryl-Coenzym A als Vorprodukt bei der Herstellung von 3-Hydroxyisobuttersäure oder von auf 3-Hydroxyisobuttersäure basierenden Polyhydroxyalkanoaten gebildet werden.

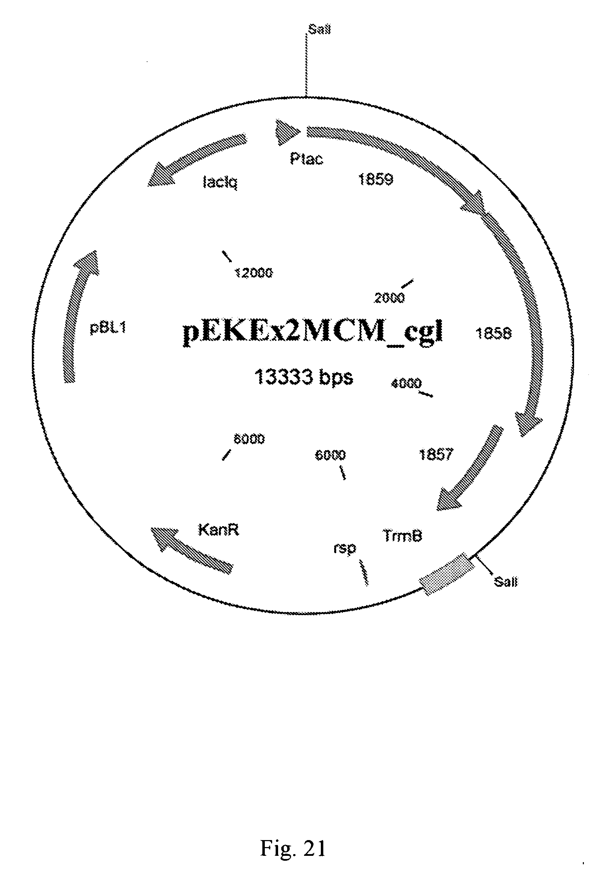

Es zeigt die Figur 21 das Plasmid pEKEx2MCM cg1 aus Beispiel 3.

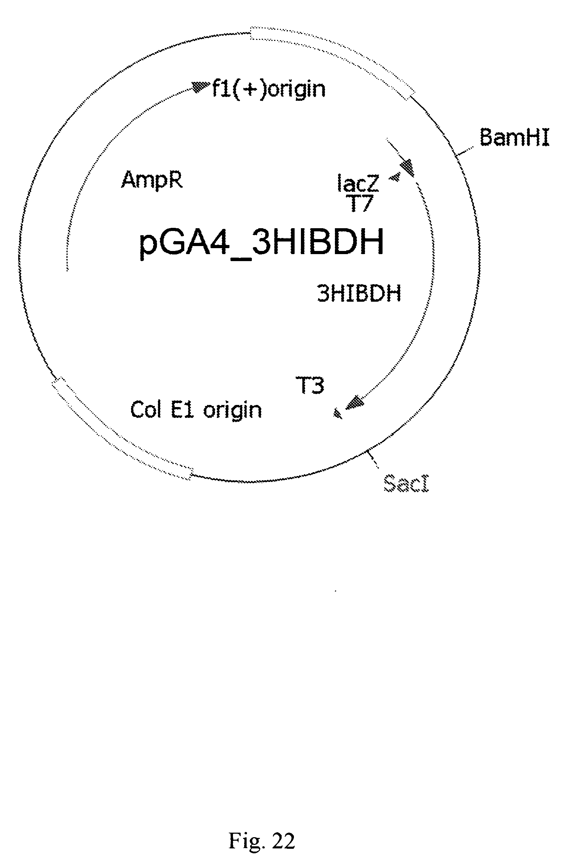

Es zeigt die Figur 22 den Vektor pGA4_3HIBDH aus Beispiel 3.

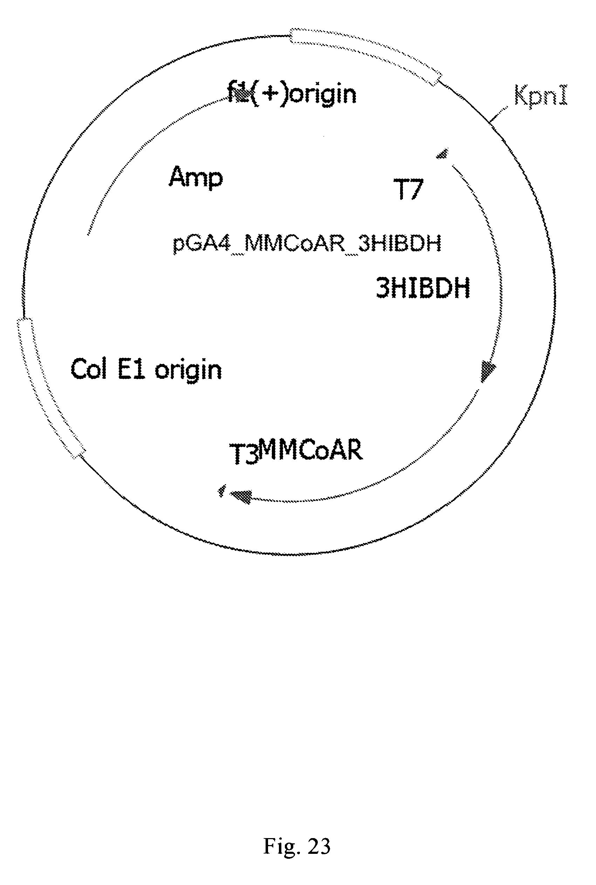

Es zeigt die Figur 23 den Vektor pGA5_MMCoAR_3HIBDH aus Beispiel 3.

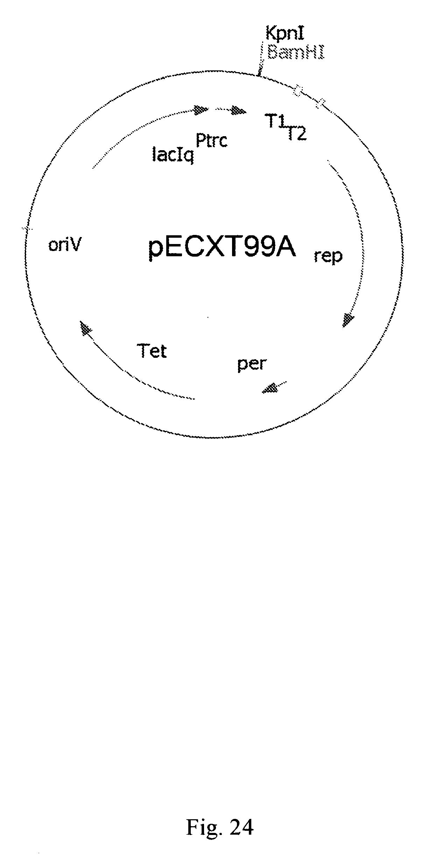

Es zeigt die Figur 24 den Vektor pECXT99A aus Beispiel 3.

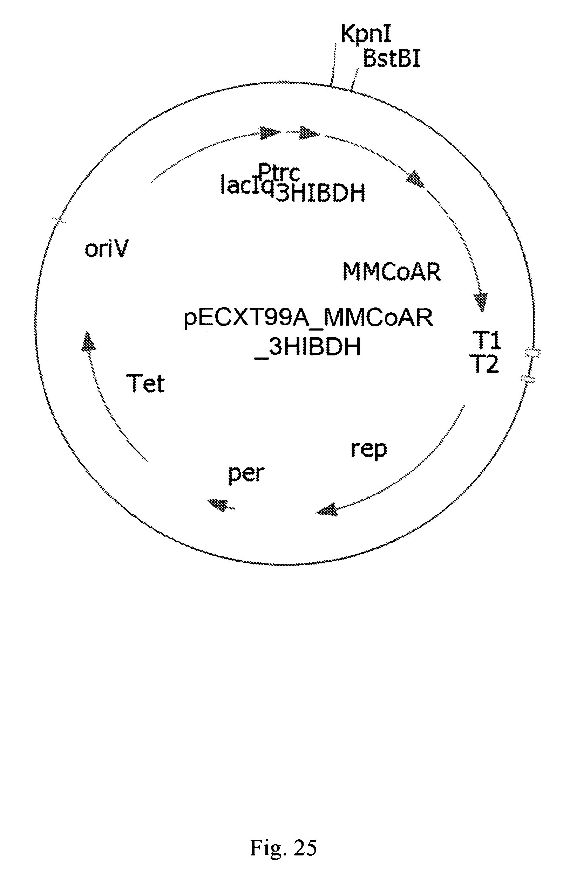

Es zeigt die Figur 25 den Vektor pECXT99A_MMCoAR_3HIBDH aus Beispiel 3.

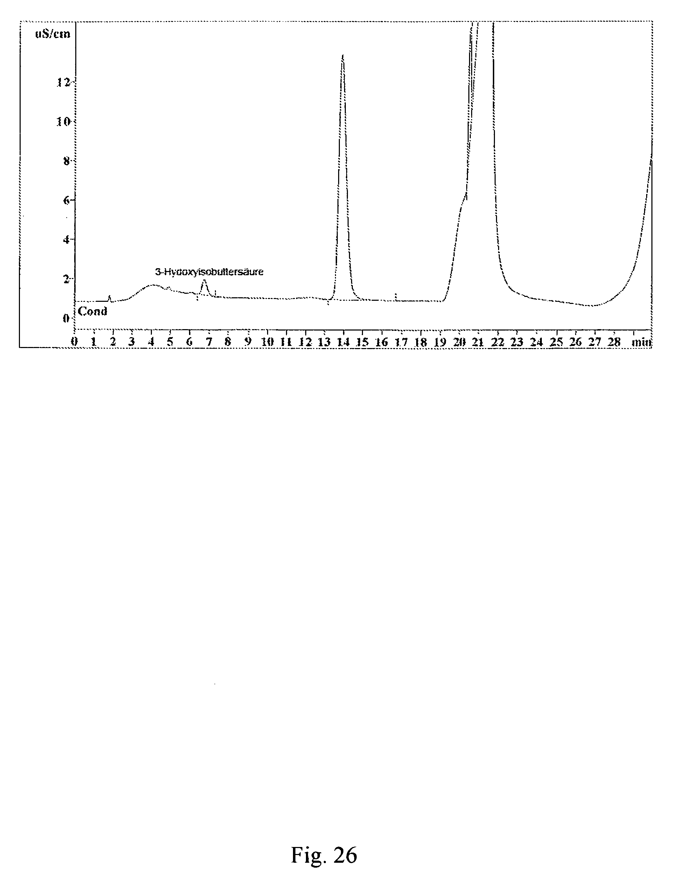

Es zeigt die Figur 26 den ionenchromatographischen Nachweis einer Bildung von 3-Hydroxyisobuttersäure durch die rekombinanten Zellen im Beispiel 3.

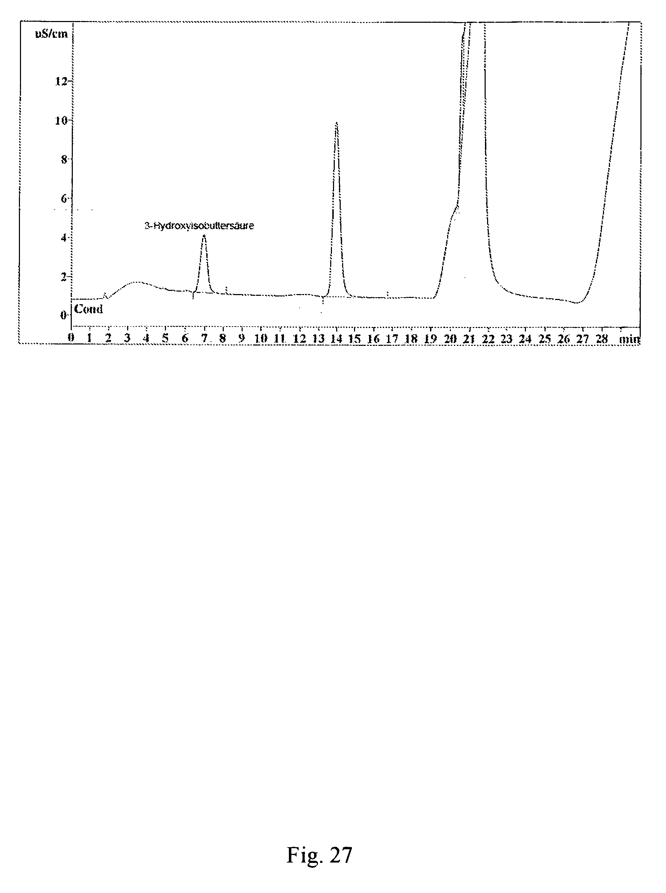

Es zeigt die Figur 27 den ionenchromatographischen Nachweis, dass es sich bei dem in Figur 26 gekennzeichneten Peak um den für 3-Hydroxyisobuttersäure handelt.

### Beispiele

### Beispiel 1

Die vorliegende Erfindung wird nun im Beispiel 1 anhand einer rekombinanten Zelle veranschaulicht, die 3-Hydroxyisobuttersäure über 3-Hydroxyisobutyryl-Coenzym A als Vorprodukt und Isobutyryl-Coenzym A als Zwischenprodukt ausgehend von L-Valin als Kohlenstoffquelle herzustellen vermag. Diese Zelle kann erfindungsgemäß zur Herstellung von Methacrylsäure eingesetzt werden. Dazu wurden in *E. co*li BL21 (DE3) die Enzyme EC 2.6.1.42 und EC 1.2.4.4 (jeweils aus *Pseudomonas aeruginosa*) sowie ein Cluster umfassend die drei Enzyme EC 1.3.99.12, EC 4.2.1.17 und EC 3.1.2.4 (aus *Acinetobacter calcoaceticus*) überexprimiert.

Das Enzym EC 1.2.4.4 wird dabei von einem Gen mit der DNA-Sequenz gemäß SEQ.-ID-Nr. 07 und 08 (α- und β-Untereinheit) kodiert, während das Enzym EC 2.6.1.42 von einem Gen mit der DNA-Sequenz gemäß SEQ.-ID-Nr. 09 kodiert wird. Das Enzym EC 1.3.99.12 wird von einem Gen mit der DNA-Sequenz mit der SEQ.-ID-Nr. 10 kodiert, das Enzym EC 4.2.1.17 von einem Gen mit der DNA-Sequenz gemäß SEQ.-ID-Nr. 11 und das Enzym EC 3.1.2.4 von einem Gen mit der DNA-Sequenz gemäß SEQ.-ID-Nr. 12.

### 1. Organismen, Plasmide und Oligonucleotide

Zur Herstellung dieser rekombinanten Zelle wurden folgende Bakterienstämme, Vektoren, genomische DNA und Oligonucleotide verwendet:

**Tab. 1: Verwendete Bakterienstämme**

| Stamm | Referenz (Her-steller) |
|---|---|
| *E. coli* DH5 | NEB |
| *E. coli* BL21 (DE3) | Invitrogen |

**Tab. 2: Verwendete Vektoren**

| Vektor | Referenz (Her-steller) |
|---|---|
| pCDFDuet-1 | Novagen |
| pET101/D-TOPO | Invitrogen |
| pCR2.1-TOPO | Invitrogen |

**Tab. 3: Verwendete genomische DNA**

| Stamm |
|---|
| *Pseudomonas aeruginosa* PAO1 |
| *Acinetobacter calcoaceticus* |
| ADP1 |

**Tab. 4: Verwendete Oligonucleotide**

| Name | Sequenz |
|---|---|
| A-ca_VClus_fw | 5'-ATGCAATTTAATGAAGAACAGCTATTAATTC-3' (SEQ.-ID-Nr. 13) |
| A-ca_VClus_rev | 5'-CAGTCTGAAATGACTAACCTAATTGGC-3' (SEQ.-ID-Nr. 14) |
| Pae_26142_fw | 5'-ACGGAATTCTGAAGGAGCTGGCAACTATG-3' (SEQ.-ID-Nr. 15) |
| Pae_26142_rev | 5'-TTGTCGACTTACTTGACCAGGGTACGCC-3' (SEQ.-ID-Nr. 16) |
| Pae_1244_fw | 5'-ACAGATCTGGAGGCCTGTCATGAGTGATTAC-3' (SEQ.-ID-Nr. 17) |
| Pae_1244_rev | 5'-ATGGGTACCCATTCAGACCTCCATC-3' (SEQ.-ID-Nr. 18) |

### 2. Amplifikation der PCR-Fragmente 1.2.4.4 (2.313 kb) und 2.6.1.42 (958 bp)

Zunächst wurden die Fragmente der 1.2.4.4 und 2.6.1.42 mittels der in der Tabelle 4 angegebenen Primern gemäß SEQ.-ID-Nr. 15 bis SEQ.-ID-Nr. 18 ausgehend von der Gesamt-DNA aus *Pseudomonas aeroginosa* mittels PCR amplifiziert.

### 3. Verdau des Vektors pCDF-Duet-1 und des PCR-Fragmentes 2.6.1.42 (958 bp)

Der Vektor pCDFDuet-1 (aufweisend eine Streptomycin/Spectinomycin-Resistenz) wird, ebenso wie das PCR-Fragment 2.6.1.42, mittels *EcoR*I/*Sal*I geschnitten und die so erhaltenen Restriktionen mit T4-Ligase über Nacht ligiert. Es wird der Vektor pCDFDuet::2.6.1.42 erhalten.

### 4. Klonierung der PCR-Fragmente in den Vektor pCR2.1-TOPO

Die Herstellung eines Klonierungsvektors umfassend das Fragment 2.6.1.42 bzw. das Fragment 1.2.4.4 unter Einsatz der Vektors pCR2.1-TOPO erfolgte gemäß den Angaben des Herstellers. Mit den auf diese Weise erhaltenen Klonierungsvektoren pCR2.1-TOPO::1.2.4.4 und pCR2.1-TOPO::2.6.1.42 wurden *E*. *coli* DH5α-Zellen transformiert. Da die Vektoren pCR2.1-TOPO eine Kanamycin- und eine Ampicillin-Resistenz aufweisen, wurde auf 2 AXI-und KXI-Platten ausplattiert (20 und 40 µl). Die Plasmide der erhaltenen Klone wurden isoliert und verdaut:

| | |
|---|---|
| pCR2.1-TOPO::1.2.4.4 | *Bg*III + *Kpn*I Fragmentgröße 2313 bp |
| pCR2.1-TOPO::2.6.1.42 | *EcoR*I + *Sal*I Fragmentgröße 958 bp |

Die Fragmente wurden jeweils aus dem Gel eluiert und mit dem Qiagen-Kit QIAquick (nach Anleitung) aufgereinigt.

### 5. Herstellung des Vektors pCDFDuet:2.6.1.42-1.2.4.4

Der Vektor pCDFDuet::2.6.1.42 sowie der Vektor pCR2.1-TOPO::1.2.4.4 werden mit *Bg*III/*Kpn*I verdaut. Anschließend erfolgt die Ligation von pCDFDuet::2.6.1.42 (*Bg*III/*Kpn*I) mit pCR2.1-TOPO::1.2.4.4 unter Erhalt des Vektors pCDFDuet::2.6.1.42-1.2.4.4. Mittels dieses Klonierungsvektors wurden wiederum *E*. *coli* DH5α-Zellen transformiert. Die Plasmide wurden isoliert. Das Plasmid pCDFDuet::2.6.1.42-1.2.4.4 weist die DNA-Sequenz gemäß SEQ.-ID-Nr. 19 auf.

### 6. Klonierung des Valin-Clusters aus Acinetobacter calcoaceticus (V-Clus_{Aca})

Der Stamm ATCC 33304 *Acinetobacter calcoaceticus* wurde für eine Isolierung von Gesamt-DNA kultiviert (HH Agar bzw. Medium). Die Isolierung von Gesamt-DNA erfolgte mit dem Kit DNEasy von Qiagen (L1 und L2) und durch ein Verfahren umfassend die Verfahrensschritte i) Zentrifugation von 1 mL Kultur, ii) Zugabe von 200 µL H₂O zum Pellet, iii) Erhitzen für 10 min auf 95 °C, iv) Zentrifugation (10 min, 13.000 rpm) sowie v) Abnehmen des Überstandes für eine PCR.

Zur Amplifikation des Valin-Clusters aus *A. calcoaceticus* wurde eine PCR unter Verwendung der in der Tabelle 4 angegebenen Primer gemäß SEQ.-ID-Nr. 13 und SEQ.-ID-Nr. 14 durchgeführt (nach Anleitung des Herstellers mit den Polymerasen *Pfu* bzw. *Taq).*

Die PCR-Produkte wurden aufgereinigt und nach Anleitung zum Plasmid pET101/D-TOPO ligiert sowie in E. coli DH5α gebracht. Es wird das Plasmid pET101/D-TOPO::V-Cluster_{Aca} erhalten. Das Plasmid pET101/D-TOPO::V-Cluster_{Aca} weist die DNA-Sequenz gemäß SEQ.-ID-Nr. 20 auf.

### 7. Herstellung einer rekombinanten Zelle, die 3-Hydroxyisobuttersäure aus L-Valin zu bilden vermag

*E. coli* BL21 (DE3) wurde mit den Plasmiden pET101/D-TOPO::V-Cluster*_{Aca}* und pCDF-Duet::2.6.1.42-1.2.4.4 transformiert (ausplattiert auf LB-Spec./Amp-Medium). Die so erhaltenen Zellen waren in der Lage, in einem Nährmedium beinhaltend L-Valin das L-Valin zu 3-Hydroxyisobuttersäure umzusetzen. Der Wildtyp der Zellen (*E. coli* BL21 (DE3)) konnte hingegen in einem derartigen Nährmedium keine nachweisbaren Mengen an 3-Hydroxyisobuttersäure bilden.

### Beispiel 2

In diesem Beispiel wird eine für ein Gen kodierende DNA isoliert und das Gen in *E*. *coli* überexprimiert. Die DNA kodiert für ein Enzym, welches sowohl die Aktivität des Enzyms E₂ als auch die des Enzyms E₃ aufweist.

### 1. Züchtung und Ernte von Sulfolobus tokodaii

*Sulfolobus tokodaii* wurde in kleinen Kulturvolumen (40-200 ml) bei 75°C und einem pH-Wert von 3,0 unter Schütteln (150 rpm) angezogen. Das Wachstum wurde photometrisch über die Messung der optischen Dichte bei 578 nm (OD_{578 nm}) verfolgt. Es wurde ein modifiziertes *Sulfolo*bus-Medium verwendet (modifiziert nach Brock et al., Archives of Microbiology 84, Seiten 54 - 68, 1972; Suzuki et al., Extremophiles, 6, Seiten 39 - 44, 2002). Als Energie- und Kohlenstoffquelle dienten Hefeextrakt, Casaminosäuren und Glucose. Das Medium bestand aus folgenden Komponenten: Grundmedium, Glucosestammlösung, Eisenstammlösung und Spurenelementestammlösung. Bei einer OD₅₇₈ₙₘ von 0,3-0,5 (exponentielle Phase) wurden die Zellen geerntet. Die Zentrifugation erfolgte in einer Sorvall-Zentrifuge (SS34-Rotor) für 15 min bei 9.000 rpm. Der Zellniederschlag wurde direkt für die DNA-Extraktion eingesetzt.
*Grundmedium.* KH₂PO₄ (0,28 g/l), (NH₄)₂SO₄ (1,3 g/l), MgSO₄ × 7 H₂O (0,25 g/l), CaCl₂ × 6 H₂O (0,07 g/l), Hefeextrakt (1 g/l) und Casaminosäuren (1 g/l). Der pH-Wert wurde mit H₂SO₄ vor dem Autoklavieren auf 3,0 eingestellt.
*Glucosestammlösung (100fach).* Glucose (100 g/l). Die Lösung wurde sterilfiltriert.
*Eisenstammlösung (1000fach).* FeCl₃ × 6 H₂O (20 g/l). Die Lösung wurde sterilfiltriert.
*Spurenelementestammlösung (1000fach).* MnCl₂ × 4 H₂O (1,8 g/l), Na₂B₄O₇ × 10 H₂O (4, 5 g/l), ZnSO₄ × 7 H₂O (220 mg/l), CuCl₂ × 2 H₂O (50 mg/l), Na₂MoO₄ × 2 H₂O (30 mg/l), VOSO₄ × 5 H₂O (30 mg/l), CoCl₂ × 6 H₂O (8,4 mg/l). Die einzelnen Komponenten wurden nacheinander in H₂O dest gelöst, der pH-Wert mit HCl auf 3,0 eingestellt und die Lösung sterilfiltriert.

### 2. Isolierung von genomischer DNA aus S. tokodaii

Die Isolierung von genomischer DNA wurde nach der Methode von Murray und Thompson (Nucleic Acid Research, 8, Seiten 4.321-4.325, 1980) durchgeführt. Hierzu wurden 10 - 50 mg (Feuchtgewicht) frisch geerntete Zellen in ein 1,5 ml Eppendorf-Reaktionsgefäß eingewogen und in 570 ml TE-Puffer (10 mM Tris/HCl (pH 8,0), 1 mM NaEDTA) resuspendiert. Es wurden 30 µl einer 10% (w/v) SDS-Lösung (Natriumdodecylsulfat-Lösung) und 3 µl Proteinase K (20 µg/µl) zugesetzt und 1 h bei 52°C inkubiert. Danach wurden 100 µl 5 M NaCl-Lösung und 80 µl vorgewärmte 10% (w/v) Cetyltrimethyl-ammoniumbromid (CTAB)-Lösung (10% (w/v) CTAB in 0,7 M NaCl) zugegeben. Nach 10 min Inkubation bei 65°C wurden die Komplexe aus CTAB, Zellwandfragmenten und Proteinen mit 780 µl Chloroform/iso-Amylalkohol (24 : 1 (v/v)) extrahiert und 15 min bei 14.000 rpm abzentrifugiert. Die obere wässrige Phase wurde in ein neues Eppendorf-Reaktionsgefäß überführt und die Extraktion wiederholt. Nachdem die wässrige Phase frei war von Pigmenten, wurde sie mit 400 µl 100 % Isopropanol überschichtet. Durch vorsichtiges Mischen beider Phasen präzipitierte die chromosomale DNA an der Grenzschicht. Die DNA konnte mit einer ausgezogenen Pasteurpipette geangelt und in 200 µl 70 % Ethanol gewaschen werden. Nach erneuter Zentrifugation (5 min, 14.000 rpm) wurde der Überstand abgezogen, die DNA 2 h bei Raumtemperatur getrocknet und schließlich in 100 µl TE-Puffer gelöst.

### 3. Amplifizierung des Gens für die Malonyl-Coenzym A-Reduktase

Die Polymerase-Ketten-Reaktion (PCR) (Mullis et al., Cold Spring Harbor Symp. Quant. Biol., 51, Seiten 263-273, 1986) wurde eingesetzt, um ausgehend von der im Beispiel 2 erhaltenen genomischen DNA von *Sulfolobus tokodaii* das Gen für die Malonyl-CoA-Reduktase gezielt zu amplifizieren. Sie wurde in einem Thermocycler (Biometra, Göttingen) durchgeführt.

Zur Anwendung gelangte eine präparative PCR, bei der Pfu-Polymerase (Pfunds, Genaxxon) verwendet wurde. Die Pfu-Polymerase enthält eine 3'-5' Exonuklease ("proofreading") Funktion.

Folgende Primer wurden verwendet:
5'-ATTATCCCATGGGGAGAACATTAAAAGC-3' ("forward primer"; NcoI-Schnittstelle ist unterstrichen; Seq.-ID-Nr. 21) und
5'-CGGGATCCTTACTTTTCAATATATCC-3' ("reverse primer"; *Bam*HI-Schnittstelle ist unterstrichen; Seq.-ID-Nr. 22)

Für die PCR-Reaktionen wurde der in der nachfolgenden Tabelle 1 angegebene Reaktionsansatz verwendet. Die PCR wurde als Hot-Start-PCR durchgeführt, d.h. der Reaktionsansatz wurde vor der Zugabe der Pfu-Polymerase 2 min bei 95 °C inkubiert. Anschließend folgten 30 Zyklen mit jeweils 1 Minute bei 95 °C, 1 Minute bei 45 °C und 5 Minuten bei 72 °C, gefolgt von einem letzten Schritt mit 30 Sekunden bei 45 °C, 15 Minuten bei 72 °C und schließlich einer Pause bei 6 °C.

**Tabelle 1: Standardreaktionsansatz (50 µl) für Proofreading-PCR mit Pfu-Polymerase**

| **Zusammensetzung** | **µl/50 µl-Ansatz** |
|---|---|
| 10 × Pfu-PCR-Reaktionspuffer | 5 |
| dNTP-Mix (2 mM je Nukleotid) | 5 |
| Primer forward (2 µM) | 12,5 |
| Primer reverse (2 µM) | 12,5 |
| chromosomale DNA | 1 (10-50 ng) |
| Pfu-Polymerase (2, 5 U/µl) | 2 |
| H₂O_{bidest} | 12 |

Es wurde ein Gen-Fragment mit einer Länge von 1,1 kb erhalten.

### 4. Klonierung des Gens für die Malonyl-Coenzym A-Reduktase

Zur Klonierung des Malonyl-Coenzym A-Reduktase-Gens aus *Sulfolobus tokodaii* wurde das im Beispiel 3 amplifizierte Gen unspezifisch mit dem "pCR T7 Topo TA Expression Kit" (Invitrogen, Karlsruhe) in den Vektor pCR T7/CT-Topo (Invitrogen, Karlsruhe) kloniert. Die Durchführung erfolgte laut den Angaben des Herstellers.

Zur Isolierung der Plasmid-DNA wurde aus 5 ml Übernacht-Kulturen von transformierten *E*. *coli* TOP10F'-Zellen die Plasmid-DNA mit dem "QIAprep Spin Plasmid Miniprep Kit" von Qiagen (Hilden) nach Anleitung des Herstellers präpariert.

### 5. Herstellung eines Expressionsvektors

Zur Herstellung eines Expressionsvektors umfassend das Malonyl-Coenzym A-Reduktase-Gen wird der im Beispiel 4 erhaltene, isolierte Klonierungsvektor einem Restriktionsverdau mit den Restriktionsenzymen *Nco*I und *Bam*HI unterzogen. Dazu werden 25-27 µl Plasmid-DNA (Expressionsvektor pTrc99A bzw. pCR T7/CT-Topo Vektor mit dem eingebauten Gen der Malonyl-Coenzym A-Reduktase) mit 5 µl 10fach konzentriertem Reaktionspuffer und 2-3 µl Restriktionsenzym (10 U/µl; Fermentas, St. Leon-Rot) gut gemischt. Der Reaktionsansatz wurde mit H₂O dest auf 50 µl aufgefüllt und bei der vom Hersteller angegebenen Temperatur für 5 h inkubiert. Vor der weiteren Verwendung wurde eine Ethanolfällung durchgeführt. Dazu wurde die DNA mit 3 Volumeneinheiten 100% Ethanol und 0,1 Volumeneinheiten 3 M Natrium-Acetat-Puffer (pH 5,3) gemischt und 2 h oder über Nacht bei -80°C inkubiert. Nach einem Zentrifugationsschritt (20 min, 14.000 rpm, 4°C, Eppendorf Tischzentrifuge) wurde der Überstand vorsichtig abgenommen und die DNA mit 3 Volumeneinheiten 70% (v/v) Ethanol gewaschen. Nach 10 min Inkubation bei Raumtemperatur wurde erneut zentrifugiert (10 min, 14.000 rpm, 4 °C, Eppendorf Tischzentrifuge) und der Überstand verworfen. Die DNA wurde für 1 Stunde bei Raumtemperatur getrocknet und anschließend im gewünschten Volumen H₂O oder TE-Puffer (10 mM Tris/HCl (pH 8,0), 1 mM NaEDTA) aufgenommen.

Anschließend wird alkalische Phosphatase verwendet, um die 5'-Phosphatgruppen des linearisierten doppelsträngigen Vektors zu entfernen. Auf diese Weise wird die Klonierungseffizienz gesteigert, da eine Religation des Vektors verhindert wird. Es wurde alkalische Phosphatase aus Kälberdarm zur Dephosphorylierung des verdauten Vektors verwendet.

Die Dephosphorylierung wurde im gleichen Puffer wie der Restriktionsverdau durchgeführt. 50 µl Restriktionsansatz wurden mit 1,5 µl CIAP (Calf Intestine Alkaline Phosphatase) (1U/µl; Fermentas, St. Leon-Rot) gemischt und 30 min bei 37 °C inkubiert. Vor der weiteren Verwendung des geschnittenen und dephosphorylierten Vektors wurde eine Ethanolfällung, wie vorstehend beschrieben, durchgeführt.

Die Ligation der Insert-DNA mit dem Expressionsvektor wird die T4 DNA-Ligase verwendet, wobei Plasmid-DNA und Insert-DNA in einem molaren Verhältnis von 1:3 - 1:6 eingesetzt wurden.

### Stammlösungen:

| | |
|---|---|
| *Ligationspuffer* (10fach) : | 0, 5 M Tris/HCl, pH 7, 6 100 mM MgCl₂ 0,5 mg/ml BSA sterilfiltrieren, Lagerung bei Raumtemperatur. |
| *5 mM ATP* (Adenosintriphosphat) | immer frisch ansetzten in sterilem H₂O dest |
| *50 mM DTE* (Dithioerythritol) | immer frisch ansetzten in Ligationspuffer |

Die Ligationsansätze hatten ein Volumen von 50 µl. Plasmid-DNA (2 - 10 µl), Insert-DNA (2 - 20 µl), 5 µl Ligationspuffer mit DTE (50 mM) und die entsprechende Menge steriles H₂O_{dest} wurden zusammenpipettiert, gevortext, kurz abzentrifugiert und anschließend 5 min bei 45 °C inkubiert. Der Ansatz wurde auf Eis abgekühlt. Es wurden 5 µl 5 mM ATP und 1,5 µl T4 DNA-Ligase (1 U/µl; Fermentas; St. Leon-Rot) hinzugegeben und der Ansatz gemischt. Die Ligation erfolgte über Nacht bei 16 °C.

Der Ligationsansatz wurde direkt zur Transformation chemisch-kompetenter Zellen eingesetzt.

### 6. Transformation von E. Coli-Zellen mit dem Expressionsvektor

Ausgehend von einer Einzelkolonie von *E*. *coli* Rosetta 2-Zellen wurde eine 5 ml Übernachtkultur angezogen. Mit 0,5 - 1,0 ml dieser Kultur wurden am nächsten Morgen 50 ml LB-Medium (Sambrook et al., "Molecular Cloning: A Laboratory Manual", Cold Spring Harbor Laboratory Press, Cold Spring Harbor, New York, 1989) beimpft. Nach 1,5 - 2 h Inkubation (37 °C, Schütteln (180 UpM)) wurde eine OD₅₇₈ₙₘ von 0,6 erreicht. Die Zellen wurden 10 min auf Eis abgekühlt und anschließend 5 min bei 5000 UpM und 4 °C (GSA-Rotor, Sorvall-Zentrifuge) abzentrifugiert. Der Überstand wurde verworfen und der Zellniederschlag in 2,7 ml kalter 0,1 M CaCl₂-Lösung resuspendiert. Nach Zugabe von 2,3 ml sterilem 50 % (v/v) Glycerin wurde die Zellsuspension in Portionen (je 300 µl) in 1,5 ml Eppendorf-Reaktionsgefäße verteilt. Die kompetenten Zellen wurden sofort in flüssigem Stickstoff eingefroren und anschließend bei -80 °C gelagert.

Zur Transformation der Zellen wurde ein Aliquot der chemisch-kompetenten Zellen (300 µl) auf Eis aufgetaut und mit 25 µl eines Ligationsansatzes versetzt. Der Ansatz wurde vorsichtig gemischt und für 30 min auf Eis inkubiert. Nach einem Hitzeschock (42 °C, 1 min) wurde nochmals 5 min auf Eis inkubiert. Anschließend wurden 800 µl LB-Medium (Sambrook et al., 1989) zugegeben und die Zellen 1 h bei 37 °C geschüttelt (Thermomixer, Eppendorf 5436). Vor dem Ausstreichen des Ansatzes auf LB-Medium wurde der Ansatz noch aufkonzentriert. Dazu wurde 1 min bei 10.000 rpm abzentrifugiert, vom Überstand 750 µl verworfen und der Zellniederschlag resuspendiert. Von diesem konzentrierten Ansatz wurden 50 µl, 100 µl und 200 µl auf LB-Platten (Sambrook et al., 1989) mit 100 µg/ml Ampilcillin ausgestrichen und über Nacht bei 37 °C im Brutschrank inkubiert. Die Platten wurden mit 1 ml LB-Medium abgespült. Mit dieser Zellsuspension wurden anschließend 150 ml LB-Medium (mit 100 µg/ml Ampilcillin) in 500 ml Erlenmeyer-Schikanekolben beimpft. Die Kulturen wuchsen bei 37 °C und 180 UpM. Die Überexpression erfolgte durch Induktion des Promotors in pTrc99A durch Zugabe von 0,5 M IPTG (Isopropyl-β-D-thiogalactopyranosid) bei einer OD₅₇₈ₙₘ von 0,6. Die induzierten Kulturen wurden 3 h unter den genannten Bedingungen inkubiert und anschließend bei einer OD₅₇₈ₙₘ = 2,7 geerntet.

### 7. Nachweis der Enzymaktivität

Der im Beispiel 6 erhaltene E. coli-Stamm wurde mittels Zellmühle aufgeschlossen. Der Zellaufschluss wurde 15 min lang auf 85 °C erhitzt. Bei dieser Hitzefällung koagulieren nicht-hitzestabile Enzyme und werden gefällt. Da das Zielprotein hitze-stabil ist, bleibt es im Überstand enthalten. Zur Messung der Malonyl-Coenzym A-Reduktaseaktivität wurde der Überstand im Verhältnis 1 : 50 in TM-Puffer (50 mM Tris/Cl, 1 mM MgCl₂, pH 8,1) verdünnt. Zu 500 µl HIPS-Puffer (100 mM HEPES/NaOH, 5 mM MgCl₂, 1 mM Dithioerythritol, der 0.5 mM NADPH enthielt, wurden 30 µl des verdünnten bzw. unverdünnten (für Nachweise der Methylmalonyl-Coenzym A-Reduktaseaktivität) Überstands pipetiert. In einem ersten Ansatz wurde die Reaktion durch Zugabe von Malonyl-Coenzym A gestartet, wobei die Endkonzentration 0.5 mM betrug. Die Abnahme der NADPH-Absorption bei 365 nm wurde ermittelt. Die ermittelte Enzymaktivität betrug 15,5 µmol/min/mg Protein (15,5 U/mg).

In einem zweiten Ansatz wurde die Reaktion durch Zugabe von Methylmalonyl-Coenzym A gestartet (Firma Fluka, Art.-Nr.: 67767), wobei die Endkonzentration 2.0 mM betrug. Die Abnahme der NADPH-Absorption wurde ermittelt. Die ermittelte Enzymaktivität betrug 0,24 µmol/min/mg Protein (0,24 U/mg).
Diesen Ergebnissen ist zu entnehmen, dass das Polypeptid, für welches die DNA-Sequenz mit der SEQ.-ID-Nr. 03 kodiert, sowohl die Umsetzung von Malonyl-CoA als auch Methylmalonyl-Coenzym A katalysiert.
Pro eingesetztes Mol Malonyl-CoA bzw. Methylmalonyl-CoA wurde 1 Mol NADPH oxidiert. Daraus lässt sich schließen, dass die Enzymreaktion zum entsprechenden Semialdehyd führt.

### Beispiel 3

Die vorliegende Erfindung wird weiterhin im Beispiel 3 anhand der Herstellung von Methacrylsäure durch eine rekombinanten Zelle veranschaulicht, die 3-Hydroxyisobuttersäure über Methylmalonat-Semialdehyd als Vorprodukt ausgehend von Glucose als Kohlenstoffquelle herzustellen vermag. Dazu wurden in *C*. *glutamicum* ATCC13032 unter anderem die Enzyme Methylmalonyl-Coenzym A-Mutase (E₁) und 3-Hydroxyisobutyrat-Dehydrogenase (E₄) überexprimiert.

### 1. Klonierung der Gene NCgl1470, NCgl1471 und NCgl1472 (Arginin/Ornithin-Transportsystem-ATPase, Methylmalonyl-Coenzym A-Mutase und Methylmalonyl-Coenzym A-Mutase, N-terminale Domain/Untereinheit) in pEKEx2, Konstruktion von pEKEx2MCM cg1

Für die DNA-Manipulationen wurden Standardtechniken benutzt wie sie in Sambrook, J. et al. (1989), "Molecular Cloning: a laboratory manual", 2nd Ed., Cold Spring Harbor Laboratory Press, Cold Spring Harbor, New York, angegeben sind. DNA-Amplifikationen wurden mit der SAWADY Pwo-DNA Polymerase (Peqlab Biotechnologie, Erlangen, Germany) oder Platinum Pfx-DNA-Polymerase (Invitrogen, Karlsruhe, Germany) durchgeführt. Wenn nicht anderes angegeben wurde, erfolgte die Benutzung der Polymerasen entsprechend den Angaben der Hersteller. Oligonukleotide für die PCR-Amplifikationen und die Einführung von Restriktionsschnittstellen wurden von MWG-Biotech (Ebersberg, Germany) erhalten. Der Nachweis konstruierter Stämme erfolgte durch Kolonie-PCR mit der Taq *Polymerase READYMIX* (Sigma, Taufkirchen, Germany), sowie Plasmidpräparationen. DNA Fragmente wurden mit dem MinElute Gel Extraction Kit (Quiagen, Hilden, Germany) nach Angaben des Herstellers gereinigt und gewonnen. Plasmid DNA wurde mittels des Qiaprep spin Miniprep Kits (Quiagen, Hilden, Germany) isoliert. Alle konstruierten Plasmide wurden durch Restriktionsanalyse mit anschließender Sequenzierung verifiziert.

Zur Konstruktion von pEKEx2MCM_cgl wurde der Vektor pEKEx2 eingesetzt (Kleinertz et al., 1991 Gene 102:93), welcher die Transkiption klonierter Gene unter Kontrolle des Isopropyl-β-D-thiogalactopyranosid (IPTG) induzierbaren tac Promoters und des lac Repressorsystems (lacIq) erlaubt. Das 5,2 kb große DNA-Fragment, das für die Gene NCgl1470 NCgl1471 und NCgl1472 kodiert, wurde mittels der folgenden Oligonukleotide und DNA von *Corynebacterium glutamicum* ATCC13032 als Template amplifiziert:
pcg1859-57_vorwärts (SEQ.-ID-Nr. 23):
   5'-cggtcgacaaggagatatagataTGACTGATCTCACAAAGACTGC-3,
   und
pcg1857-59_rückwärts (SEQ.-ID-Nr. 24):
   5'-cTTAGGCTTTGTCGAACGCCTCC-3'.
(In Großbuchstaben sind zur Genomsequenz komplementäre Sequenzen angegeben).

Es wurden in die Amplifikate zusätzlich Restriktionsschnittstellen (unterstrichen) und eine Ribosomenbindestelle (aaggag) 8 Nukleotide vor dem Startcodon eingeführt. Dabei wurde das ursprüngliche Startcodon TTG gegen ATG ausgetauscht). Das PCR-Amplifikat wurde mit Polynukleotidkinase (Roche, Basel, Switzerland) phosphoryliert und blunt end in die *Sma*I-Schnittstelle des Vektors pUC19 kloniert (Yanisch-Perron et al., 1985, Gene 33:103-19). Die Identität und Korrektheit des 5,2 kb Inserts wurde durch Sequenzierung bestätigt. Anschließend wurde aus dem pUC19 Derivat das 5,2 kb-Fragment als *Sal*I-Fragment isoliert und in die *Sal*I-Schnittstelle des Vektors pEKEx2 ligiert. Anhand von Restriktionsverdaus wurden Plasmide mit der richtigen Orientierung ausgewählt, und einer davon als pEKEx2MCM cg1 bezeichnet. Das erhaltene Plasmid ist in Figur 21 gezeigt.

### 2. Klonierung der 3-Hydroxyisobutyrat Dehydrogenase aus Thermus thermophilus in pEKEx2, Konstruktion von pEC-XT99A MMCoAR 3HIBDH

Das Gen der 3-Hydroxyisobutyrat Dehydrogenase (3HIBDH)aus *Thermus thermophilus* (TTHA0237) wurde unter Berücksichtigung der Codon-Usage von *C*. *glutamicum* synthetisiert. Das Codon-Usage optimierte Gen (SEQ.-ID-Nr. 25) wurde in den Vektor pGA4 synthetisiert (pGA4_3HIBDH, Figur 22). Durch Verdau des Vektors pGA4_3HIBDH mit den Endonukleasen *Kpn*I und *Ecl*136II wurde das Gen der 3HIBDH in den mit den Restriktionsenzymen *Bam*HI (und anschließender Auffüllreaktion) und *Kpn*I linearisierten Vektor pGA5_MMCoAR, einen Vektor der bereits eine von der Methylmalonyl-Coenzym A-Mutase aus *Sulfolobus tokodaii* abgeleitete Sequenz unter funktioneller Expressionkontrolle des T7 Promoters enthält, ligiert. Der resultierende Vektor pGA5_MMCoAR_3HIBDH ist in Figur 23 dargestellt.

Aus dem Vektor pGA5_MMCoAR_3HIBDH wurde durch Verdau mit *KpnI* und *Ecl*136II das Insert mit der Methylmalonyl-Coenzym A-Reduktase und der 3HIBDH in den durch Verdau mit den Restriktionsendonukleasen *Bam*HI (und anschl. Auffüllreaktion) und *KpnI* linearisierten Zielvektor pECXT99A (Accession Number: AY219684, Figur 24) kloniert (resultierender Vektor pECXT99A_MMCoAR_3HIBDH, Figur 25).

### 3. Herstellung mit pEKEx2MCM cg1 und pEC-XT99A 3HIBDH varIIMMCoAR transformierter C. glutamicum Zellen.

Die Herstellung kompetenter Zellen von *C*. *glutamicum* ATCC13032 erfolgte wie bei Tauch et al. beschrieben (Curr Microbiol. 2002,45:362-367). DNA von pEKEx2MCM_cgl und pEC-XT99A_3HIBDH_varIIMMCoAR wurde mittels Elektroporation eingeführt, und Transformanten auf Hirn-Herz-Agar der Firma Merck (Darmstadt, Deutschland), der mit 50 mg/l Kanamycin und 5 mg/l Tetracyclin supplementiert worden war, selektioniert (Liebl et al. FEMS Microbiol Lett., 1989, 53:299-303). Von Transformanten wurde Plasmid DNA isoliert, und diese mittels Restriktionsverdau charakterisiert. Auf diese Weise wurde *C*. *glutamicum* pEKEx2MCM_cgl/pEC-XT99A_3HIBDH_varIIMMCoAR erhalten.

### 4. Kultivierung und Herstellung von 3-Hydroxyisobuttersäure

Eine Einzelkolonien von *C*. *glutamicum* pEKEx2MCM_cgl/pEC-XT99A_3HIBDH_varIIMMCoAR wurden in 25 ml Vollmedium (Hirn-Herz-Medium) mit 15 µg/l Kanamicin und 5 µg/l Tetracyclin angeimpft und über Nacht bei 30 °C und 200 rpm kultiviert. Als Kontrolle wurde der Wildtyp ohne Antibiotika angezogen.

Die Kulturen wurden abzentrifugiert (10 min, 4 °C, 5292 x g) und mit Saline (0,9 % NaCl) gewaschen. Die Zellen wurden in 25 ml (in 250 ml Kolben) GCXII-Medium mit Antibiotika (15 µg/l Kanamicin und 5 µg/l Tetracyclin) resuspendiert. Der plasmidtragende Stamm wurde mit 0,5 mM (12,5 µl der 1 M Stammlösung) IPTG induziert.

Minimalmedium CGXII (nach Keilhauer et al., J Bacteriol. 1993, 175:5595-5603):

| | |
|---|---|
| 20 g | NH₄)₂SO₄ |
| 5 g | Harnstoff |
| 1 g | K₂HPO₄ |
| 1 g | KH₂PO₄ |
| 42 g | MOPS |
| 1 ml | MgSO₄ × 7 H₂O (25 g/100ml, sterilfiltriert) |
| 1 ml | CaCl₂ × 2 H₂O (1,32 g/100ml, sterilfiltriert) |

Die Salze wurden in ca. 800 ml Aqua bidest. gelöst und der pH mit KOH auf 7 eingestellt. Dafür wurden ca. 20-25 KOH-Plätzchen zugeben und anschließend mit 10 N KOH auf pH 7 titriert. Die Medienbestandteile wurden dann mit Aqua bidest. auf 900 ml aufgefüllt und autoklaviert.

Nach dem Autoklavieren wurde 1 ml Spurensalzlösung zugegeben.

### Spurensalzlösung:

| | |
|---|---|
| 1 g | FeSO₄ × 7 H₂O |
| 1 g | MnSO₄ × H₂O |
| 0, 1g | ZnSO₄ × 7 H₂O |
| 0,02 g | CuSO₄ |
| 0,002 g | NiCl₂ × 6 H₂O |

Die Salze werden in 100 ml deionisiertem H₂O gelöst, und dabei mit HCl angesäuert (pH-Papier ca. pH 1). Anschließend wird die Lösung sterilfiltriert.

Zu dem Medium wurden 100 ml 50%iger Glucose (Endkonz. 5 %), für eine Endkonzentration von 4% werden 80 ml 50%ige Glucose und 20 ml steriles Aqua bidest. zugegeben

Das Medium wurde mit 1 ml Biotin (20 mg/100 ml), 60 µg/l Coenzym B12 (nach 7 h), 0,1 mM Propionat (nach 22 h) und Spurensalzlösung supplementiert.

Nach 27 h konnten in der Probe 6 mg/l 3-Hydroxyisobuttersäure durch Ionenchromatographie (Metrohm Compact IC 761 mit Autosampler, Mobile Phase: 8 mM NaOH; Säule: Dionex AS15 4 x 250 mm + Vorsäule AG15 4 x 50 mm; Säulentemperatur: 25°C, Fluss: 1,4 mL/min; Detektor: Leitfähigkeit; Injektionsvolumen: 10µl; Laufzeit: 30 min) nachgewiesen werden (Figur 26). Die Identität der 3-Hydroxyisobuttersäure wurde durch Aufstockung von chemisch reiner 3-Hydroxyisobuttersäure (30 mg/l) bestätigt (Figur 27).

### 5. Dehydratisierung von 3-Hydroxyisobuttersäure zu Methacrylat

5 ml Lösung von 3-Hydroxyisobuttersäure (0,2 g/L), produziert nach dem vorstehenden Beispiel 4, wird unter Rühren mit NaOH (0,06 mg) versetzt. Die Lösung wird unter Rührung und Rückflusskühlung bei 185 - 195°C unter Vakuum (300 torr) inkubiert. Über einen Zeitraum von 5 h werden stündlich jeweils weitere 0,5 mg 3-Hydroxyisobuttersäure in 5 mL zugesetzt. Die Lösung enthält 0,4 Gewichtsprozent p-Methoxyphenol, um ein Polymerisieren von Methacrylat zu verhindern. Nach 24 h Inkubation wird die Reaktion beendet. Der Umsatz von 3-Hydroxyisobuttersäure zu Methacrylat beläuft sich auf über 90 %. Die Abtrennung von Methacrylsäure aus dem Reaktionsansatz erfolgt durch Destillation.

### Beispiel 4

Die vorliegende Erfindung wird weiterhin im Beispiel 4 anhand der Herstellung von Methacrylsäure durch eine rekombinanten *E. Coli*-Zelle veranschaulicht, die 3-Hydroxyisobuttersäure über Methylmalonat-Semialdehyd als Vorprodukt *und über* Acryloyl-Coenzym A als Zwischenprodukt zu bilden vermag.

Zur Umsetzung der C-Quelle Glycerin zu 3-Hydroxyisobuttersäure mit rekombinanten *E. coli*-Zellen wurden die Gene für sieben verschiedene Enzyme in eine Reihe von Expressionsplasmiden kloniert. Dazu wurden die Duet-Vektoren (Merck, Deutschland) genutzt. Hierbei handelt es sich um ein System von vier Expressionsvektoren, die alle miteinander kompatibel sind und zudem unterschiedliche Antibiotikaresistenzmarker aufweisen.
1. Im Einzelnen wurden für die Umsetzung von Glycerin zu 3-Hydroxyisobuttersäure die Gene kodierend für folgende Enzyme in Expressionsvektoren kloniert:
   a) Glycerin-Dehydratase (EC 4.2.1.30)(GD)aus *Klebsiella pneumoniae.* Das Enzym katalysiert die Adenosylcobalamin-abhängige Dehydratisierung von Glycerin zu 3-HPA (3-Hydroxypropionaldehyd). Es besteht aus 3 Untereinheiten (GD-alpha, GD-beta und GD-gamma), die in *K.pneumoniae* von 3 Genen (*gldA, gldB* und *gldc*) in einem Operon kodiert vorliegen.
   b) Reaktivierungsfaktor aus *K. pneumoniae.* Da Adenosylcobalamin-abhängige Glycerin-Dehydratasen durch Glycerin inaktiviert werden, ist zusätzlich für die Umsetzung von Glycerin zu 3-HPA die Aktivität eines Reaktivierungsfaktors erforderlich. Der Reaktivierungsfaktor für die Glycerin Dehydratase aus *K*. *pneumoniae* wird von den Genen *gdrA* und *gdrB* kodiert.
   c) Aldehyd-Dehydrogenase AldH aus *E*. *coli.* Zur Umsetzung von 3-HPA zu 3-Hydroxypropionsäure (3-HP) wurde das *E*. *coli aldH*-Gen amplifiziert.
   d) Propionyl-Coenzym A Synthase (Pcs) aus *Chloroflexus aurantiacus* (kodiert durch das Gen *pcs*)*.* Die Propionyl-Coenzym A Synthase katalysiert die Umsetzung von 3-HP zu Propionyl-Coenzym A. Es handelt sich um ein trifunktionelles Enzym und beinhaltet drei funktionelle Domänen. Die Acyl-Coenzym A Synthetase (ACS)-Domäne katalysiert die Aktivierung von 3-HP zum 3-Hydroxypropionyl-CoA. Daran anschließend folgt die Dehydratisierung zum Acrylyl-CoA, katalysiert durch die Enoyl-CoA Hydratase (ECH)-Domäne der Pcs. Die Enoyl-CoA Reduktase (ECR)-Domäne der Pcs katalysiert schließlich die NADPH-abhängige Reduktion des Acrylyl-CoA zum Propionyl-CoA. Diese Reaktion ist für das beschriebene Vorhaben allerdings irrelevant, weil hier das Zwischenprodukt Acrylyl-CoA gleich durch das nächste Enzym (Crotonyl-CoA Carboxylase/Reduktase, s. u.) weiter umgesetzt wird.
   e) Crotonyl-Coenzym A Carboxylase/Reduktase (Enzym E₆₂) (Ccr) aus *Rhodobacter sphaeroides* (kodiert durch das Gen ccR). Die Hauptaktivität der Ccr liegt in der reduktiven Carboxylierung von Crotonyl-Coenzym A zu Ethylmalonyl-CoA. Das Enzym zeigt aber eine breite Substrat-Spezifität und setzt sehr effizient Acrylyl-Coenzym A zu Methylmalonyl-Coenzym A um
   f) Malonyl-CoA Reduktase (Mcr, E₂ und E₃) aus *Sulfolobus tokodaii* (kodiert durch das Gen *mcr.* Die Mcr katalysiert präferentiell die NADPH-abhängige Reduktion von Malonyl-Coenzym A zu MalonatSemialdehyd. Sie zeigt aber auch eine Nebenaktivität mit Methylmalonyl-Coenzym A als Substrat und setzt dieses zu Methylmalonat-Semialdehyd um.
   g) 3-Hydroxyisobutyrat-Dehydrogenase (E₄) (3-HIB-DH) aus *Thermus thermophilus* (kodiert durch das Gen *MmsB*)*.* Dieses Enzym katalysiert die NADPH-abhängige, reversible Umsetzung von Methylmalonat-Semialdehyd zu 3-Hydroxyisobuttersäure (3-HIB)
2. Die Klonierungsstrategie zur heterologen Überexpression der oben beschriebenen Enzyme wird im Folgenden detailliert beschrieben.
   a) Konstruktion von Plasmid pACYCDuet-KpGDRF zur Überexpression des Glycerin Dehydratase-Reaktivierungsfaktors (GDRF).
      Zunächst wurden die Gene *gdrA* (Synonym ORF4) und *gdrB* (Synonym ORF2b), welche die zwei Untereinheiten des *K. pneumoniae* GDRF kodieren, mittels PCR amplifiziert. Als Matrize wurde chromosomale DNA vom Stamm *K. pneumoniae DSM2026* verwendet.
      Zur Amplifizierung von *gdrA* wurden folgende Oligonukleotide eingesetzt:
      orf4fw (SEQ.-ID-Nr. 26):
         5'-TGAAGATCCTAGGAGGTTTAAACATATGCCGTTAATAGCCGGGATTG-3')
      orf4Salrv (SEQ.-ID-Nr. 27):
         5'-TATATAGTCGACTTAATTCGCCTGACCGGCCAG-3';
         (*Sal*I Erkennungssequenz unterstrichen).

      Zur Amplifizierung von *gdrB* wurden folgende Oligonukleotide eingesetzt:
      orf2bPcifw (SEQ.-ID-Nr. 28):
         5'-TATATAACATGTCGCTTTCACCGCCAGGC-3'
         (PciI-Erkennungssequenz unterstrichen)
      orf2brv (SEQ.-ID-Nr. 29):
         5'-CATATGTTTAAACCTCCTAGGATCTTCAGTTTCTCTCACTTAACGGCA GG-3').

      Die erhaltenen PCR-Produkte wurden nachfolgend durch Crossover-PCR miteinander fusioniert.
      Dafür wurden folgende Oligonukleotide eingesetzt:
      orf2bNcofw (SEQ.-ID-Nr. 30):
         (5'-TATATACCATGGCGCTTTCACCGCCAGGC-3'
         (NcoI-Erkennungssequenz unterstrichen)
      orf4Salrv (SEQ.-ID-Nr. 31):
         5'-TATATAGTCGACTTAATTCGCCTGACCGGCCAG-3'
         (SalI-Erkennungssequenz unterstrichen)

      Das PCR-Produkt (2.220 bp) wurde mittels des QIAquick-PCR-Aufreinigungskits von Qiagen, Hilden, gemäß den Angaben des Herstellers aufgereinigt und in den Vektor pCR-BluntII-TOPO unter Erhalt des pCR-BluntII-Topo-KpGDRF-Vektors ligiert. Die Ligation und anschließende Transformation in E. *coli* Zellen erfolgt nach den Angaben des Herstellers Invitrogen Corporation, Carlsbad (Zero Blunt TOPO PCR Cloning Kit).
      Die GDRF-Sequenz wurde anschließend durch Verdau von pCR-BluntII-Topo-KpGDRF mit *Pci*I und *Sal*I aus dem Vektor herausgeschnitten und in den mit *Nco*I und *Sal*I geschnittenen pACYC-Duet-Expressionsvektor unter Erhalt von pACYCDuet-KpGDRF (6.142 bp) ligiert.
   b) Konstruktion von Plasmid pAS50_Ec_aldH zur Überexpression der *K. pneumoniae* Glycerin-Dehydratase (GD) und der *E. coli* Aldehyd-Dehydrogenase aldH.
      Die drei Untereinheiten der *K. pneumoniae* GD sind natürlicherweise in einem Operon organisiert (Gene *gldA, gldB* und *gldC*)*.* Sie wurden mittels PCR amplifiziert, wobei als Matrize wieder chromosomale DNA von *K. pneumoniae DSM2026* verwendet wurde.
      Folgende Oligonukleotide wurden für die Amplifizierung eingesetzt:
      KpGDNdefw (SEQ.-ID-Nr. 32)
         5'-TATATACATATGAAAAGATCAAAACGATTTGCAGTACTGG-3'
         (NdeI-Erkennungssequenz unterstrichen)
      KpGDSalrv (SEQ.-ID-Nr. 33):
         5'-TATATAGTCGACTTAGCTTCCTTTACGCAGCTTATGC-3'
         (SalI-Erkennungssequenz unterstrichen)

      Das Amplifikat wurde in den Vektor pCR-BluntII-TOPO unter Erhalt des pCR-BluntII-Topo-KpGD-Vektors ligiert. Die Ligation und anschließende Transformation in *E*. *coli* Zellen erfolgte nach den Angaben des Herstellers Invitrogen Corporation, Carlsbad (Zero Blunt TOPO PCR Cloning Kit).
      Das die GD kodierende Fragment wurde mit *Xba*I (geblunted durch Klenow fill in) und *Nde*I aus dem Vektor pCR-BluntII-Topo-KpGD herausgeschnitten und in einen mit *Nde*I und *Eco*RV geschnittenen pET-Duet-Expressionsvektor unter Erhalt des Plasmids pAS50(8161bp) ligiert.
      Nachfolgend wurde das *E*. *coli aldH*-Gen amplifiziert. Hierfür wurde chromosomale DNA von *E*. *coli K12* als Templat eingesetzt, als PCR-Primer wurden die Oligonukleotide
      1228_ald_fp (SEQ.-ID-Nr. 34):
         5'-AAAACATATGAATTTTCATCATCTGGCTTACTGG-3'
         (NdeI-Erkennungssequenz unterstrichen) und
      1228_ald_rp (SEQ.-ID-Nr. 35)
         5'-AAAACATATGTATATTTCCTTCTTTCAGGCCTCCAGGCTTATCCAGATG-3')
         (NdeI-Erkennungssequenz unterstrichen)

      eingesetzt. Das PCR-Amplifikat wurde übers Gel gereinigt und anschließend durch NdeI-Verdau in die *Nde*I site von Plasmid pAS50 ligiert, wobei das Plasmid pAS50_Ec_aldH (9.666 bp)erhalten wurde.
   c) Konstruktion von Plasmid pCDFDuet-1_Rs_ccR_Cau_pcs zur Überexpression der *Chloroflexus aurantiacus* Propionyl-Coenzym A Synthase (Pcs) sowie der *Rhodobacter sphaeroides* Crotonyl-Coenzym A Carboxylase/Reduktase (CCR).
      Zur heterologen Expression in *E. coli* und Aufreinigung der CCR wurde das Gen unter Erhalt von Plasmid pTE13 in den Expressionvektor pET3d kloniert: Das *R. sphaeroides* ccr-Gen wurde unter Verwendung der Oligonukleotide
      ccr-fw (SEQ.-ID-Nr. 36):
         5'-GGAGGCAACCATGGCCCTCGACGTGCAGAG-3'
         (NcoI-Erkennungssequenz unterstrichen) und
      ccr-rev (SEQ.-ID-Nr. 37):
         5'-GAGACTTGCGGATCCCTCCGATCAGGCCTTGC-3'
         (BamHI-Erkennungssequenz unterstrichen)

      durch PCR amplifiziert, wobei chromosomale DNA des Stammes *R. sphaeroides* 2.4.1 (DSMZ 158) als *Templa*te eingesetzt wurde. Das PCR-Produkt wurde als *Nco*I/*BamH*I-Fragment in den mit *Nco*I/*BamH*Igeschnittenen Vektor pET3d (Merck, Deutschland) ligiert, wobei das Plasmid pTE13 erhalten wurde.
      Aus dem pTE13 wurde das ccr-Gen als *Nco*I/*BamH*I-Fragment in die *Nco*I/*BamH*I-Schnittstellen des Plasmids pCDFDuet-1 (Merck, Deutschland) umkloniert, wobei das Plasmid pCDFDuet-1_Rs_ccr erhalten wurde.
      Nachfolgend wurde das *C*. *aurantiacus pcs*-Gen mit den Oligonukleotiden
      1228_Cau_pcs_fp(71) (SEQ.-ID-Nr. 38):
         5'-AAAACATATGATCGACACTGCGCCCCTTGC-3'
         (NdeI-Erkennungssequenz unterstrichen)und
      1228_Cau_pcs_rp(74) (SEQ.-ID-Nr. 39):
         5'-AAGACGTCCTACCGCTCGCCGGCCGTCC-3'
         (AatII-Erkennungssequenz unterstrichen)
      durch PCR amplifiziert, wobei chromosomale DNA des Stammes *C. aurantiacus* OK-70-fl (DSM 636) als *Template* verwendet wurde. Das Amplifikat wurde nach Aufreinigung durch Gelextraktion über NdeI/AatII-Verdau in den entsprechend geschnittenen Vektor pCDFDuet-1_Rs_ccr ligiert, wobei das Plasmid pCDFDuet-1_Rs_ccR_Cau_pcs (10472 bp) erhalten wurde.
   d) Konstruktion von Plasmid pCOLADuet_St_mcr_oCg_Tth_HIBDH_oCg zur Überexpression der *Sulfolobus tokodaii* Malonyl-CoA Reduktase (Mcr) sowie der *Thermus thermophilus* 3-Hydroxyisobutyrat Dehydrogenase (3-HIB-DH)
      Durch Gen-Synthese wurde zunächst eine an die Codon-Usage von *Corynebacterium glutamicum* angepasste Variante des *S*. *tokodaii* Gens *mcr* hergestellt (*St_mcr_oCg*). Die Synthese erfolgte bei der Firma GeneArt, Deutschland, und das artifizielle Gen *St_mcr_oCg* wurde in Form des Plasmids pGA4_MMCoAR_ST (SEQ.-ID-Nr. 40) bereitgestellt. pGA4_MMCoAR_ST DNA wurde als PCR-Template eingesetzt, um das artifizielle Gen *St_mcr_oCg* mit den Oligonukleotiden
      1228_MMCoAR_fp (SEQ.-ID-Nr. 41):
         5'-AACCATGGGCCGCACCCTGAAGG-3'
         (NcoI-Erkennungssequenz unterstrichen) und
      1228_MMCoAR_rp (SEQ.-ID-Nr. 42)
         5'-AAGGATCCTTACTTTTCGATGTAGCCCTTTTCC-3'
         (*BamH*I-Erkennungssequenz unterstrichen)

      zu amplifizieren. Nach Aufreinigung durch Gelextraktion wurde das Amplifikat mit *Nco*I/*BamH*I verdaut und in die entsprechenden Schnittstellen des Plasmids pCOLADuet_1 (Merck, Deutschland) ligiert, wobei das Plasmid pCOLADuet_St_mcr_oCg erhalten wurde.
      Eine an die *Codon Usage* von *Corynebacterium glutamicum* angepasste Variante des *T. thermophilus* Gens *MmsB* (kodierend eine 3-HIB-DH) wurde ebenfalls durch Gensynthese (GeneArt, Deutschland) bereitgestellt, und zwar in Form des Plasmids pGA4_3HIBDH_TT (SEQ.-ID-Nr. 43).
      pGA4_3HIBDH_TT wurde als PCR-Template eingesetzt, um das artifizielle Gen *Tth_HIBDH_oCg* mit den Oligonukleotiden
      1228_Tth_HIBDH_fp (SEQ.-ID-Nr. 44):
         5'-AAAACATATGGAAAAGGTGGCATTCATCG-3'
         (*Nde*I-Erkennungssequenz unterstrichen) und
      1228_Tth_HIBDH_rp (SEQ.-ID-Nr. 45):
         5'-AAA*AGAT*C*T*TTAGCGGATTTCCACACCGCC-3'
         (*Bgl*II-Erkennungssequenz unterstrichen)
      zu amplifizieren. Nach Gel-Extraktion wurde das Amplifikat mit *Nde*I/*Bgl*II geschnitten und in die NdeI/BglII-Schnittstellen des Plasmids pCOLADuet_St_mcr_oCg ligiert, wobei das Plasmid pCOLADuet_St_mcr_oCg_Tth_HIBDH_oCg (5.620 bp) erhalten wurde.
   e) Die 4 Plasmide pACYCDuet-KpGDRF, pAS50_Ec_aldH, pCDFDuet-1_Rs_ccR_Cau_pcs und pCOLADuet_St_mcr_oCg_Tth_HIBDH_oCg wurden nachfolgend gemäß Herstellerprotokoll in kommerziell erhältliche, chemisch kompetente *E*. *coli* BL21 (DE3)-Zellen (Merck, Deutschland) co-transformiert. Die Selektion erfolgte auf LB-Agar supplementiert mit Ampicillin (25 µg/ml), Chloramphenicol (17 µg/ml), Kanamycin (15 µg/ml) und Streptomycin (25 µg/ml).
   f) Induktion der Expressionsplasmide in *E*. *coli*
      Die in e) vorstehend beschriebenen, plasmidtragenden *E*. *coli*-Stämme wurden in modifiziertem M9-Medium (6,8 g/l Na₂HPO₄ × 2H₂O; 3 g/l KH₂PO₄; 0,5 g/l NaCl; 1 g/l NH₄Cl; 1,25 g/l Hefeextrakt; 1% v/v Glycerin; 15 mg/l CaCl₂ × 2H₂O; 250 mg/l MgSO₄ × 7H₂O; 1% v/v Gibco MEM Vitamin Solution; 41,9 g/l MOPS) kultiviert. Das Medium wurde mit Ampicillin (25 µg/ml), Chloramphenicol (17 µg/ml), Kanamycin (15 µg/ml) und Streptomycin (25 µg/ml) supplementiert. Die gesamte Kultivierung (Vor- und Hauptkulturen) erfolgte auf einem temperierbaren Schüttler bei 37 °C. Zunächst wurden die Stämme in 5 ml Medium über Nacht kultiviert. Nachfolgend wurden 20 ml Medium im 100 ml Schikanekolben im Verhältnis 1 : 20 aus der Übernachtkultur beimpft und weiter kultiviert. Bei Erreichen von OD₆₀₀ ca 0,8 wurden 6 µM Cobalamin und 1 µM IPTG zugegeben und 4 Stunden weiter inkubiert. Zu diesem Zeitpunkt wurden 2,5 ml Zellsuspension abgenommen und bis zur Analyse bei - 20 °C gelagert.
   g) Der Nachweis und Quantifizierung von 3-HIB erfolgte mittels Ionenchromatografie (IC) und Leitfähigkeitsdetektion. Hierfür werden 2,5 ml Proben bei Raumtemperatur aufgetaut und abzentrifugiert (10 min 13.200 rpm). Der Überstand wird über einen Spritzenfilter (Porengrösse 0,44 µm) gereinigt. Die Messung erfolgt mit einem Metrohm Compact IC 761 mit Autosampler. Mobile Phase: 8 mM NaOH. Säule: Dionex AS15 4 x 250 mm, Vorsäule AG15 4 x 50 mm. Säulentemperatur: 25 °C. Flussrate: 1,4 ml/min. Injektionsvolumen: 10 µl.
   h) Dehydratisierung von 3-Hydroxyisobuttersäure zu Methacrylat
      5 ml aufkonzentrierte Lösung von 3-Hydroxyisobuttersäure (0,2 g/L), produziert nach dem vorstehenden, unter f) beschriebenen Verfahren, wird unter Rühren mit NaOH (0,06 mg) versetzt. Die Lösung wird unter Rührung und Rückflusskühlung bei 185 - 195°C unter Vakuum (300 torr) inkubiert. Über einen Zeitraum von 5 h werden stündlich jeweils weitere 0,5 mg 3-Hydroxyisobuttersäure in 5 mL zugesetzt. Die Lösung enthält 0,4 Gewichtsprozent p-Methoxyphenol, um ein Polymerisieren von Methacrylat zu verhindern. Nach 24 h Inkubation wird die Reaktion beendet. Der Umsatz von 3-Hydroxyisobuttersäure zu Methacrylat beläuft sich auf über 90 %. Die Abtrennung von Methacrylsäure aus dem Reaktionsansatz erfolgt durch Destillation.

## Patentansprüche

1. Ein Verfahren zur Herstellung von Methacrylsäure oder Methacrylsäureestern, umfassend die Verfahrensschritte
IA) Herstellung von 3-Hydroxyisobuttersäure durch ein Verfahren umfassend den Verfahrensschritt des in Kontakt bringens einer Zelle, welche gegenüber ihrem Wildtyp derart gentechnisch verändert wurde, dass sie im Vergleich zu ihrem Wildtyp mehr 3-Hydroxyisobuttersäure oder auf 3-Hydroxyisobuttersäure basierende Polyhydroxyalkanoate zu bilden vermag, mit einem Nährmedium beinhaltend als Kohlenstoffquelle Kohlenhydrate, Glycerin, Kohlendioxid, Methan, Methanol, L-Valin oder L-Glutamat unter Bedingungen, unter denen aus der Kohlenstoffquelle 3-Hydroxyisobuttersäure oder auf 3-Hydroxyisobuttersäure basierende Polyhydroxyalkanoate gebildet werden, gegebenenfalls Aufreinigung der 3-Hydroxyisobuttersäure aus dem Nährmedium sowie gegebenenfalls Neutralisation der 3-Hydroxyisobuttersäure,
IB) Dehydratisierung des 3-Hydroxyisobuttersäure unter Bildung von Methacrylsäure sowie gegebenenfalls Veresterung der Methacrylsäure, wobei
1) die Bildung von 3-Hydroxyisobuttersäure oder von auf 3-Hydroxyisobuttersäure basierenden Polyhydroxyalkanoaten über Methylmalonat-Semialdehyd als Vorprodukt erfolgt, und wobei
1a) die Zelle 3-Hydroxyisobuttersäure oder auf 3-Hydroxyisobuttersäure basierende Polyhydroxyalkanoate über Succinyl-Coenzym A als Zwischenprodukt zu bilden vermag, und wobei
1a₁) die Zelle eine im Vergleich zu ihrem Wildtyp gesteigerte Aktivität eines Enzyms E₁ aufweist, welches die Umsetzung von Succinyl-Coenzym A zu Methylmalonyl-Coenzym A katalysiert, und/oder
1a₂) die Zelle eine im Vergleich zu ihrem Wildtyp gesteigerte Aktivität mindestens eines der folgenden Enzyme E₂ bis E₄ aufweist:
- eines Enzyms E₂, welches die Umsetzung von Methylmalonyl-Coenzym A zu Methylmalonat katalysiert,
- eines Enzyms E₃, welches die Umsetzung von Methylmalonat zu Methylmalonatsemialdehyd katalysiert,
- eines Enzyms E₄, welches die Umsetzung von Methylmalonat-semialdehyd zu 3-Hydroxyisobutyrat katalysiert, oder
1a₃) die Zelle eine im Vergleich zu ihrem Wildtyp gesteigerte Aktivität mindestens eines der folgenden Enzyme E₄, E₅, E₆ und E₇ aufweist:
- eines Enzyms E₆, welches die Umsetzung von (R)-Methylmalonyl-Coenzym A zu (S)-Methylmalonyl-Coenzym A katalysiert,
- eines Enzyms E₇, welches die Umsetzung von (S)-Methylmalonyl-Coenzym A zu Propionyl-Coenzym A katalysiert,
- eines Enzyms E₅, welches die Umsetzung von Propionyl-Coenzym A zu Methylmalonatsemialdehyd katalysiert,
- eines Enzyms E₄, welches die Umsetzung von Methylmalonatsemialdehyd zu 3-Hydroxyisobuttersäure katalysiert, oder
1a₄) die Zelle eine im Vergleich zu ihrem Wildtyp gesteigerte Aktivität mindestens eines der folgenden Enzyme E₄, E₅ und E₇ aufweist:
- eines Enzyms E₇, welches die Umsetzung von Methylmalonyl-Coenzym A zu Propionyl-Coenzym A katalysiert,
- eines Enzyms E₅, welches die Umsetzung von Propionyl-Coenzym A zu Methylmalonatsemialdehyd katalysiert,
- eines Enzyms E₄, welches die Umsetzung von Methylmalonatsemialdehyd zu 3-Hydroxyisobuttersäure katalysiert, oder
1a₅) die Zelle eine im Vergleich zu ihrem Wildtyp gesteigerte Aktivität mindestens eines der folgenden Enzyme E₂₈ und E₄₆ aufweist:
- eines Enzyms E₄₆, welches die Umsetzung - von L-Glutamat zu 2-Oxoglutarat katalysiert,
- eines Enzyms E₂₈, welches die Umsetzung von 2-Oxoglutarat zu Succinyl-Coenzym A katalysiert, oder
1b) die Zelle 3-Hydroxyisobuttersäure oder auf 3-Hydroxyisobuttersäure basierende Polyhydroxyalkanoate über Propionyl-Coenzym A als Zwischenprodukt zu bilden vermag und wobei 1b₁
) die Zelle eine im Vergleich zu ihrem Wildtyp gesteigerte Aktivität mindestens eines der folgenden Enzyme E₄, E₅ und E₄₇ bis E₅₂ aufweist:
- eines Enzyms E₄₇, welches die Umsetzung von Acetyl-Coenzym A zu Malonyl-Coenzym A katalysiert,
- eines Enzyms E₄₈, welches die Umsetzung von Malonyl-Coenzym A zu Malonatsemialdehyd katalysiert,
- eines Enzyms E₄₉, welches die Umsetzung von Malonatsemialdehyd zu 3-Hydroxypropionat katalysiert,
- eines Enzyms E₅₀, welches die Umsetzung von 3-Hydroxypropionat zu 3-Hydroxypropionyl-Coenzym A katalysiert,
- eines Enzyms E₅₁, welches die Umsetzung von 3-Hydroxypropionyl-Coenzym A zu Acryloyl-Coenzym A katalysiert,
- eines Enzyms E₅₂, welches die Umsetzung von Acryloyl-Coenzym A zu Propionyl-Coenzym A katalysiert,
- eines Enzyms E₅, welches die Umsetzung von Propionyl-Coenzym A zu Methylmalonatsemialdehyd katalysiert,
- eines Enzyms E₄, welches die Umsetzung von Methylmalonatsemialdehyd zu 3-Hydroxyisobutyrat katalysiert, oder
1c) die Zelle 3-Hydroxyisobuttersäure oder auf 3-Hydroxyisobuttersäure basierende Polyhydroxyalkanoate über Acryloyl-Coenzym A als Zwischenprodukt zu bilden vermag und
1c₁) die Zelle eine im Vergleich zu ihrem Wildtyp gesteigerte Aktivität mindestens eines der nachfolgenden Enzyme E₂ bis E₄, E₅₆, E₇₂ und E₇₃ aufweist:
- eines Enzyms E₇₂, welches die Umsetzung von Beta-Alanin zu Beta-Alanyl-Coenzym A katalysiert,
- eines Enzyms E₇₃, welches die Umsetzung von Beta-Alanyl-Coenzym A zu Acrylyl-Coenzym A katalysiert,
- eines Enzyms E₅₆, welches die Umsetzung von Acrylyl-Coenzym A zu Methylmalonyl-Coenzym A katalysiert,
- eines Enzyms E₂, welches die Umsetzung von Methylmalonyl-Coenzym A zu Methylmalonat katalysiert,
- eines Enzyms E₃, welches die Umsetzung von Methylmalonat zu Methylmalonat-Semialdehyd katalysiert,
- eines Enzyms E₄, welches die Umsetzung von Methylmalonat-Semialdehyd zu 3-Hydroxyisobuttersäure katalysiert, oder
2) die Bildung von 3-Hydroxyisobuttersäure oder von auf 3-Hydroxyisobuttersäure basierenden Polyhydroxyalkanoaten über 3-Hydroxyisobutyryl-Coenzym A als Vorprodukt erfolgt und wobei
2a) die Zelle 3-Hydroxyisobuttersäure oder auf 3-Hydroxyisobuttersäure basierende Polyhydroxyalkanoate über Isobutyryl-Coenzym A als Zwischenprodukt zu bilden vermag, wobei 2a₁
) die Zelle eine im Vergleich zu ihrem Wildtyp gesteigerte Aktivität mindestens eines der folgenden Enzyme E₇₆ bis E₇₉, E₆₀, E₆₁ und E₈ aufweist:
- eines Enzyms E₇₆, welches die Umsetzung von Pyruvat zu 2-Acetolactat katalysiert,
- eines Enzyms E₇₇, welches die Umsetzung von 2-Acetolactat zu 2,3-Dihydroxyisolvalerat katalysiert,
- eines Enzyms E₇₈, welches die Umsetzung von 2,3-Dihydroxyisolvalerat zu 2-Oxoisovalerat katalysiert,
- eines Enzyms E₇₉, welches die Umsetzung von 2-Oxoisovalerat zu Isobutyryl-Coenzym A katalysiert,
- eines Enzyms E₆₀, welches die Umsetzung von Isobutyryl-Coenzym A zu Methacrylyl-Coenzym A katalysiert,
- eines Enzyms E₆₁, welches die Umsetzung von Methacrylyl-Coenzym A zu 3-Hydroxyisobutyryl-Coenzym A katalysiert,
- eines Enzyms E₈, welches die Umsetzung von 3-Hydroxyisobutyryl-Coenzym A zu 3-Hydroxyisobutyrat katalysiert, oder
2a₂) die Zelle eine im Vergleich zu ihrem Wildtyp gesteigerte Aktivität mindestens eines der folgenden Enzyme E₈, E₆₀ bis E₆₁ und E₇₉ bis E₈₀ aufweist:
- eines Enzyms E₈₀, welches die Umsetzung von L-Valin zu 2-Oxoisovalerat katalysiert,
- eines Enzyms E₇₉, welches die Umsetzung von 2-Oxoisovalerat zu Isobutyryl-Coenzym A katalysiert,
- eines Enzyms E₆₀, welches die Umsetzung von Isobutyryl-Coenzym A zu Methacrylyl-Coenzym A katalysiert,
- eines Enzyms E₆₁, welches die Umsetzung von Methacrylyl-Coenzym A zu 3-Hydroxyisobutyryl-Coenzym A katalysiert,
- eines Enzyms E₈, welches die Umsetzung von 3-Hydroxyisobutyryl-Coenzym A zu 3-Hydroxyisobutyrat katalysiert.

2. Das Verfahren nach Anspruch 1, wobei das Enzym E₁ in 1a₁) eine Methylmalonyl-Coenzym A-Mutase (EC 5.4.99.2) ist.

3. Das Verfahren nach Anspruch 1, wobei in 1a₁) das Enzym
E₂ eine Methylmalonyl-Coenzym A-Hydrolase (EC 3.2.1.17),
E₃ eine Aldehyd-Dehydrogenase (EC 1.2.1.3) oder eine Aldehyd-Oxidase (EC 1.2.3.1) und
E₄ eine 3-Hydroxyisobutyrat-Dehydrogenase (EC 1.1.1.31) oder eine 3-Hydroxyacyl-Coenzym A-Dehydrogenase (EC 1.1.1.35)
ist.

4. Das Verfahren nach Anspruch 1, wobei in 1a₃₎ das Enzym
E₆ eine Methylmalonyl-Coenzym A-Epimerase (EC 5.1.99.1),
E₇ eine Methylmalonyl-Coenzym A-Decarboxylase (EC 4.1.1.41),
E₅ eine Methylmalonatsemialdehyd-Dehydrogenase (EC 1.2.1.27), und
E₄ eine 3-Hydroxyisobutyrat-Dehydrogenase (EC 1.1.1.31) oder eine 3-Hydroxyacyl-Coenzym A-Dehydrogenase (EC 1.1.1.35)
ist.

5. Das Verfahren nach Anspruch 1, wobei in 1a₄) das Enzym
E₇ eine Methylmalonyl-Coenzym A-Decarboxylase (EC 4.1.1.41),
E₅ eine Methylmalonatsemialdehyd-Dehydrogenase (EC 1.2.1.27), und
E₄ eine 3-Hydroxyisobutyrat-Dehydrogenase (EC 1.1.1.31) oder eine 3-Hydroxyacyl-Coenzym A-Dehydrogenase (EC 1.1.1.35)
ist.

6. Das Verfahren nach Anspruch 1, wobei in 1a₅) das Enzym
E₄₆ eine Glutamat-Synthase (EC 1.4.1.13 oder EC 1.4.1.14), eine Glutamat-Dehydrogenase (EC 1.4.1.2, EC 1.4.1.3 oder EC 1.4.1.4) oder eine Aspartat-Transaminase (EC 2.6.1.1 oder EC 2.6.1.2) und
E₂₈ eine 2-Oxoglutarat-Synthase (EC 1.2.7.3)
ist.

7. Das Verfahren nach Anspruch 1, wobei in 1b₁) das Enzym
E₄ eine 3-Hydroxyisobutyrat-Dehydrogenase (EC 1.1.1..31) oder eine 3-Hydroxyacyl-Coenzym A-Dehydrogenase (EC 1.1.1.35),
E₅ eine Methylmalonatsemialdehyd-Dehydrogenase (EC 1.2.1..27),
E₄₇ eine Malonyl-Coenzym A-Decarboxylase (EC 4.1.1.9), eine Malonat-Coenzym A-Transferase (EC 2.8.3.3), eine Methylmalonyl-Coenzym A-Carboxytransferase (EC 2.1.3.1) oder eine Acetyl-Coenzym A-Carboxylase (EC 6.4.1.2),
E₄₈ eine Malonatsemialdehyd-Dehydrogenase (EC 1.2.1.18),
E₄₉ eine 3-Hydroxypropionat-Dehydrogenase (EC 1.1.1.59),
E₅₀ eine 3-Hydroxyisobutyryl-Coenzym A Hydrolase (EC 3.1.2.4),
E₅₁ eine Enoyl-Coenzym A-Hydratase (EC 4.2.1.17) und
E₅₂ eine Acyl-Coenzym A-Dehydrogenase (EC 1.3.99.3),
ist.

8. Das Verfahren nach Anspruch 1, wobei in 1c₁) das Enzym
E₇₂ eine Coenzym A-Transferase (EC 2.8.3.1) oder Coenzym A-Synthetase, vorzugsweise eine Coenzym A-Transferase,
E₇₃ eine Beta-Alanyl-Coenzym A-Ammonium-Lysase (EC 4.3.1.6),
E₅₆ eine Crotonyl-Coenzym A-Decarboxylase
E₂ eine Methylmalonyl-Coenzym A-Hydrolase (EC 3.1.2.17),
E₃ eine Aldehyd-Dehydrogenase (EC 1.2.1.3) oder eine Aldehyd-Oxidase (EC 1.2.3.1) und
E₄ eine 3-Hydroxyisobutyrat-Dehydrogenase (EC 1.1.1.31) oder eine 3-Hydroxyacyl-Coenzym A-Dehydrogenase (EC 1.1.1.35)
ist.

9. Das Verfahren nach Anspruch 1, wobei in 2a₂) das Enzym
E₈ eine 3-Hydroxyisobutyryl-Coenzym A-Hydrolase (EC 3.1.2.4),
E₇₆ eine Acetolactat-Synthase (EC 2.2.1.6),
E₇₇ eine Dihydroxyisovalerat-Dehydrogenase (EC 1.1.1.86),
E₇₈ eine 2,3-Dihydroxyisovalerat-Dehydratase (EC 4.2.1.9),
E₇₉ eine 2-Oxoisovalerat-Dehydrogenase (EC 1.2.1.25 oder EC 1.2.4.4),
E₆₀ eine Acyl-Coenzym A-Dehydrogenase (EC 1.3.99.3), eine Butyryl-Coenzym A-Dehydrogenase (EC 1.3.99.2) oder eine 2-Methylacyl-Coenzym A-Dehydrogenase (EC 1.3.99.12), und
E₆₁ eine Enoyl-Coenzym A-Hydratase (EC 4.2.1.17)
ist.

10. Das Verfahren nach Anspruch 1, wobei in 2a₂) das Enzym
E₈ eine 3-Hydroxyisobutyryl-Coenzym A-Hydrolase (EC 3.1.2.4),
E₆₀ eine Acyl-Coenzym A-Dehydrogenase (EC 1.3.99.3), eine Butyryl-Coenzym A-Dehydrogenase (EC 1.3.99.2) oder eine 2-Methylacyl-Coenzym A-Dehydrogenase (EC 1.3.99.12),
E₆₁ eine Enoyl-Coenzym A-Hydratase (EC 4-2.1.17),
E₇₉ eine 2-Oxoisovalerat-Dehydrogenase (EC 1.2.1.25 oder EC 1.2.4.4), und
E₈₀ eine Aminosäure-Transferase (EC 2.6.1.42),
ist.

11. Das Verfahren nach Anspruch 1, wobei die Zelle erhältlich ist durch ein Verfahren zur Herstellung einer gentechnisch veränderten Zelle, umfassend den Verfahrensschritt der Erhöhung der Aktivität mindestens eines der in den Ansprüchen 1 bis 10 genannten Enzyme in der Zelle.

12. Ein Verfahren zur Herstellung von Polymethacrylsäure oder Polymethacrylsäureestern, umfassend die Verfahrensschritte
IIIA) Herstellung von Methacrylsäure durch ein Verfahren nach einem der Ansprüche 1 bis 11,
IIIB) Radikalische Polymerisation der Methacrylsäure, wobei gegebenenfalls die Carboxylgruppen der Methacrylsäure bzw. die Carboxylatgruppe des Methacrylates vor oder nach der radikalischen Polymerisation zumindest teilweise verestert werden können.

## Claims

1. A process for the preparation of methacrylic acid or methacrylic esters, comprising the process steps of
IA) preparation of 3-hydroxyisobutyric acid by a process comprising the process step of bringing a cell which has been genetically modified in comparison with its wild type in such a way that it is capable of forming more 3-hydroxyisobutyric acid, or polyhydroxyalkanoates based on 3-hydroxyisobutyric acid in comparison with its wild type, into contact with a nutrient medium comprising, as carbon source, carbohydrates, glycerol, carbon dioxide, methane, methanol, L-valine or L-glutamate under conditions under which 3-hydroxyisobutyric acid or polyhydroxyalkanoates based on 3-hydroxyisobutyric acid are formed from the carbon source, if appropriate, isolation of the 3-hydroxyisobutyric acid from the nutrient medium and also, if appropriate, neutralization of the 3-hydroxyisobutyric acid, IB
) dehydration of the 3-hydroxyisobutyric acid with formation of methacrylic acid and also, where appropriate, esterification of the methacrylic acid, where
1) the formation of 3-hydroxyisobutyric acid or of polyhydroxyalkanoates based on 3-hydroxyisobutyric acid takes place via methylmalonate semialdehyde as precursor, and where
1a) the cell is capable of forming 3-hydroxyisobutyric acid or polyhydroxyalkanoates based on 3-hydroxyisobutyric acid via succinyl-coenzyme A as intermediate, and where 1a₁) the cell features an activity of an enzyme E₁, which catalyzes the conversion of succinyl-coenzyme A into methylmalonyl-coenzyme A, which is increased in comparison with its wild type, and/or
1a₂) the cell features an activity of at least one of the following enzymes E₂ to E₄ which is increased in comparison with its wild type:
- of an enzyme E₂, which catalyzes the conversion of methylmalonyl-coenzyme A into methyl malonate;
- of an enzyme E₃, which catalyzes the conversion of methyl malonate into methylmalonate semialdehyde;
- of an enzyme E₄ which catalyzes the conversion of methylmalonate semialdehyde into 3-hydroxyisobutyrate, or
1a₃) the cell features an activity of at least one of the following enzymes E₄, E₅, E₆ and E₇ which is increased in comparison with its wild type:
- of an enzyme E₆, which catalyzes the conversion of (R) methylmalonyl-coenzyme A into (S) methylmalonyl-coenzyme A;
- of an enzyme E₇, which catalyzes the conversion of (S) methylmalonyl-coenzyme A into propionyl-coenzyme A;
- of an enzyme E₅, which catalyzes the conversion of propionyl-coenzyme A into methylmalonate semialdehyde;
- of an enzyme E₄, which catalyzes the conversion of methylmalonate semialdehyde into 3-hydroxyisobutyric acid, or
1a₄) the cell features an activity of at least one of the following enzymes E₄, E₅ and E₇ which is increased in comparison with its wild type:
- of an enzyme E₇, which catalyzes the conversion of methylmalonyl-coenzyme A into propionyl-coenzyme A;
- of an enzyme E₅, which catalyzes the conversion of propionyl-coenzyme A into methylmalonate semialdehyde;
- of an enzyme E₄, which catalyzes the conversion of methylmalonate semialdehyde into 3-hydroxyisobutyric acid, or
1a₅) the cell features an activity of at least one of the following enzymes E₂₈ and E₄₆ which is increased in comparison with its wild type:
- of an enzyme E₄₆, which catalyzes the conversion of L-glutamate into 2-oxoglutarate;
- of an enzyme E₂₈, which catalyzes the conversion of 2-octoglutarate into succinyl-coenzyme A, or
1b) the cell is capable of forming 3-hydroxyisobutyric acid or polyhydroxyalkanoates based on 3-hydroxyisobutyric acid via propionyl-coenzyme A as intermediate, and where 1b₁) the cell features an activity of at least one of the following enzymes E₄, E₅ and E₄₇ to E₅₂ which is increased in comparison with its wild type:
- of an enzyme E₄₇, which catalyzes the conversion of acetyl-coenzyme A into malonyl-coenzyme A;
- of an enzyme E₄₈, which catalyzes the conversion of malonyl-coenzyme A into malonate semialdehyde;
- of an enzyme E₄₉, which catalyzes the conversion of malonate semialdehyde into 3-hydroxypropionate;
- of an enzyme E₅₀, which catalyzes the conversion of 3-hydroxypropionate into 3-hydroxypropionyl-coenzyme A;
- of an enzyme E₅₁, which catalyzes the conversion of 3-hydroxypropionyl-coenzyme A into acryloyl-coenzyme A;
- of an enzyme E₅₂, which catalyzes the conversion of acryloyl-coenzyme A into propionyl-coenzyme A;
- of an enzyme E₅, which catalyzes the conversion of propionyl-coenzyme A into methylmalonate semialdehyde;
- of an enzyme E₄, which catalyzes the conversion of methylmalonate semialdehyde into 3-hydroxyisobutyrate, or
1c) the cell is capable of forming 3-hydroxyisobutyric acid or polyhydroxyalkanoates based on 3-hydroxyisobutyric acid via acryloyl-coenzyme A as intermediate, and
1c₁) the cell features an activity of at least one of the following enzymes E₂ to E₄, E₅₆, E₇₂ and E₇₃ which is increased in comparison with its wild type:
- of an enzyme E₇₂, which catalyzes the conversion of beta-alanine into beta-alanyl-coenzyme A,
- of an enzyme E₇₃, which catalyzes the conversion of beta-alanyl-coenzyme A into acrylyl-coenzyme A,
- of an enzyme E₅₆, which catalyzes the conversion of acrylyl-coenzyme A into methylmalonyl-coenzyme A,
- of an enzyme E₂, which catalyzes the conversion of methylmalonyl-coenzyme A into methyl malonate;
- of an enzyme E₃, which catalyzes the conversion of methyl malonate into methylmalonate semialdehyde;
- of an enzyme E₄, which catalyzes the conversion of methylmalonate semialdehyde into 3-hydroxyisobutyric acid, or
2) the formation of 3-hydroxyisobutyric acid or polyhydroxyalkanoates based on 3-hydroxyisobutyric acid takes place via 3-hydroxybutyryl-coenzyme A as precursor, and where
2a) the cell is capable of forming 3-hydroxyisobutyric acid or polyhydroxyalkanoates based on 3-hydroxyisobutyric acid via isobutyryl coenzyme A as intermediate, where
2a₁) the cell features an activity of at least one of the following enzymes E₇₆ to E₇₉, E₆₀, E₆₁ and E₈ which is increased in comparison with its wild type:
- of an enzyme E₇₆, which catalyzes the conversion of pyruvate into 2-acetolactate;
- of an enzyme E₇₇, which catalyzes the conversion of 2-acetolactate into 2,3-dihydroxyisovalerate;
- of an enzyme E₇₈, which catalyzes the conversion of 2,3-dihydroxyisolvalerate into 2-oxoisovalerate;
- of an enzyme E₇₉, which catalyzes the conversion of 2-oxoisovalerate into isobutyryl-coenzyme A;
- of an enzyme E₆₀, which catalyzes the conversion of isobutyryl-coenzyme A into methacrylyl-coenzyme A;
- of an enzyme E₆₁, which catalyzes the conversion of methacrylyl-coenzyme A into 3-hydroxyisobutyryl-coenzyme A;
- of an enzyme E₈, which catalyzes the conversion of 3-hydroxyisobutyryl-coenzyme A into 3-hydroxyisobutyrate, or
2a₂) the cell features an activity of at least one of the following enzymes E₈, E₆₀ to E₆₁ and E₇₉ to E₈₀ which is increased in comparison with its wild type:
- of an enzyme E₈₀, which catalyzes the conversion of L-valine into 2-oxoisovalerate;
- of an enzyme E₇₉, which catalyzes the conversion of 2-oxoisovalerate into isobutyryl-coenzyme A;
- of an enzyme E₆₀, which catalyzes the conversion of isobutyryl-coenzyme A into methacrylyl-coenzyme A;
- of an enzyme E₆₁, which catalyzes the conversion of methacrylyl-coenzyme A into 3-hydroxyisobutyryl-coenzyme A;
- of an enzyme E₈, which catalyzes the conversion of 3-hydroxyisobutyryl-coenzyme A into 3-hydroxyisobutyrate.

2. The process according to Claim 1, where the enzyme E₁ in 1a₁) is a methylmalonyl-coenzyme A mutase (EC 5.4.99.2).

3. The process according to Claim 1, where in 1a₂) the enzyme
E₂ is a methylmalonyl-coenzyme A hydrolase (EC 3.2.1.17),
E₃ is an aldehyde dehydrogenase (EC 1.2.1.3) or an aldehyde oxidase (EC 1.2.3.1) and
E₄ is a 3-hydroxyisobutyrate dehydrogenase (EC 1.1.1.31) or a 3-hydroxyacyl-coenzyme A dehydrogenase (EC 1.1.1.35).

4. The process according to Claim 1, where in 1a₃) the enzyme
E₆ is a methylmalonyl-coenzyme A epimerase (EC 5.1.99.1)
E₇ is a methylmalonyl-coenzyme A decarboxylase (EC 4.1.1.41),
E₅ is a methylmalonate-semialdehyde dehydrogenase (EC 1.2.1.27), and
E₄ is a 3-hydroxyisobutyrate dehydrogenase (EC 1.1.1.31) or a 3-hydroxyacyl-coenzyme A dehydrogenase (EC 1.1.1.35).

5. The process according to Claim 1, where in 1a₄) the enzyme
E₇ is a methylmalonyl-coenzyme A decarboxylase (EC 4.1.1.41),
E₅ is a methylmalonate-semialdehyde dehydrogenase (EC 1.2.1.27), and
E₄ is a 3-hydroxyisobutyrate dehydrogenase (EC 1.1.1.31) or a 3-hydroxyacyl-coenzyme A dehydrogenase (EC 1.1.1.35).

6. The process according to Claim 1, where in 1a₅) the enzyme
E₄₆ is a glutamate synthase (EC 1.4.1.13 or EC 1.4.1.14), a glutamate dehydrogenase (EC 1.4.1.2, EC 1.4.1.3 or EC 1.4.1.4) or an aspartate transaminase (EC 2.6.1.1 or EC 2.6.1.2) and
E₂₈ is a 2-oxoglutarate synthase (EC 1.2.7.3).

7. The process according to Claim 1, where in 1b₁) the enzyme
E₄ is a 3-hydroxyisobutyrate dehydrogenase (EC 1.1.1.31) or a 3-hydroxyacyl-coenzyme A dehydrogenase (EC 1.1.1.35),
E₅ is a methylmalonate-semialdehyde dehydrogenase (EC 1.2.1.27),
E₄₇ is a malonyl-coenzyme A decarboxylase (EC 4.1.1.9), a malonate coenzyme A transferase (EC 2.8.3.3), a methylmalonyl-coenzyme A carboxytransferase (EC 2.1.3.1) or an acetyl-coenzyme A carboxylase (EC 6.4.1.2),
E₄₈ is a malonate-semialdehyde dehydrogenase (EC 1.2.1.18),
E₄₉ is a 3-hydroxypropionate dehydrogenase (EC 1.1.1.59),
E₅₀ is a 3-hydroxyisobutyryl-coenzyme A hydrolase (EC 3.1.2.4),
E₅₁ is an enoyl-coenzyme A hydratase (EC 4.2.1.17) and
E₅₂ is an acyl-coenzyme A dehydrogenase (EC 1.3.99.3).

8. The process according to Claim 1, where in 1c₁) the enzyme
E₇₂ is a coenzyme A transferase (EC 2.8.3.1) or coenzyme A synthetase, preferably a coenzyme A transferase,
E₇₃ is a beta-alanyl-coenzyme A ammonia-lyase (EC 4.3.1.6),
E₅₆ is a crotonyl-coenzyme A decarboxylase
E₂ is a methylmalonyl-coenzyme A hydrolase (EC 3.1.2.17),
E₃ is an aldehyde dehydrogenase (EC 1.2.1.3) or an aldehyde oxidase (EC 1.2.3.1) and
E₄ is a 3-hydroxyisobutyrate dehydrogenase (EC 1.1.1.31) or a 3-hydroxyacyl-coenzyme A dehydrogenase (EC 1.1.1.35).

9. The process according to Claim 1, where in 2a₁) the enzyme
E₈ is a 3-hydroxyisobutyryl-coenzyme A hydrolase (EC 3.1.2.4),
E₇₆ is an acetolactate synthase (EC 2.2.1.6),
E₇₇ is a dihydroxyisovalerate dehydrogenase (EC 1.1.1.86),
E₇₈ is a 2,3-dihydroxyisovalerate dehydratase (EC 4.2.1.9),
E₇₉ is a 2-oxoisovalerate dehydrogenase (EC 1.2.1.25 or EC 1.2.4.4),
E₆₀ is an acyl-coenzyme A dehydrogenase (EC 1.3.99.3), a butyryl-coenzyme A dehydrogenase (EC 1.3.99.2) or a 2-methylacyl-coenzyme A dehydrogenase (EC 1.3.99.12), and
E₆₁ is an enoyl-coenzyme A hydratase (EC 4.2.1.17).

10. The process according to Claim 1, where in 2a₂) the enzyme
E₈ is a 3-hydroxyisobutyryl-coenzyme A hydrolase (EC 3.1.2.4),
E₆₀ is an acyl-coenzyme A dehydrogenase (EC 1.3.99.3), a butyryl-coenzyme A dehydrogenase (EC 1.3.99.2) or a 2-methylacyl-coenzyme A dehydrogenase (EC 1.3.99.12),
E₆₁ is an enoyl-coenzyme A hydratase (EC 4.2.1.17),
E₇₉ is a 2-oxoisovalerate dehydrogenase (EC 1.2.1.25 or EC 1.2.4.4), and
E₈₀ is an amino acid transferase (EC 2.6.1.42).

11. The process according to Claim 1, where the cell may be obtained by a process for preparing a genetically modified cell, comprising the process step of increasing, in the cell, the activity of at least one of the enzymes mentioned in Claims 1 to 10.

12. A process of preparing polymethacrylic acid or polymethacrylic esters, comprising the process steps
IIIA) preparation of methacrylic acid by a method according to any of Claims 1 to 11,
IIIB) free-radical polymerization of the methacrylic acid,
it being possible, if appropriate, to esterify at least in part the carboxyl groups of the methacrylic acid or the carboxylate group of the methacrylate before or after the free-radical polymerization reaction.

## Revendications

1. Procédé de préparation d'acide méthacrylique ou d'esters de l'acide méthacrylique, comprenant les étapes :
IA) préparation d'acide 3-hydroxyisobutyrique par un procédé comprenant l'étape de mise en contact d'une cellule qui a subi une modification génétique par rapport à son type sauvage de telle sorte qu'elle soit à même de former, par comparaison avec son type sauvage, plus d'acide 3-hydroxyisobutyrique ou de polyhydroxyalcanoates à base d'acide 3-hydroxyisobutyrique, avec un milieu nutritif contenant en tant que source de carbone des hydrates de carbone, du glycérol, du dioxyde de carbone, du méthane, du méthanol, de la L-valine ou du L-glutamate, dans des conditions dans lesquelles il y a formation, à partir de la source de carbone, d'acide 3-hydroxyisobutyrique ou de polyhydroxyalcanoates à base d'acide 3-hydroxyisobutyrique, éventuellement purification de l'acide 3-hydroxyisobutyrique à partir du milieu nutritif, ainsi qu'éventuellement neutralisation de l'acide 3-hydroxyisobutyrique,
IB) déshydratation de l'acide 3-hydroxyisobutyrique, avec formation d'acide méthacrylique, ainsi qu'éventuellement estérification de l'acide méthacrylique, dans lequel
1) la formation de l'acide 3-hydroxyisobutyrique ou des polyhydroxyalcanoates à base d'acide 3-hydroxyisobutyrique a lieu par l'intermédiaire de méthylmalonate-semi-aldéhyde servant de précurseur, et où
1a) la cellule est à même de former de l'acide 3-hydroxyisobutyrique ou des polyhydroxyalcanoates à base d'acide 3-hydroxyisobutyrique, par l'intermédiaire de succinyl-coenzyme A servant de produit intermédiaire,
et où
1a₁) la cellule présente une activité d'une enzyme E₁, augmentée par comparaison à son type sauvage, qui catalyse la conversion de la succinyl-coenzyme A en méthylmalonyl-coenzyme A, et/ou
1a₂) la cellule présente une activité, augmentée par rapport à son type sauvage, d'au moins l'une des enzymes suivantes E₂ à E₄ :
- une enzyme E₂, qui catalyse la conversion de la méthylmalonyl-coenzyme A en méthylmalonate,
- une enzyme E₃, qui catalyse la conversion du méthylmalonate en méthylmalonate-semi-aldéhyde,
- une enzyme E₄, qui catalyse la conversion du méthylmalonate-semi-aldéhyde en 3-hydroxyisobutyrate, ou
1a₃) la cellule présente une activité, augmentée par comparaison avec son type sauvage, d'au moins l'une des enzymes suivantes E₄, E₅, E₆ et E₇ :
- une enzyme E₆, qui catalyse la conversion de la (R)-méthylmalonyl-coenzyme A en (S)-méthylmalonyl-coenzyme A,
- une enzyme E₇, qui catalyse la conversion de la (S)-méthylmalonyl-coenzyme A en propionyl-coenzyme A,
- une enzyme E₅, qui catalyse la conversion de la propionyl-coenzyme A en méthylmalonate-semi-aldéhyde,
- une enzyme E₄, qui catalyse la conversion du méthylmalonate-semi-aldéhyde en acide 3-hydroxyisobutyrique, ou
1a₄) la cellule présente une activité, augmentée par comparaison avec le type sauvage, d'au moins l'une des enzymes suivantes E₄, E₅ et E₇ :
- une enzyme E₇, qui catalyse la conversion de la méthylmalonyl-coenzyme A en propionyl-coenzyme A,
- une enzyme E₅, qui catalyse la conversion de la propionyl-coenzyme A en méthylmalonate-semi-aldéhyde,
- une enzyme E₄, qui catalyse la conversion du méthylmalonate-semi-aldéhyde en acide 3-hydroxyisobutyrique, ou
1a₅) la cellule présente une activité, augmentée par comparaison avec son type sauvage, d'au moins l'une des enzymes suivantes E₂₈ et E₄₆ :
- une enzyme E₄₆, qui catalyse la conversion du L-glutamate en 2-oxoglutarate,
- une enzyme E₂₈, qui catalyse la conversion du 2-oxoglutarate en succinyl-coenzyme A, ou
1b) la cellule est à même de produire de l'acide 3-hydroxyisobutyrique ou de polyhydroxyalcanoates à base d'acide 3-hydroxyisobutyrique par l'intermédiaire de la propionyl-coenzyme A servant de produit intermédiaire,
et où
1b₁) la cellule présente une activité, augmentée par comparaison avec son type sauvage, d'au moins l'une des enzymes suivantes E₄, E₅, et E₄₇ à E₅₂ :
- une enzyme E₄₇, qui catalyse la conversion de l'acétyl-coenzyme A en malonyl-coenzyme A,
- une enzyme E₄₈, qui catalyse la conversion de la malonyl-coenzyme A en malonate-semi-aldéhyde,
- une enzyme E₄₉, qui catalyse la conversion du malonate-semi-aldéhyde en 3-hydroxypropionate,
- une enzyme E₅₀, qui catalyse la conversion du 3-hydroxypropionate en 3-hydroxypropionyl-coenzyme A,
- une enzyme E₅₁, qui catalyse la conversion de la 3-hydroxypropionyl-coenzyme A en acryloyl-coenzyme A,
- une enzyme E₅₂, qui catalyse la conversion de l'acryloyl-coenzyme A en propionyl-coenzyme A,
- une enzyme E₅, qui catalyse la conversion de la propionyl-coenzyme A en méthylmalonate-semi-aldéhyde,
- une enzyme E₄, qui catalyse la conversion du méthylmalonate-semi-aldéhyde en 3-hydroxyisobutyrate, ou
1c) la cellule est à même de former de l'acide 3-hydroxyisobutyrique ou des polyhydroxyalcanoates à base d'acide 3-hydroxyisobutyrique par l'intermédiaire de l'acryloyl-coenzyme A servant de produit intermédiaire,
et
1c₁) la cellule présente une activité, augmentée par comparaison avec son type sauvage, d'au moins l'une des enzymes suivantes E₂ à E₄, E₅₆, E₇₂ et E₇₃ :
- une enzyme E₇₂, qui catalyse la conversion de la bêta-alanine en bêta-alanyl-coenzyme A,
- une coenzyme E₇₃, qui catalyse la conversion de la bêta-alanyl-coenzyme A en acrylyl-coenzyme A,
- une enzyme E₅₆, qui catalyse la conversion de l'acrylyl-coenzyme A en méthylmalonyl-coenzyme A,
- une enzyme E₂, qui catalyse la conversion de la méthylmalonyl-coenzyme A en méthylmalonate,
- une enzyme E₃, qui catalyse la conversion du méthylmalonate en méthylmalonate-semi-aldéhyde,
- une enzyme E₄, qui catalyse la conversion du méthylmalonate-semi-aldéhyde en acide 3-hydroxyisobutyrique, ou
2) la formation de l'acide 3-hydroxyisobutyrique ou des polyhydroxyalcanoates à base d'acide 3-hydroxyisobutyrique a lieu par l'intermédiaire de la 3-hydroxyisobutyryl-coenzyme A servant de précurseur, et où
2a) la cellule est à même de former de l'acide 3-hydroxyisobutyrique ou des polyhydroxyalcanoates à base d'acide 3-hydroxyisobutyrique par l'intermédiaire de l'isobutyryl-coenzyme A servant de produit intermédiaire, où
2a₁) la cellule présente une activité, augmentée par comparaison avec son type sauvage, d'au moins l'une des enzymes suivantes E₇₆ à E₇₉, E₆₀, E₆₁ et E₈ :
- une enzyme E₇₆, qui catalyse la conversion du pyruvate en 2-acétolactate,
- une enzyme E₇₇, qui catalyse la conversion du 2-acétolactate en 2,3-dihydroxyisovalérate,
- une enzyme E₇₈, qui catalyse la conversion du 2,3-dihydroxyisovalérate en 2-oxoisovalérate,
- une enzyme E₇₉, qui catalyse la conversion du 2-oxoisovalérate en isobutyryl-coenzyme A,
- une enzyme E₆₀, qui catalyse la conversion de l'isobutyryl-coenzyme A en méthacrylyl-coenzyme A,
- une enzyme E₆₁, qui catalyse la conversion de la méthacrylyl-coenzyme A en 3-hydroxyisobutyryl-coenzyme A,
- une enzyme E₈, qui catalyse la conversion de la 3-hydroxyisobutyryl-coenzyme A en 3-hydroxyisobutyrate, ou
2a₂) la cellule présente une activité, augmentée par comparaison avec son type sauvage, d'au moins l'une des enzymes suivantes E₈, E₆₀ à E₆₁ et E₇₉ à E₈₀ :
- une enzyme E₈₀, qui catalyse la conversion de la L-valine en 2-oxoisovalérate,
- une enzyme E₇₉, qui catalyse la conversion du 2-oxoisovalérate en isobutyryl-coenzyme A,
- une enzyme E₆₀, qui catalyse la conversion de l'isobutyryl-coenzyme A en méthacrylyl-coenzyme A,
- une enzyme E₆₁, qui catalyse la conversion de la méthacrylyl-coenzyme A en 3-hydroxyisobutyryl-coenzyme A,
- une enzyme E₈, qui catalyse la conversion de la 3-hydroxyisobutyryl-coenzyme A en 3-hydroxyisobutyrate.

2. Procédé selon la revendication 1, dans lequel l'enzyme E₁ de 1a₁) est une méthylmalonyl-coenzyme A-mutase (EC 5.4.99.2).

3. Procédé selon la revendication 1, dans lequel, dans 1a₂), l'enzyme
E₂ est une méthylmalonyl-coenzyme A-hydrolase (EC 3.2.1.17),
E₃ est une aldéhyde-déshydrogénase (EC 1.2.1.3) ou une aldéhyde-oxydase (EC 1.2.3.1) et
E₄ est une 3-hydroxyisobutyrate-déshydrogénase (EC 1.1.1.31) ou une 3-hydroxyacyl-coenzyme A-déshydrogénase (EC 1.1.1.35).

4. Procédé selon la revendication 1, dans lequel, dans 1a₃), l'enzyme
E₆ est une méthylmalonyl-coenzyme A-épimérase (EC 5.1.99.1),
E₇ est une méthylmalonyl-coenzyme A-décarboxylase (EC 4.1.1.41),
E₅ est une méthylmalonate-semi-aldéhyde-déshydrogénase (EC 1.2.1.27), et
E₄ est une 3-hydroxyisobutyrate-déshydrogénase (EC 1.1.1.31) ou une 3-hydroxyacyl-coenzyme A-déshydrogénase (EC 1.1.1.35).

5. Procédé selon la revendication 1, dans lequel, dans 1a₄), l'enzyme
E₇ est une méthylmalonyl-coenzyme A-décarboxylase (EC 4.1.1.41),
E₅ est une méthylmalonate-semi-aldéhyde-déshydrogénase (EC 1.2.1.27), et
E₄ est une 3-hydroxyisobutyrate-déshydrogénase (EC 1.1.1.31) ou une 3-hydroxyacyl-coenzyme A-déshydrogénase (EC 1.1.1.35).

6. Procédé selon la revendication 1, dans lequel, dans 1a₅), l'enzyme
E₄₆ est une glutamate-synthase (EC 1.4.1.13) ou EC 1.4.1.14), une glutamate-déshydrogénase (EC 1.4.1.2), EC 1.4.1.3 ou EC 1.4.1.4) ou une aspartate-transaminase (EC 2.6.1.1 ou EC 2.6.1.2) et
E₂₈ est une 2-oxoglutarate-synthase (EC 1.2.7.3).

7. Procédé selon la revendication 1, dans lequel, dans 1b₁), l'enzyme
E₄ est une 3-hydroxyisobutyrate-déshydrogénase (EC 1.1.1.31) ou une 3-hydroxyacyl-coenzyme A-déshydrogénase (EC 1.1.1.35),
E₅ est une méthylmalonate-semi-aldéhyde-déshydrogénase (EC 1.2.1.27),
E₄₇ est une malonyl-coenzyme A-décarboxylase (EC 4.1.1.9), une malonate-coenzyme A-transférase (EC 2.8.3.3), une méthylmalonyl-coenzyme A-carboxytransférase (EC 2.1.3.1) ou une acétyl-coenzyme A-carboxylase (EC 6.4.1.2),
E₄₈ est une malonate-semi-aldéhyde-déshydrogénase (EC 1.2.1.18),
E₄₉ est une 3-hydroxypropionate-déshydrogénase (EC 1.1.1.59),
E₅₀ est une 3-hydroxyisobutyryl-coenzyme A-hydrolase (EC 3.1.2.4),
E₅₁ est une énoyl-coenzyme A-hydratase (EC 4.2.1.17) et E₅₂ est une acyl-coenzyme A-déshydrogénase (EC 1.3.99.3).

8. Procédé selon la revendication 1, dans lequel, dans 1c₁), l'enzyme
E₇₂ est une coenzyme A-transférase (EC 2.8.3.1) ou une coenzyme A-synthétase, de préférence une coenzyme A-transférase,
E₇₃ est une bêta-alanyl-coenzyme A-ammonium-lysase (EC 4.3.1.6),
E₅₆ est une crotonyl-coenzyme A-décarboxylase,
E₂ est une méthylmalonyl-coenzyme A-hydrolase (EC 3.1.2.17),
E₃ est une aldéhyde-déshydrogénase (EC 1.2.1.3) ou une aldéhyde-oxydase (EC 1.2.3.1) et
E₄ est une 3-hydroxyisobutyrate-déshydrogénase (EC 1.1.1.31) ou une 3-hydroxyacyl-coenzyme A-déshydrogénase (EC 1.1.1.35).

9. Procédé selon la revendication 1, dans lequel, dans 2a₁), l'enzyme
E₈ est une 3-hydroxyisobutyryl-coenzyme A-hydrolase (EC 3.1.2.4),
E₇₆ est une acétolactate-synthase (EC 2.2.1.6),
E₇₇ est une dihydroxyisovalérate-déshydrogénase (EC 1.1.1.86),
E₇₈ est une 2,3-dihydroxyisovalérate-déshydratase (EC 4.2.1.9),
E₇₉ est une 2-oxoisovalérate-déshydrogénase (EC 1.2.1.25 ou EC 1.2.4.4),
E₆₀ est une acyl-coenzyme A-déshydrogénase (EC 1.3.99.3), une butyryl-coenzyme A-déshydrogénase (EC 1.3.99.2) ou une 2-méthylacyl-coenzyme A-déshydrogénase (EC 1.3.99.12), et
E₆₁ est une énoyl-coenzyme A-hydratase (EC 4.2.1.17).

10. Procédé selon la revendication 1, dans lequel, dans 2a₂), l'enzyme
E₈ est une 3-hydroxyisobutyryl-coenzyme A-hydrolase (EC 3.1.2.4),
E₆₀ est une acyl-coenzyme A-déshydrogénase (EC 1.3.99.3), une butyryl-coenzyme A-déshydrogénase (EC 1.3.99.2) ou une 2-méthylacyl-coenzyme A-déshydrogénase (EC 1.3.99.12),
E₆₁ est une énoyl-coenzyme A-hydratase (EC 4.2.1.17),
E₇₉ est une 2-oxoisovalérate-déshydrogénase (EC 1.2.1.25 ou EC 1.2.4.4), et
E₈₀ est une acide aminé-transférase (EC 2.6.1.42).

11. Procédé selon la revendication 1, dans lequel la cellule peut être obtenue par un procédé de préparation d'une cellule génétiquement modifiée, comprenant l'étape d'augmentation de l'activité, dans la cellule, d'au moins une des enzymes mentionnées dans les revendications 1 à 10.

12. Procédé de préparation de poly(acide méthacrylique) ou de polyméthacrylates, comprenant les étapes
IIIA) préparation d'acide méthacrylique par un procédé selon l'une des revendications 1 à 11,
IIIB) polymérisation radicalaire de l'acide méthacrylique,
dans lequel éventuellement les groupes carboxyle de l'acide méthacrylique ou le groupe carboxylate du méthacrylate peuvent être au moins partiellement estérifiés avant ou après la polymérisation radicalaire.
